# EUROPEAN PATENT SPECIFICATION

(11) **EP 1 317 466 B1**
(45) Date of publication and mention of the grant of the patent: **23.02.2005**
(21) Application number: 01963238.9
(22) Date of filing: 06.09.2001
(51) Int. Cl.: C07H 21/00

(54) **SYNTHONS FOR OLIGONUCLEOTIDE SYNTHESIS**
SYNTHONE ZUR HERSTELLUNG VON OLIGONUKLEOTIDEN
SYNTHONS DESTINES A LA SYNTHESE D'OLIGONUCLEOTIDES

(30) Priority: 07.09.2000 US 230685 P
(43) Date of publication of application: 11.06.2003
(73) Proprietor: Avecia Biotechnology, Inc., Milford, MA 01757 (US)
(72) Inventor: SINHA, Nanda, Milford, MA 01757 (US)
(74) Representative: Revell, Christopher
(86) International application number: PCT/GB2001/003973
(87) International publication number: WO 2002/020543

(56) References cited:
- EP-A- 0 035 719
- EP-A- 0 090 789
- EP-A- 0 386 987
- WO-A-93/13121
- WO-A-94/00472
- WO-A-96/40708
- US-A- 5 218 088
- XU Z.S. ET AL.: "REGIOSELECTIVE SYNTHESIS OF OLIGONUCLEOTIDES" CHIN. CHEM. LETT., vol. 5, no. 6, - 1994 pages 467-470, XP008000676
- DATABASE CA [Online] CHEMICAL ABSTRACTS SERVICE, COLUMBUS, OHIO, US; IMANISHI, TAKESHI ET AL: "Preparation of novel bicyclo nucleoside analogues as intermediates for oligonucleotide analogs both having anti-HIV activity" retrieved from STN Database accession no. 134:131768 XP002190148 & WO 2001 007455 A (SANKYO COMPANY, LIMITED, JAPAN) 1 February 2001 (2001-02-01)
- BEAUCAGE S L ET AL: "ADVANCES IN THE SYNTHESIS OF OLIGONUCLEOTIDES BY THE PHOSPHORAMIDITE APPROACH" TETRAHEDRON, ELSEVIER SCIENCE PUBLISHERS, AMSTERDAM, NL, vol. 48, no. 12, 1992, pages 2223-2311, XP000915225 ISSN: 0040-4020
- CARUTHERS H H ET AL: "SYNTHESIS OF OLIGONUCLEOTIDES USING THE PHOSPHORAMIDITE METHOD" BIOACTIVE MOLECULES, ELSEVIER, AMSTERDAM, NL, vol. 3, 1987, pages 3-21, XP000671967 ISSN: 0921-0687
- CARUTHERS M H ET AL: "CHEMICAL SYNTHESIS OF DEOXYOLIGONUCLEOTIDES BY THE PHOSPHORAMIDITE METHOD" METHODS IN ENZYMOLOGY, ACADEMIC PRESS INC, SAN DIEGO, CA, US, vol. 154, 1987, pages 287-326, XP001008645 ISSN: 0076-6879
- BARDELLA, F. ET AL.: "GEL-PHASE 13P-NMR. A NEW ANALYTICAL TOOL TO EVALUATE SOLID PHASE OLIGONUCLEOTIDE SYNTHESIS" BIOORGANIC & MEDICINAL CHEMISTRY LETTERS, vol. 3, no. 12, 1993, pages 2793-2796, XP008000632
- ONO A ET AL: "THE SYNTHESIS OF BLOCKED TRIPLET-PHOSPHORAMIDITES AND THEIR USE IN MUTAGENESIS" NUCLEIC ACIDS RESEARCH, OXFORD UNIVERSITY PRESS, SURREY, GB, vol. 23, no. 22, 1995, pages 4677-4682, XP001026095 ISSN: 0305-1048

### SEQUENCE LISTING

<110> Avecia Biotechnology, Inc. et al
<120> Synthons for oligonucleotide synthesis
<130> SMC 60437/WO
<160> 2
<170> PatentIn version 3.1
<210> 1
   <211> 21
   <212> DNA
   <213> Artificial Sequence
<220>
   <223> Sequence prepared in Example 4
<400> 1
   acacacacac acacacacac t
   21
<210> 2
   <211> 21
   <212> DNA
   <213> Artificial Sequence
<220>
   <223> Sequence prepared in Example 4
<400> 2
   gtgtgtgtgt gtgtgtgtgt t
   21

## Description

### BACKGROUND OF THE INVENTION

Synthetic oligonucleotides are important in the diagnostic field for the detection of genetic and viral diseases. In addition, large scale synthesis of oligonucleotides for use in antisense and related therapies has become increasingly important since FDA approval of an oligonucleotide analog for the treatment of cytomegalovirus (CMV), and several other oligonucleotide analogs are currently in clinical trials. Kilogram quantities of a purified oligonucleotide analog are needed for each clinical trial.

Oligonucleotide analogs, which contain modified linkages between nucleotides, such as phosphorothioate linkages or a combination of phosphorothioate and phosphate diester linkages (chimeric oligonucleotides), have been found to be more useful than oligonucleotides which have only phosphate diester linkages in antisense applications because they are more resistant to degradation in the body. Typically, oligonucleotide analogs are synthesized by adding each nucleotide monomer one at a time until the desired sequence is complete. Addition of each nucleotide requires a cycle which minimally has three reaction steps: a coupling step, an oxidation or sulfurization step, and a 5'-deprotection step. Typically, a capping step is also performed after the oxidation or sulfurization step. Therefore, synthesis of an oligonucleotide having 21 bases requires 20 cycles or from 60-80 reactions (depending on whether the capping step is preformed in each cycle). Since the yield in each reaction step is generally less than 100%, it is difficult to synthesize long oligonucleotides in good yield and very difficult, if not impossible, to synthesize oligonucleotides that have greater than about 150 nucleotide bases with the current technology. In addition, the large number of reaction steps uses up large quantities of reagents, thus making the synthesis more expensive. In order to obtain longer oligonucleotides in good yield and/or to reduce the cost of reagents used to synthesize oligonucleotides, it would be desirable to reduce the number of reaction steps necessary to synthesize oligonucleotides.

EP 0 090 789 discloses a process for the preparation of oligonucleotides on solid support.

The use of dimers or trimers has been suggested to increase the yield and availability of longer synthetic oligonucleotides. However, dimer and trimers utilized to date have phosphorus linkages which have been either oxidized to form a phosphate diester or sulfurized to form a phosphorothioate. Therefore, these building blocks are not as versatile as monomer building blocks in which the same monomer building blocks can be used to form oligonucleotides with either a phosphate diester, a phosphorothioate backbone or a combination of phosphodiester and phosphorothioate backbone.

After synthesis an oligonucleotide is usually separated from impurities generated during synthesis. Typically, these impurities consist of reagents, reaction by-product and failure sequences. Failure sequences are oligonucleotides where one or more coupling step failed so that the failure sequence is shorter than the desired length by one or more nucleotides. Failure sequences are generally removed by ion-exchange chromatography. However, failure sequences which are only one nucleotide shorter than the desired length (i.e. N-1 species) are difficult to remove because their structural similarity to the desired product causes them to have a similar chromatographic mobility as the product oligonucleotide. N-2 and N-3 failure sequences are more readily separated from the desired product because they differ enough in structure to cause them to have substantially different chromatographic mobilities from the product oligonucleotide. Therefore, a synthetic method that does not produce N-1 failure sequences is desirable in order to improve the yield and purity of the desired oligonucleotide. Xu et al Chinese Chemical Letters 5, 467-470 (1994) describes a method for the solution phase synthesis of oligonucleotides employing the phoshitilating agent 9-fluorenemethylphosphorodiimidazolide.

Improvements in the synthesis of oligonucleotide and oligonucleotide analogs are necessary in order to produce these compounds in the quantity and quality necessary for therapeutic use. In addition, the ability to synthesize longer oligonucleotides in useful yields could extend their use in therapeutic and recombinant applications.

### SUMMARY OF THE INVENTION

The present invention relates to a trivalent phosphorus multimer, a method of utilizing a trivalent phosphorus multimer to prepare an oligonucleotide, and a method of preparing a trivalent phosphorus multimer. In addition, the invention relates to a solid support that is derivatized with a trivalent phosphorus multimer and a method of preparing the same.

In one aspect of the present invention, there is provided a phosphoramidite compound comprising two or more nucleoside moieties linked by one or more internucleoside phosphorus atoms, wherein the intemucleoside phosphorus atoms are phosphorus (III) atoms comprising a phosphite triester group and the phosphoramidite moiety is a group of formula -X¹-PR³NR⁴R⁵ wherein X¹, R³, R⁴ and R⁵ are as described below.

The phosphoramidite compounds comprise a phosphoramidite moiety which may be bonded to the 5'-position or preferably the 3'-position of the nucleoside moiety carrying the phosphoramidite moiety.

The nucleoside moieties may be d- or I-nucleosides, but in many embodiments are d-nucleosides. The nucleosides may be abasic, but in many embodiments comprises nucleobases. Commonly, the nucleobases are protected by suitable protecting groups known in the art.

In many embodiments, the phosphite triester group links the 5'-position of the nucleoside moiety carrying the phosphoramidite moiety with the 3'-position of a second nucleoside moiety. Most preferably, the phosphite triester group comprises a beta-cyanoethyloxy or beta-cyanoethylthio moiety.

In another aspect, there is provided a trivalent phosphorus multimer which can be represented by Structural Formula I, or a stereoisomer thereof:

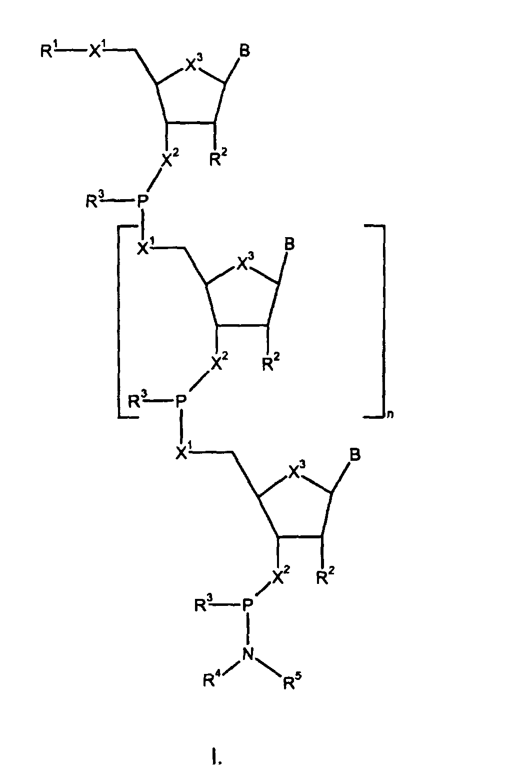

In Structural Formula I, each X¹ is -O-. Each X² is -O-. Each X³ is, independently, -O-, -S-, -CH₂-, or -(CH₂)₂-. In a preferred embodiment, each X³ is -O-. R¹ is a protecting group, preferably an acid labile protecting group or a trialkylsilyl group, such as *t*-butyldimethylsilyl or triisopropylsilyl. In a more preferred embodiment, R¹ is a substituted or unsubstituted trityl, 9-(phenyl-) xanthenyl (hereinafter "pixyl") or tetrahydropyranyl (hereinafter "THP"). In an even more preferred embodiment, R¹ is an unsubstituted trityl, a monoalkoxytrityl, a dialkoxytrityl, a trialkoxytrityl, THP or pixyl. Most preferably, R¹ is 4,4'-dimethoxytrityl. Each R² is, independently, -H, -F, -NHR⁶, -CH₂R⁶ or -OR⁶. Each R³ is, independently, -OCH₂CH₂CN, -SCH₂CH₂CN, 4-cyanobut-2-enylthio, 4-cyanobut-2-enyloxy, allylthio, allyloxy, crotylthio, or crotyloxy. R⁴ and R⁵ are each, independently, a substituted or unsubstituted aliphatic group, or R⁴ and R⁵ taken together with the nitrogen to which they are bonded form a heterocycloalkyl group in which the heterocycloalkyl ring is preferably a five or six membered ring. Preferably, each R⁴ and R⁵ is an isopropyl group. R⁶ is -H, a substituted or unsubstituted aliphatic group, a substituted or unsubstituted aromatic group, a substituted or unsubstituted aralkyl, or a protecting group, such as an alcohol protecting group, for example *t*-butyldimethylsilyl, or an amine protecting group. Each B is, independently, a protected or an unprotected nucleoside base or may represent H when one or more abasic moieties are present, n is 0 or a positive integer. When a trivalent phosphorus multimer of the invention is used to synthesize an oligonucleotide on a solid support, n is preferably 0 to 2. Preferably, n is 0 to 10 when a trivalent phosphorous multimer is used to synthesize an oligonucleotide in solution. In certain embodiments, R² represents a C-allyl group. Most preferably, R² represents H, OMe or OCH₂CH₂OMe.

In one embodiment, the trivalent phosphorus multimer can be represented by structural Formula II, or a stereoisomer thereof:

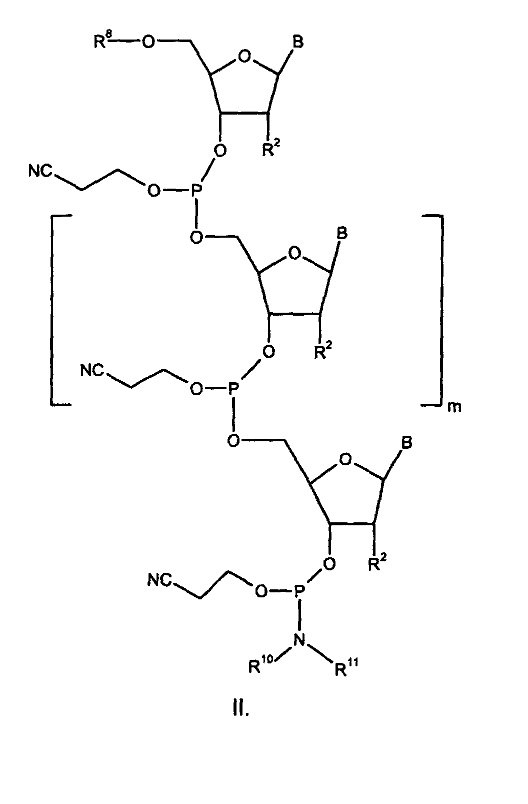

In Structural Formula II, B and R² are defined as for Structural Formula I. R⁸ is a substituted or unsubstituted trityl, such as 4,4'-dimethoxytrityl. R¹⁰ and R¹¹ are each, independently a substituted or unsubstituted aliphatic group. Preferably, each R¹⁰ and R¹¹ is an isopropyl group. m is 0 or 1.

The trivalent phosphorus multimer represented by Structural Formula I can be used to prepare an oligonucleotide represented by Structural Formula III, or a stereoisomer thereof:

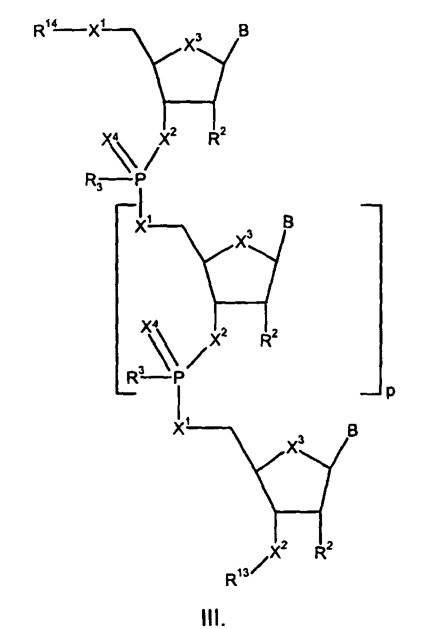

In Structural Formula III, X¹, X², X³, R², R³ and B are as defined for Structural Formula I. Each X⁴ is, independently, =O or =S. R¹³ is a hydroxyl protecting group, a thiol protecting group, an amine protecting group, a group of the formula -Y²-L-Y¹, a solid support or a group of the formula -Y²-L-Y²-R¹⁵. Y¹ is a functional group which can react with an amine, thiol or hydroxyl group. Preferably, Y¹ is an ester or a carboxylic acid group. Each Y², is, independently, a single bond, -C(O)-, -C(O)NR¹⁷-, -C(O)O-, -NR¹⁷- or -O-. L is a linker which is preferably a substituted or unsubstituted aliphatic group or a substituted or unsubstituted aromatic group. More preferably, L is a ethylene group. R¹⁷ is -H, a substituted or unsubstituted aliphatic group or a substituted or unsubstituted aromatic group. R¹⁵ is a solid support suitable for solid phase oligonucleotide synthesis, such as controlled-pore glass, polystyrene, microporous polyamide such as polydimethylacrylamide, polystyrene coated with polyethylene glycol, and polyethylene glycol supported on polystyrene, such as those solid supports commercially available under the trade name Tentagel. R¹⁴ is -H or a protecting group. Preferably, when R¹⁴ is a protecting group, it is an acid labile protecting group, *t*-butyldimethylsilyl or triisopropylsilyl. p is a positive integer. In one embodiment, the oligonucleotide synthesized with the trivalent phosphorus multimers is a phosphate and, therefore, has only phosphate linkages (ie each internucleotide phosphorus is bonded only to oxygen). In another embodiment, the oligonucleotide synthesized is a phosphorothioate and, therefore, has only phosphorothioate linkages (each internucleotide phosphorus is bonded to at least one S, preferably only one S). In yet another embodiment, the oligonucleotide synthesized is a chimeric oligonucleotide which comprises both phosphate and phosphorothioate internucleotide linkages.

In the method of preparing the oligonucleotide represented by Structural Formula III, a trivalent phosphorus multimer represented by Structural Formula I is coupled to a 5'-deprotected nucleotide or nucleoside represented by Structural Formula IV, or a stereoisomer thereof:

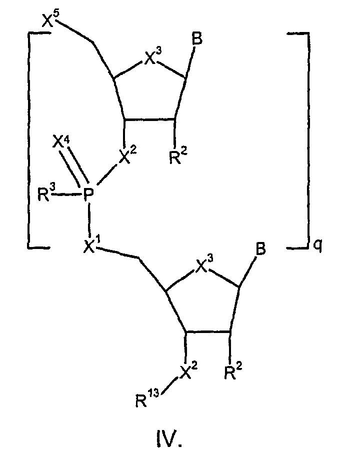

In Structural Formula IV, X¹, X², X³, X⁴, R², R³, R¹³, and B are defined as above. X⁵ is -OH or -SH. q is 0 or a positive integer. The coupling reaction forms a first intermediate represented by Structural Formula V, or a stereoisomer thereof:

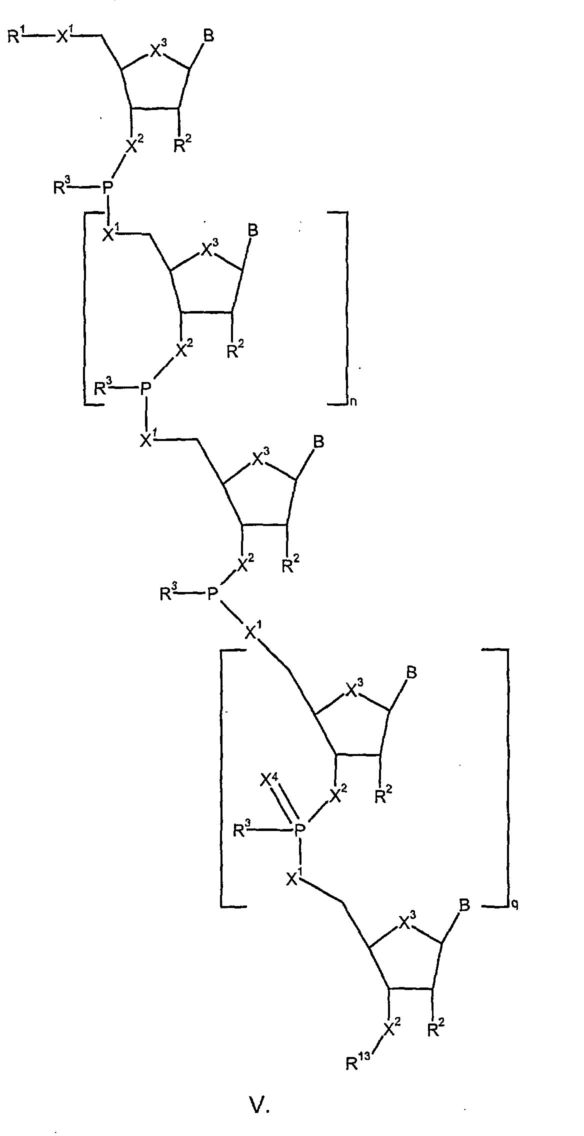

In Structural Formula V, X¹, X², X³, X⁴, R¹, R², R³, R¹³, B, n and q are defined as above. The trivalent phosphorus groups of the first intermediate are then oxidized or sulfurized to form a second intermediate represented by Structural Formula VI, or a stereoisomer thereof:

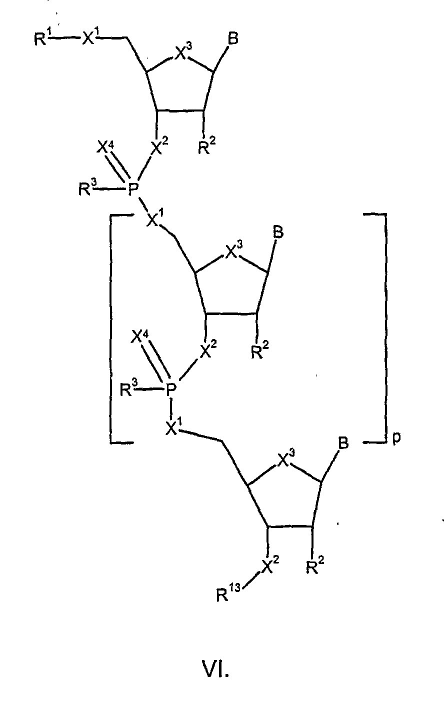

In Structural Formula VI, R¹, R², R³, R¹³, X¹, X², X³, X⁴, B and p are as defined above. Any 5'-deprotected nucleoside or nucleotide represented by Structural Formula IV which remains in the reaction mixture can optionally be treated with a reagent to cap the unreacted X⁵ groups. The second intermediate is then treated to remove R¹. If R¹ is an acid labile protecting group, the second intermediate is treated with an acid to remove R¹. If R¹ is a trialkylsilyl group, such as *t*-butyldimethylsilyl group or a triisopropylsilyl group, the second intermediate can be treated with fluoride ions to remove R¹. Typically, *t*-butyldimethylsilyl and a triisopropylsilyl are removed by treatment with a solution of tetrabutylammonium fluoride in THF. Methods for removing *t*-butyldimethylsilyl can be found in Greene, *et al*., *Protective Groups in Organic Synthesis* (1991), John Wiley & Sons, Inc., pages 77-83. The above reaction steps, or reaction cycle, can be repeated one or more times to form an oligonucleotide of the desired length. When it is desired to obtain an oligonucleotide product in which the 5'-end group is protected, the final step of the reaction cycle can be the capping step, if a capping step is done, or the final step of the reaction can be an oxidation or sulfurization step if a capping step is not done. Alternatively, the final step of the reaction cycle can be removal of R¹ if it is desired to obtain an oligonucleotide which does not have a 5'-protecting group.

The invention also relates to a method of preparing a trivalent phosphorus multimer represented by Structural Formula I, or a stereoisomer thereof. The method involves protecting a 3'-substituent of a nucleoside represented by Structural Formula VII, or a stereoisomer thereof: 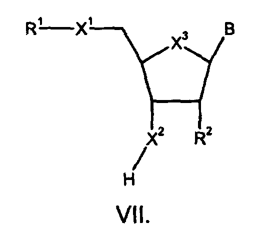 to form a first intermediate represented by Structural Formula VIII, or a stereoisomer thereof:

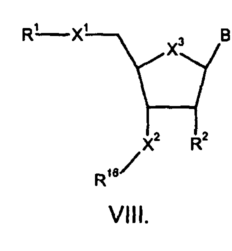

In Structural Formulas VII and VIII, X¹, X², X³, R¹ and B are as defined above. In Structural Formula VIII, R¹⁶ is a protecting group which is orthogonal to R¹. Therefore, R¹ is a protecting group that can be removed in the presence of R¹⁶ without reaction occurring at R¹⁶, and R¹⁶ is a protecting group which can be removed in the presence of R¹ without reaction occurring at R¹. Preferably, R¹⁶ is a levulynoyl group or a 3-benzoylpropionyl group. The first intermediate represented by Structural Formula VIII is then treated to remove R¹ to form a 5'-deprotected nucleoside. When R¹ is an acid labile protecting group, the first intermediate is treated with an acid to remove the acid labile protecting group. When R¹ is a trialkylsilyl protecting group, the first intermediate is typically treated with fluoride ions to remove R¹. The 5'-deprotected nucleoside is reacted in the presence of a coupling catalyst with a compound represented by Structural Formula IX, or a stereoisomer thereof:

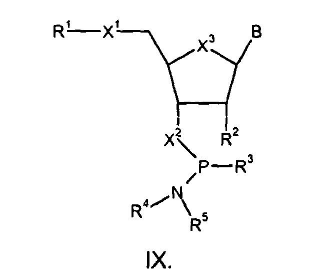

In Structural Formula IX, X¹, X², X³, R¹, R², R³, R⁴, R⁵ and B are as defined above. The 3',5'-protected dimer formed from the reaction can be represented by Structural Formula X, or a stereoisomer thereof:

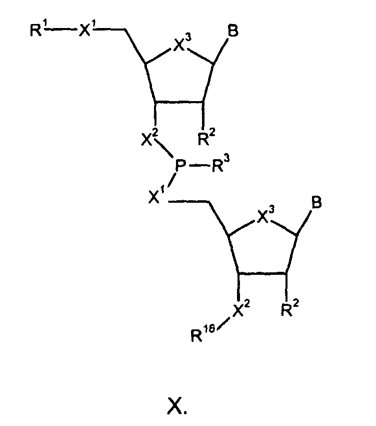

In Structural Formula X, X¹, X², X³, R¹, R², R³, R¹⁶ and B are as defined above. The 3',5'-protected dimer is treated to remove R¹⁶ to form a 3'-deprotected dimer. The 3'-deprotected dimer is optionally reacted in the presence of a coupling catalyst with a compound represented by Structural Formula XI, or a stereoisomer thereof:

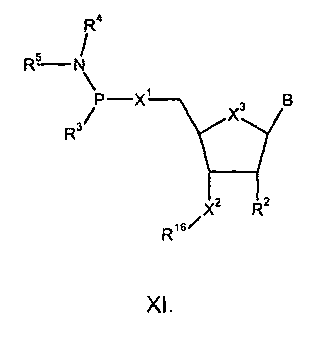

In Structural Formula XI, X¹, X², X³, R³, R⁴, R⁵, R¹⁶ and B are as defined previously. The product of the reaction is a 3',5'-protected trimer represented by Structural Formula XII, or a stereoisomer thereof:

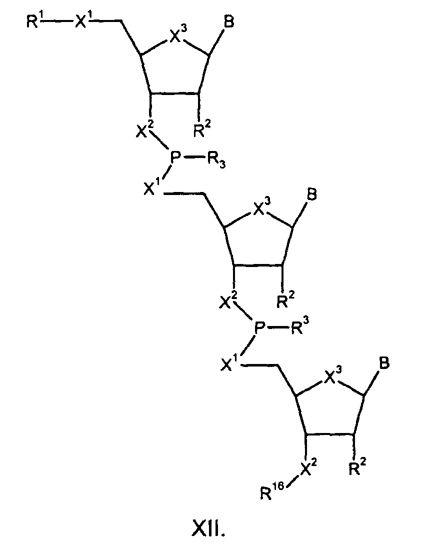

In Structural Formula XII, X¹, X², X³, R¹, R², R³, R¹⁶ and B are as previously defined.

Optionally, the 3',5'-protected trimer can be treated to remove R¹⁶ to form a 3'-deprotected trimer which can be reacted in the presence of a coupling catalyst with a compound represented by Structural Formula XI one or more times to form a 3',5'-protected multimer. The 3',5'-protected multimer is then treated to remove R¹⁶ to form a 3'-deprotected multimer. The 3'-deprotected multimer is reacted with a trivalent phosphorus compound represented by either XIIIa or XIIIb: 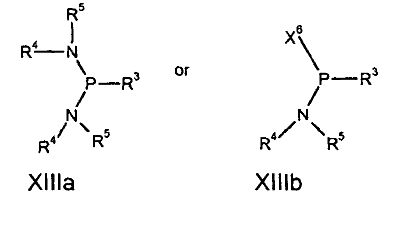 to form a trivalent phosphorus multimer represented by Structural Formula I. In Structural Formulas Xllla and Xlllb, R³, R⁴ and R⁵ are defined as above. X⁶ is a halogen, preferably chloro or bromo.

Alternatively, a trivalent phosphorus multimer represented by Structural Formula I, or a stereoisomer thereof can be prepared by protecting the 3'-substituent of a nucleoside represented by Structural Formula VII, or a stereoisomer thereof to form a first intermediate represented by Structural Formula VIII, or a stereoisomer thereof. The first intermediate is treated to remove R¹, thereby forming a 5'-deprotected nucleoside. The 5'-deprotected nucleoside is reacted with a trivalent phosphorus compound represented by either Xllla or Xlllb to form a 5'-phosphoramidite represented by Structural Formula XI or a stereoisomer thereof. The 5'-phosphoramidite is reacted, in the presence of a coupling catalyst, with a compound represented by Structural Formula VII or a stereoisomer thereof to form a 3',5'-protected dimer represented by Structural Formula X or a stereoisomer thereof. The 3',5'-protected dimer is treated to remove R¹⁶ to form a 3'-deprotected dimer. Optionally, the 3'-deprotected dimer is reacted in the presence of a coupling catalyst with a compound represented by Structural Formula XI or a stereoisomer thereof to form a 3',5'-protected trimer represented by Structural Formula XII or a stereoisomer thereof. Optionally, removal of R¹⁶ and the coupling step can be repeated one more time to form a 3',5'-protected multimer. R¹⁶ is removed from the 3',5'-protected multimer to form a 3'-deprotected multimer which is reacted with a trivalent phosphorus compound represented by one of Structural Formulas XIIIa or XIIIb to form the trivalent phosphorus multimer represented by Structural Formula I or a stereoisomer thereof.

Another embodiment of the invention is a trivalent phosphorus multimer derivatized solid support and a method of preparing the same. The trivalent phosphorus multimer derivatized solid support can be represented by Structural Formula XIV or a stereoisomer thereof:

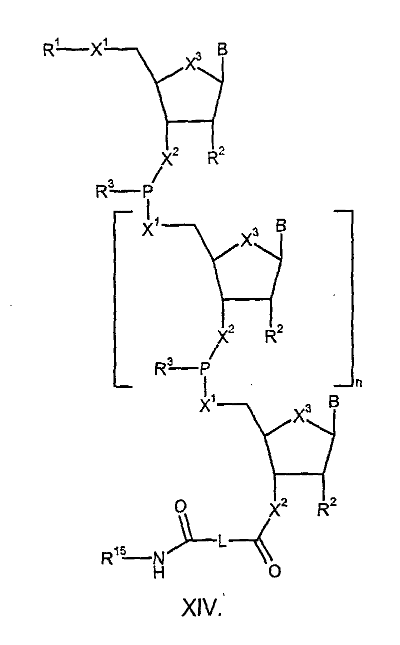

In Structural Formula XIV, X¹, X², X³, R¹, R², R³, R¹⁵, B, L and n are as defined above.

The method of preparing the trivalent phosphorus multimer derivatized solid support represented by Structural Formula XIV involves preparing a 3'-deprotected multimer by one of the methods described above. The 3'-deprotected multimer is reacted in the presence of a base with a compound represented by Structural Formula XVa or XVb: 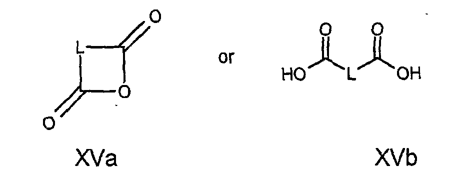 to form a solid support loading reagent represented by Structural Formula XVI or a stereoisomer thereof:

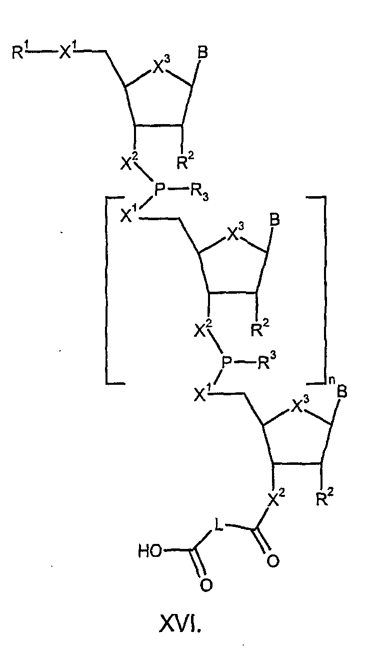

In Structural Formulas XVa, XVb, and XVI, L is as defined above. In Structural Formula XVI, X¹, X², X³, R¹, R², R³, and B are as defined above. The solid support loading reagent is reacted with a solid support derivatized with primary or secondary amine functional groups in the presence of a base and a substituted or unsubstituted dialiphatic carbodiimide to form the trivalent phosphorus multimer derivatized solid support represented by Structural Formula XIV.

In certain embodiments, the stereoisomers of the compounds of Structural Formulae I to XII, XIV and XVI are the corresponding structural formulae in which the sugar moieties are the I-stereoisomers as opposed to the d-stereoisomers illustrated.

Because the multimers of the invention have trivalent phosphorus linkages, chimeric oligonucleotides (i.e., oligonucleotides that have both phosphodiester and phosphorothioate linkages) can be synthesized from the trivalent phosphorus multimers. The synthesis of oligonucleotides using trivalent phosphorus multimers is expected to improve the yield of the desired product because fewer reaction steps will be necessary than when monomers are utilized to synthesize oligonucleotides. Therefore, use of the trivalent phosphorus multimers of the invention is expected to facilitate the synthesis of longer oligonucleotides than is currently possible with available techniques. In addition, use of the multimers of the present invention will conserve reagents because half as many, or fewer, reaction steps are required to synthesize an oligonucleotide of a given length than when the synthesis is done using monomers. Finally, because N-1 side products are not likely to be produced when multimers are used to synthesize oligonucleotides, the desired oligonucleotide will be more readily separated from failure sequences. Therefore, the desired product can be prepared with higher purity and in greater yield due to less loss of product during purification.

### BRIEF DESCRIPTION OF THE DRAWINGS

Figure 1 is a synthetic scheme for a synthesis of a trivalent phosphorus multimer.
Figure 2 is an alternative synthetic scheme for a synthesis of a trivalent phosphorus multimer.
Figure 3 represents a 2'-O-methyl dimer prepared in Example 2.
Figure 4 represents two dimers utilized to prepare a phosphodiester and two phosphorothioates synthesized in Example 4.
Figure 5 is a synthetic scheme for the synthesis of oligonucleotides synthesized in Example 4 using trivalent phosphorus dimers. It will be recognised that the thymidine moiety shown bonded to the solid support may be replaced with alternative nucleosidic moieties as appropriate for the sequence desired to be manufactured.

### DETAILED DESCRIPTION OF THE INVENTION

Aliphatic groups, as used herein, include straight chained or branched C₁-C₁₈ hydrocarbons which are completely saturated or which contain one or more non-aromatic double bonds, or cyclic C₃-C₁₈ hydrocarbons which are completely saturated or which contain one or more unconjugated double bonds. Lower alkyl groups are straight chained or branched C₁-C₈ hydrocarbons or C₃-C₈ cyclic hydrocarbons which are completely saturated. Aliphatic groups are preferably lower alkyl groups.

Aromatic groups include carbocyclic aromatic ring systems (e.g., phenyl) and carbocyclic aromatic ring systems fused to one or more carbocyclic aromatic or non-aromatic ring (e.g., naphthyl, anthracenyl and 1,2,3,4-tetrahydronaphthyl).

Heteroaromatic groups, as used herein, include heteroaryl ring systems (e.g., thienyl, pyridyl, pyrazole, isoxazolyl, thiadiazolyl, oxadiazolyl, indazolyl, furans, pyrroles, imidazoles, pyrazoles, triazoles, pyrimidines, pyrazines, thiazoles, isoxazoles, isothiazoles, tetrazoles, or oxadiazoles) and heteroaryl ring systems in which a carbocyclic aromatic ring, carbocyclic non-aromatic ring, heteroaryl ring or a heterocycloalkyl ring is fused to one or more other heteroaryl rings (e.g., benzo(b)thienyl, benzimidazole, indole, tetrahydroindole, azaindole, indazole, quinoline, imidazopyridine, purine, pyrrolo[2,3-d]pyrimidine, and pyrazolo[3,4-d]pyrimidine).

An aralkyl group, as used herein, is an aromatic substituent that is linked to a moiety by an aliphatic group preferably having from one to about six carbon atoms.

A heterocycloalkyl group, as used herein, is a non-aromatic ring system that preferably has 5 to 6 atoms and includes at least one heteroatom, such as nitrogen, oxygen, or sulfur. Examples of heterocycloalkyl groups include morpholines, piperidines, and piperazines.

Suitable substituents for aliphatic groups, aromatic groups, aralkyl groups, heteroaromatic groups and heterocycloalkyl groups include aromatic groups, halogenated aromatic groups, lower alkyl groups, halogenated lower alkyl (e.g. trifluoromethyl and trichloromethyl), -O-(aliphatic group or substituted aliphatic group), -O-(aromatic group or substituted aromatic group), benzyl, substituted benzyl, halogens, cyano, nitro, -S-(aliphatic or substituted aliphatic group), and -S-(aromatic or substituted aromatic).

Amine, alcohol and thiol protecting groups are known to those skilled in the art. For examples of amine protecting groups see Greene, *et al*., *Protective Groups in Organic Synthesis* (1991), John Wiley & Sons, Inc., pages 309-405. Preferably, amines are protected as amides or carbamates. For examples of alcohol protecting groups see *Id*., pages 10-142. A preferred alcohol protecting group is *t*-butyldimethylsilyl group. For examples of thiol protecting groups see *Id*., pages 277-308.

An acid labile protecting group is a protecting group which can be removed by contacting the group with a Bronsted or a Lewis acid. Acid labile protecting groups are known to those skilled in the art. Examples of common acid labile protecting groups include substituted or unsubstituted trityl groups (*Id*., pages 60-62), substituted or unsubstituted tetrahydropyranyl groups (*Id*., pages 31-34), substituted or unsubstituted tetrahydrofuranyl groups (*Id*., pages 36-37) or pixyl groups (*Id*., page 65). A preferred acid labile protecting group is a substituted or unsubstituted trityl, for example 4,4'-dimethoxytrityl (hereinafter "DMT"). Trityl groups are preferably substituted by electron donating substituents such as alkoxy groups.

Nucleoside bases include naturally occurring bases, such as adenine, guanine, cytosine, thymine, and uracil and modified bases such as 7-deazaguanine, 7-deaza-8-azaguanine, 5-propynylcytosine, 5-propynyluricil, 7-deazaadenine, 7-deaza-8-azaadenine, 7-deaza-6-oxopurine, 6-oxopurine, 3-deazaadenosine, 2-oxo-5-methylpyrimidine, 2-oxo-4-methylthio-5-methylpyrimidine, 2-thiocarbonyl-4-oxo-5-methylpyrimidine, 4-oxo-5-methylpyrimidine, 2-amino-purine, 5-fluorouricil, 2,6-diaminopurine, 8-aminopurine, 4-triazolo-5-methylthymine, and 4-triazolo-5-methyluricil.

A protected nucleoside base is a nucleoside base in which reactive functional groups of the base are protected. Typically, nucleoside bases have amine groups which can be protected with an amine protecting group, such as by the formation of an amide or a carbamate group. For example, the amine groups of adenine and cytosine are typically protected with benzoyl protecting groups, and the amine groups of guanine is typically protected with an isobutyryl group, an acetyl group or t-butylphenoxyacetyl group. However, other protection schemes may be used. For example, for fast deprotection, the amine groups of adenine and guanine are protected with phenoxyacetyl groups and the amine group of cytosine is protected with an isobutyryl group. When an oligonucleotide is synthesized from multimers of the present invention having protected nucleotide bases, conditions for removal of the protecting group will depend on the protecting group used. When an amino group protected as an amide group is used, it can be removed by treating the oligonucleotide with a base solution, such as a concentrated ammonium hydroxide solution, *n*-methylamine solution or a solution of *t*-butylamine in ammonium hydroxide.

References in this section to Structural Formulae will be understood to include the corresponding stereoisomers as appropriate.

A trivalent phosphorus multimer represented by Structural Formula I or II can be used to prepare an oligonucleotide represented by Structural Formula III. An advantage of using trivalent phosphorus multimers over using monomers is that fewer reaction steps are necessary. Therefore, longer oligonucleotides can be synthesized. It is expected that oligonucleotides longer than 150 nucleotide bases can be synthesized using trivalent phosphorus multimers. The multimers of the present invention can also be employed to synthesise shorter oligonucleotides, such as those comprising up to about 50 bases, for example from 8 to 35 bases.

The synthesis of the oligonucleotide can be done in solution or on a solid support. When the synthesis is in solution, R¹³ is an alcohol, amine or thiol protecting group. After synthesis of the oligonucleotide the alcohol, amine or thiol protecting group can be removed. When the oligonucleotide is synthesized on a solid support, R¹³ represents a solid support or -Y²-L-Y²-R¹⁵. In general, the solution phase synthesis or the solid phase synthesis of oligonucleotides using trivalent phosphorus multimers are carried out similar to method which have been developed for synthesis of oligonucleotides from monomers, except that when a multimer is used, the coupling step often takes about 25% to about 75% longer than the coupling step takes when a monomer is used. Examples of typical conditions for solution phase synthesis and solid phase synthesis oligonucleotides using trivalent phosphorus multimers are set forth below.

The first step of preparing the oligonucleotide involves coupling the trivalent phosphorus multimer with a 5'-deprotected nucleoside or nucleotide represented by Structural Formula IV. During the coupling reaction, the 5'-deprotected group of the nucleoside or nucleotide reacts with the multimer by displacing the -NR⁴R⁵ group. When the synthesis is done in solution, the 5'-deprotected nucleotide is often present in a concentration of about 0.02 M to about 2 M, and the multimer is preferably present in a concentration of about 1.1 equivalents to about 2 equivalents of the 5'-deprotected nucleoside or nucleotide. About 2.5 equivalents to about 5.0 equivalents, with respect to the 5'-deprdtected nucleoside, of nucleophilic coupling catalyst, such as tetrazole, S-ethylthiotetrazole, dicyanoimidazole or pyridinium salts, such as pyridinium chloride, is usually added to facilitate the coupling reaction. The reaction time is commonly about 20 min. to about 60 min.

A second step of preparing an oligonucleotide involves oxidizing or sulfurizing the trivalent phosphorus groups of the oligonucleotide. In this step, the trivalent phosphorus groups of the multimer which has been coupled to the 5'-deprotected nucleoside, as well as the newly formed trivalent phosphorus linkage between the 5'-deprotected nucleoside or nucleotide and the multimer, is oxidized or sulfurized.

In a solution phase synthesis, the oxidation reaction is often carried out by treating the oligonucleotide with an oxidizing agent, for example, I₂ in the presence of water or a peroxide such as *t*-butyl hydrogen peroxide in an organic solvent. When I₂ and water are used, the oxidizing solution typically contains about 1.1 to about 1.8 equivalents of I₂ in the presence of a base and a trace amount of water. The reaction is carried out in an aprotic polar solvent, such as THF, combined with a base, such as a tertiary alkylamine and about 1% water. The ratio of aprotic solvent to base is about 4 : 1 (vol./vol.) to about 1 : 4 (vol./vol.). After about 5 min. to about 20 min., the reaction mixture is poured into an aqueous solution of sodium bisulfite to quench the excess iodine, then extracted into an organic solvent.

Alternatively, the trivalent phosphorus groups can be sulfurized using any sulfur transfer reagent known to those skilled in the art for use in oligonucleotide synthesis. Examples of sulfur transfer agents include 3H-benzodithiol-3-one 1,1-dioxide (also called "Beaucage reagent"), dibenzoyl tetrasulfide, phenylacetyl disulfide, N,N,N',N'-tetraethylthiuram disulfide, 3-amino-[1,2,4]-dithiazole-5-thione (see US patent no. 6,096,881), or elemental sulfur. Examples of reaction conditions for sulfurization of an oligonucleotide using the above reagents can be found in Beaucage, *et al*., *Tetrahedron* (1993), *49*:6123. 3-amino-[1,2,4]-dithiazole-5-thione is a preferred sulfur transfer reagent. Generally, an oligonucleotide is contacted with a solution of 3-amino-[1,2,4]-dithiazole-5-thione in an organic solvent, such as pyridine/acetonitrile (1:9 w/w), having a concentration of about 0.04 M to about 0.2 M. The sulfurization reaction is complete in about 30 sec. to about 2 min.

After oxidation or sulfurization of the oligonucleotide, any unreacted 5'-deprotected nucleoside is often capped so that it cannot react in subsequent coupling steps. Capping failure sequences allows them to be more readily separated from full length oligonucleotide product. Any reagent which will react with the nucleophilic 5'-end group (i.e., -OH, -SH or -NH₂) and prevent it from reacting with a trivalent phosphorus multimer can be used as a capping reagent. Typically, an anhydride, such as acetic anhydride or isobutyric anhydride, or an acid chloride, such as acetyl chloride or isobutyryl chloride, in the presence of a base is used as a capping reagent.

After the capping reaction is complete, the 5'-protecting group, represented by R¹ is removed. When R₁ is an acid labile protecting group, R¹ is removed by treating the oligonucleotide with an acid. Preferably, the 5'-protecting group is a trityl group, such as 4,4'-dimethoxytrityl. When the 5'-protecting group is a trityl group, it can be removed by treating the oligonucleotide with a solution of dichloroacetic acid or trichloroacetic acid in an organic solvent, such as dichloromethane. Once the 5'-protecting group has been removed, the reaction cycle (i.e., coupling step, oxidation or sulfurization step, capping step (optional) and deprotection step) optionally can be repeated one or more times to obtain an oligonucleotide of the desired length.

A phosphate oligonucleotide can be prepared by oxidising the trivalent phosphorus groups and by selection of the internucleotide phosphorus protecting groups (such as represented by R³) such that when the internucleotide groups are deprotected, phosphate groups are formed. For example, oxidation of a beta-cyanoethyloxy protected trivalent phosphorus followed by basic deprotection would form a phosphate group.

A phosphorothioate oligonucleotide can be prepared by sulfurizing the trivalent phosphorus groups or by oxidising the trivalent phosphorus groups and by selection of the internucleotide phosphorus protecting groups (such as represented by R³) such that when the internucleotide groups are deprotected, phosphorothioate groups are formed. For example, oxidation of a beta-cyanoethylthio protected trivalent phosphorus followed by anhydrous basic deprotection would form a phosphorothioate group.

A chimeric oligonucleotide can be prepared by oxidizing the trivalent phosphorus groups in one or more reaction cycles and sulfurizing the trivalent phosphorus groups in one or more different reaction cycles, combined with appropriate selection of the internucleotide phosphorus protecting groups (such as represented by R³) to form the desired phosphate or phosphorothioate linkage. Alternatively, a chimeric oligonucleotide can be prepared by selecting multimers in which some of the internucleotide protecting groups form phosphorothioate linkages on deprotection, such as beta-cyanoethylthio protecting groups, and some of the internucleotide protecting groups form phosphate linkages on deprotection, such as beta-cyanoethyloxy protecting groups. In this method, the oligonucleotide is oxidized after the coupling step in each reaction cycle.

When it is desired to obtain an oligonucleotide product in which the 5'-end group is protected, the final step of the reaction cycle can be the capping step, if a capping step is done, or the final step of the reaction can be an oxidation or sulfurization step if a capping step is not done. If a 5'-deprotected oligonucleotide is desired, the reaction cycle can end with the deprotection step. Usually, a 5'-protected oligonucleotide is the desired product if the oligonucleotide is to be purified by reverse phase high performance liquid chromatography (HPLC). If the oligonucleotide is to be purified by ion-exchange chromatography, a 5'-deprotected oligonucleotide is usually the desired product.

The solid phase synthesis of an oligonucleotide using trivalent phosphorus multimers generally utilizes the same reaction cycle and reagents as the solution phase synthesis. However, the 5'-deprotected nucleoside or nucleotide is loaded on the solid support, where loading is often about 50 µmole to about 100 µmole per gram of support. In the coupling step, a solution of trivalent phosphorus multimer, typically having a concentration of about 0.01 M to about 1 M, preferably about 0.1 M, in an organic solvent, such as acetonitrile, is added to the solid support. A solution of the nucleophilic coupling catalyst having a concentration of about 1.5 mmol to about 1.5 M, is usually mixed with the solution containing the multimer just prior to or during the coupling reaction. Then the support bound 5'-deprotected nucleoside or nucleotide is contacted with the mixture for about 2 min. to about 10 min., preferably about 5 min.

If the trivalent phosphorus linkages are to be oxidized after the coupling reaction is complete, the solid support is contacted with an oxidizing agent such as a mixture of I₂ and water or a peroxide such as *t*-butyl hydroperoxide in an organic solvent such as THF, acetonitrile or toluene. A mixture of I₂ and H₂O is a preferred oxidizing reagent. When a mixture of I₂ and water is used other water miscible organic solvents can also be present. Typically, the solid support bound oligonucleotide containing trivalent phosphorus internucleotide linkages can be contacted with a solution of I₂ in a solvent mixture of water, an aprotic, water miscible solvent, and a base. An example of a typical oxidation solution is about 0.05 M to about 1.5 M I₂ in a solution of (2:80:20) water/tetrahydrofuran/lutidine (vol./vol./vol.). The solid support is typically treated with the I₂ solution for about 30 seconds to about 1.5 min.

Alternatively, the solid support can be contacted with a solution of a sulfur transfer reagent in an organic solvent to sulfurize the trivalent phosphorus groups. For example, the solid support can be contacted with a solution of 3*H*-benzodithiol-3-one-1,1-dioxide (about 0.05 M-0.2 M) in an organic solvent, such acetonitrile, for about 30 sec. to about 1 min.

In solid phase oligonucleotide synthesis, the solid support optionally can be contacted with a solution of the capping reagent for about 30 sec. to about 1 min. Following the capping step, the deprotection step is accomplished by contacting the solid support with an acid solution for about 1 min. to about 3 min. The reaction cycle can optionally be repeated one or more times until an oligonucleotide of the desired length is synthesized. As in the solution phase synthesis, a 5'-protected oligonucleotide is obtained when the reaction cycle ends with either the capping step or the oxidation or sulfurization step. A 5'-deprotected oligonucleotide is obtained when the reaction cycle is ended with the deprotection step.

When the solid phase synthesis is completed, the oligonucleotide can be removed from the solid support by standard methods. Generally, the solid support is treated with a solution of concentrated ammonium hydroxide at 25°C-60°C for about 0.5 hours to about 16 hours or longer depending on the oligonucleotide sequence and whether it is desired to remove the nucleobase protecting groups during this step.

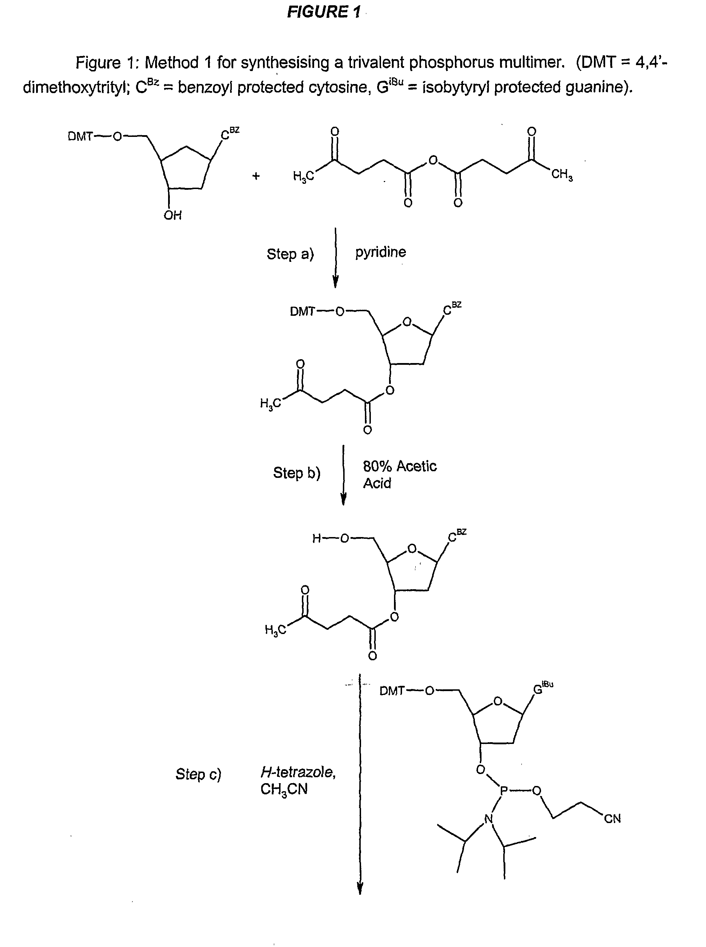

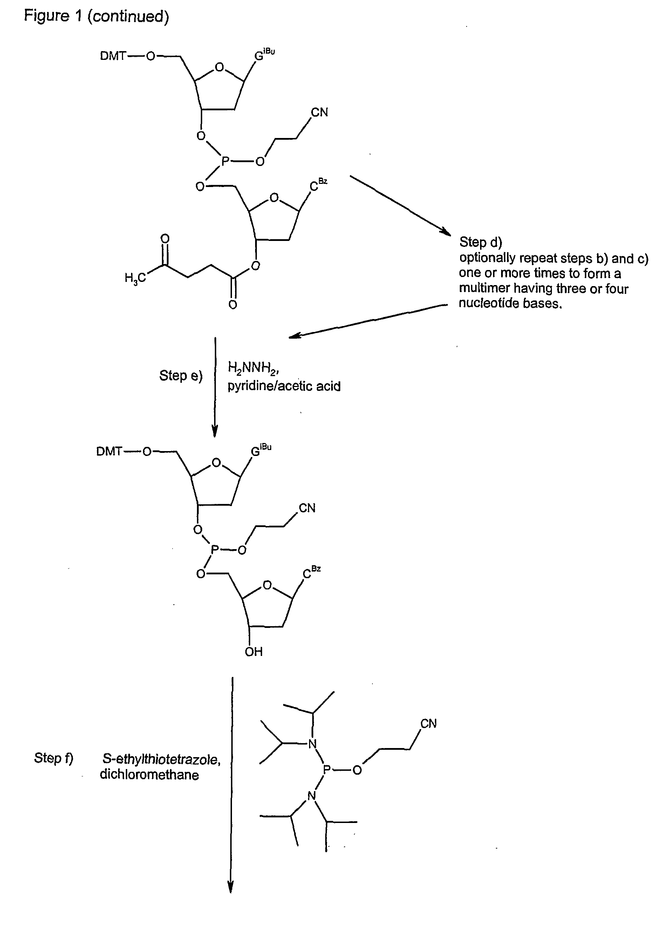

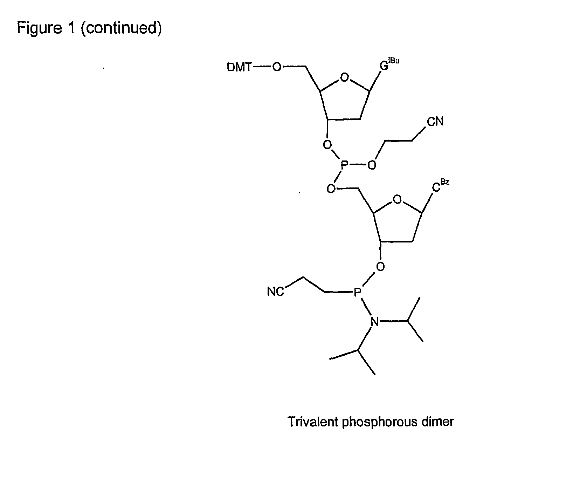

The trivalent phosphorus multimers can be prepared by two different methods. In the first method (for an example of this method see Figure 1), a nucleoside represented by Structural Formula VII is protected to form a first intermediate represented by Structural Formula VIII. Preferably, the protecting group is a levulynoyl protecting group or a 3-benzoylpropionyl protecting group. The levulynoyl protecting group can be added to the nucleoside by several different methods (see Greene, *et al*., *Protective Groups in Organic Synthesis* (1991), John Wiley & Sons, Inc., page 97). A preferred method for protecting the nucleoside with a levulynoyl or 3-benzoylpropionyl protecting group is by treating it with levulinic anhydride or 3-benzoylpropionic anhydride in pyridine for about 16-24 hours. Alternatively, the levulynoyl or 3-benzoylpropionyl protecting group can be added by treating the nucleoside with levulinic acid or 3-benzoylpropionic acid and dicyclohexylcarbodiimide in a solution of dichloromethane and pyridine.

The first intermediate is then treated to remove R₁ to form a 5'-deprotected nucleoside. Methods for removing the trityl group are listed in Greene, *et al*., *Protective Groups in Organic Synthesis* (1991), John Wiley & Sons, Inc., pages 60-62. Methods for removing a tetrahydropyranyl protecting group (pages 31-34) and tetrahydrofuranyl protecting group (pages 36-37) can be found in Greene, *et al*., *Protective Groups in Organic Synthesis* (1991), John Wiley & Sons, Inc. A trityl group is preferably removed by treating the first intermediate with an aqueous solution of 80% (vol./vol.) acetic acid in water for about 7 hours, or by treatment with about 2% trifluoroacetic acid or dichloroacetic acid in dichloromethane for about 15 minutes.

The 5'-deprotected nucleoside can then be coupled with a phosphoramidite represented by Structural Formula IX to form a dimer represented by Structural Formula X. The coupling reaction is typically carried out in an organic solvent under dry reaction conditions. The concentration of the reactants is about 0.05 M to about 0.5 M. A coupling catalyst, such as *H*-tetrazole or *S*-ethylthiotetrazole, is also present in the reaction mixture, often in about 2 equivalents to about 6 equivalents with respect to the 5'-deprotected nucleoside. The coupling reaction typically takes about 2 hours to about 16 hours.

The 3',5'-protected dimer is then treated to remove R¹⁶ to form a 3'-deprotected dimer. When R¹⁶ is a levulynoyl protecting group, it can be removed by the methods set forth in Greene, *et al*., *Protective Groups in Organic Synthesis* (1991), John Wiley & Sons, Inc., p. 97. Preferably, the levulynoyl or 3-benzoylpropionyl protecting group is removed by contacting the 3',5'-protected dimer with a solution of about 0.05 M to about 1 M hydrazine in pyridine/acetic acid in a ratio of about 4 : 1 (vol./vol.) to about 1 : 4 (vol./vol.) for about 15 min. to about 1 hour.

The 3'-deprotected dimer is optionally reacted in the presence of a coupling catalyst with a compound represented by Structural Formula XI. This coupling reaction is carried out in an organic solvent under dry reaction conditions. The concentration of the 3'-deprotected dimer and the compound represented by Structural Formula XI is about 0.05M to about 0.5M. A coupling catalyst, such as *H*-tetrazole or *S*-ethylthiotetrazole, is present in about 2 equivalents to about 6 equivalents with respect to the 3'-deprotected dimer. A 3',5'-protected trimer represented by Structural Formula XII is the product formed by the reaction.

The R¹⁶ deprotection reaction and the coupling reaction can optionally be repeated one or more times using the 3',5'-protected trimer as the starting material to form a 3',5'-protected multimer.

The 3',5'-protected multimer is then treated to remove R¹⁶ to form a 3'-deprotected multimer.

The 3'-deprotected multimer is reacted with a phosphorylating reagent to form the trivalent phosphorus multimer represented by Structural Formula I. A phosphorylating reagent represented by Structural Formula XIIIa or Xlllb is added slowly to a dry solution of the 3'-deprotected multimer in an organic solvent, such as dichloromethane, acetonitrile or THF. When the phosphorylating reagent is a compound represented by Structural Formula Xlllb, an aprotic organic base, such as triethylamine or diisopropylethylamine, is also added to the reaction mixture. The phosphorylating reagent is often present in an excess of about 1.1 equivalents to about 1.5 equivalents with respect to the multimer. The reaction is allowed to proceed for about 4 hours to about 24 hours. After the reaction is complete, n-octanol optionally can be added to the reaction mixture to react with any remaining phosphorylating reagent. Preferably, multimers are dimers (two nucleotide bases) or trimers (three nucleotide bases).

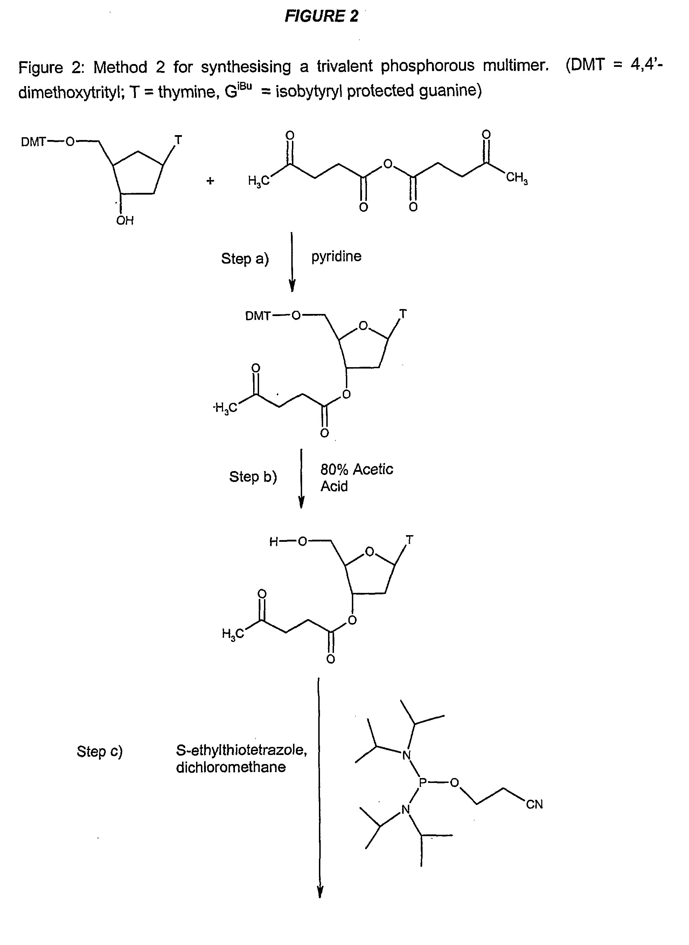

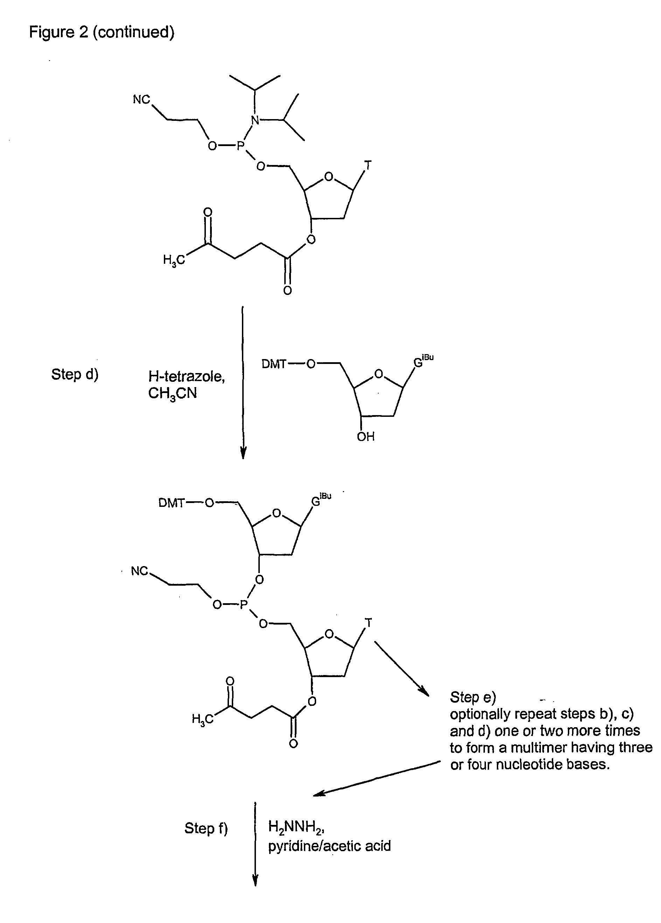

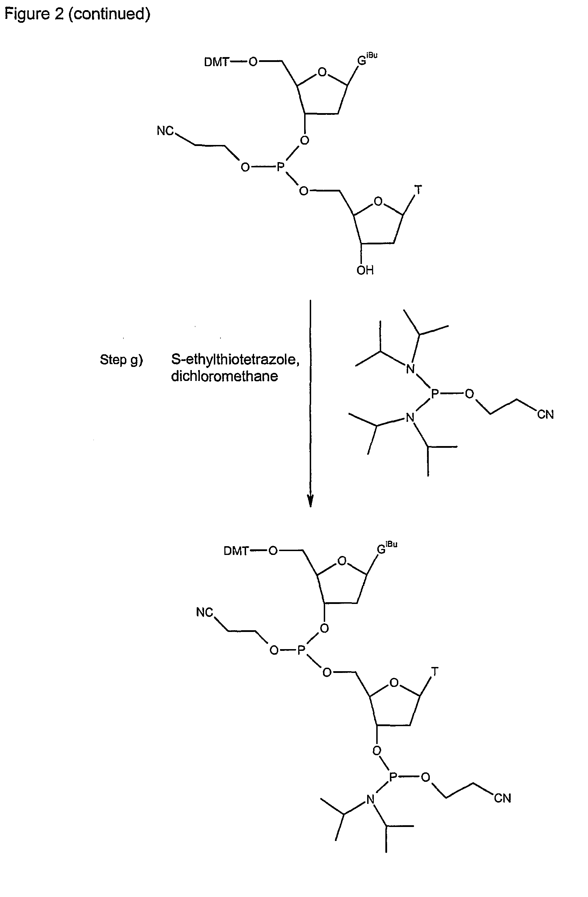

In the second method of preparing a trivalent phosphorus multimer (for an example see Figure 2), a nucleoside base represented by Structural Formula VII is protected with R¹⁶, a protecting group which is orthogonal to R¹, to form a first intermediate represented by Structural Formula VIII. Preferably, R¹⁶ is a levulynoyl or a 3-benzoylpropionyl protecting group. The first intermediate is treated to remove R¹ in the same manner as in the first method of preparing a trivalent phosphorus multimer. The 5'-deprotected nucleoside formed by this reaction sequence is treated with a phosphorylating reagent represented by Structural Formula XIIIa or XIIIb to form a 5'-phosphoramidite nucleoside represented by Structural Formula XI. The 5'-phosphorylation conditions are the same as described for the 3'-phosphorylation conditions of the first method of forming a trivalent phosphorus multimer. The 5'-phosphoramidite is then coupled with a compound represented by Structural Formula VII in the presence of a coupling catalyst to form a 3',5'-protected dimer represented by Structural Formula X. The reaction conditions for the coupling reaction are the same as for the coupling reaction in the first method of forming the multimer. The 3',5'-protected dimer is treated to remove R¹⁶ as described above to form a 3'-deprotected dimer. Also as described above, the 3'-deprotected dimer can optionally be reacted in the presence of a coupling catalyst with a compound represented by Structural Formula XI to form a 3',5'-protected trimer represented by Structural Formula XII. The 3'-deprotection reaction and the coupling reaction can be optionally repeated one or more times to form a 3',5'-protected multimer. R₁₆ is removed from the 3',5'-protected multimer to form a 3'-deprotected multimer, and the 3'-deprotected multimer is phosphorylated as described above to form a trivalent phosphorus multimer represented by Structural Formula I.

A solid support which is derivatized with a trivalent phosphorus multimer represented by Structural Formula XII can be prepared by reacting the 3'-deprotected multimer, formed by the method described above, with a compound represented by Structural Formula XVa or XVb to form a solid support loading reagent. When the 3'-deprotected multimer is reacted with a compound represented by Structural Formula XVa, the reaction is carried out in the presence of a base, such as pyridine. When the 3'-deprotected multimer is reacted with a compound represented by Structural Formula XVb, at least about 1 equivalent of a dialiphatic carbodiimide, with respect to the compound represented by Structural Formula XVb, should also be present in the reaction mixture. The solid support loading reagent optionally can be activated by reacting it with a compound which will transform the terminal carboxylic acid into an active ester. Examples of compounds which will react with the terminal carboxylic acid to form an active ester include *p*-nitrophenol, *o,p*-dinitrophenol, imidazole, and N-hydroxysuccinamide. In the reaction to form an active ester with *p*-nitrophenol or *o,p*-dinitrophenol, an aliphatic carbodiimide should also be present in the reaction mixture.

The solid support loading reagent or the activated solid support loading reagent can be utilized to derivatize an amine functionalized solid support. Amine functional groups on the solid support can be primary or secondary amines. The amine functionalized support is contacted with the activated solid support loading reagent in a basic solution to form the derivatized solid support. For an example of reaction conditions see U.S. Patent No. 5,668,268. Alternatively, the amine functionalized solid support is contacted with the unactivated loading reagent in the presence of a dialiphatic carbodiimide in a basic solution to derivatize the solid support. For an example of reaction conditions see U.S. Patent No. 5,668,268 and U.S. Patent No. 4,812,512. Preferred dialiphatic carbodiimides are dicyclohexyl carbodiimide and diisopropyl carbodiimide.

### EXAMPLES

Phosphoramidite dimers were prepared using the procedures set forth in Examples 1 and 2. A procedure for preparing phosphoramidite trimers is set forth in Example 3. Oligonucleotides having phosphodiester and/or phosphorothioate linkages were prepared using the method detailed in Example 4. In Examples 1-4, all parts and percentages are by weight unless otherwise specified.

### EXAMPLE 1 (see Figure 1):

### A. Synthesis of 2'-deoxy-N⁴-benzoyl-3'-O-levulynoyl-cytidine:

The title compound was prepared from commercially available 2'-deoxy-N⁴-benzoyl-5'-O-DMT-cytidine. 2'-deoxy-N⁴-benzoyl-5'-O-DMT-cytidine was treated at room temperature for 5 hrs. with levulinic acid anhydride (1.5 eq.) in dry pyridine to give 2'-deoxy-N⁴-benzoyl-3'-O-levulynoyl-5'-O-DMT-cytidine. The 5'-O-DMT protecting group was removed by subjecting 2'-deoxy-N⁴-benzoyl-3'-O-levulynoyl-5'-O-DMT-cytidine to 80% (vol./vol.) acetic acid in water for 1.5 hrs. at room temperature to yield 2'-deoxy-N⁴-benzoyl-3'-O-levulynoyl-cytidine. The product thus obtained was purified by silica gel column chromatography.

### B. Synthesis of 2'-deoxy-5'-O-DMT-N²-isobutyryl-guanosine-3'-O-P-(OCH₂CH₂CN)-5'-O-N⁴-benzoyl-2'deoxycytidine-3'-O-levulinate:

N⁴-Benzoyl-2'-deoxycytidine-3'-O-levulinate (8.58g, 20mmol) synthesized in step A and tetrazole (0.56g, 80mmol) were dried by co-evaporation with dry toluene under reduced pressure then dissolved in dry acetonitrile (100 mL). Commercially available N²-isobutyryl-5'-O-DMT-2'deoxyguanosine-3'-O-phosphoramidite (16.0g, 19mmol) was added to this solution and the solution was allowed to stir under argon for 4hr at room temperature. The solution was concentrated to a viscous mass, then taken up in ethyl acetate saturated with argon gas. This solution was washed with a cold sodium bicarbonate solution followed by cold water. After drying over anhydrous sodium sulfate, the solution was concentrated under reduced pressure to light yellow foam. The product was purified by column chromatography to yield fully protected dimer nucleosides phosphite triester (19.5g) which was characterized by NMR.

### C. Synthesis of 2'-deoxy-5'-O-DMT-N²-isobutyryl-guanosine-3'-O-P-(OCH₂CH₂CN)-5'-O-N⁴-benzoyl-2'deoxycytidine-3'-OH:

The fully protected di-nucleosides phosphite triester (17.5g, 15mmol) synthesized in step B was treated with hydrazine solution (0.5M, 100ml) in pyridine/acetic acid (4:1 vol./vol.) for 30 min at room temperature. Pyridine (100ml) was added to the solution, and the mixture was concentrated under reduced pressure. The product was taken up in dichloromethane (500ml), then carefully washed with sodium bicarbonate solution (2 x 300 ml) and then with water (300 ml). The organic layer was dried over anhydrous sodium sulfate. The solution was filtered and concentrated under reduced pressure to solid foam. The solid foam was then purified by silica gel chromatography to yield the desired product (13.4g, 83.4%).

### D. Synthesis of 2'-deoxy-5'-O-DMT-N²-isobutyryl-guanosine-3'-O-P-(OCH₂CH₂CN)-5'-O-N⁴-benzoyl-2'deoxycytidine-3'-O-β-cyanoethyl-(N,N-diisopropylamino)-phosphoramidite:

The 5'-O-DMT-N²-isobutyryl-2'-deoxyguanosine-3'-O-P-(OCH₂CH₂CN)-5'-O-N⁴-benzoyl-2'deoxycytidine-3-OH (12.5g, 11.7mmol) synthesized in step C was dried by co-evaporating with acetonitrile under reduced pressure. The dried material was dissolved in distilled dichloromethane. Bis-(N,N-diisopropylamino)-β-cyanoethoxyphosphine (4.5g, 15mmol) was added to the solution using a syringe, and S-ethylthiotetrazole (1.6g, 12.5mmol) was added as a solid. After stirring at room temperature for 16hr, n-octanol (500 l) was added, and the solution was stirred for another 2 hr. The reaction mixture was concentrated to a viscous liquid which was dissolved in ethyl acetate (500ml), then washed with a sodium bicarbonate solution followed by an sodium chloride solution. The ethyl acetate solution was drying over anhydrous sodium sulfate, then concentrated to a solid. The purification of the crude product was performed by silica gel column chromatography, which afforded desired dimer phosphoramidite (12.8g, 86.2%).

### EXAMPLE 2:

### A. Synthesis of 2'-deoxy-2'-O-methyl-3'-O-levulynoyl-uridine:

2'-Deoxy-2'-O-methyl-5'-O-dimethoxytrityl-uridine (11.2g, 20mmol) was dried by co-evaporation with pyridine (3 x 100 ml) then dissolved in dry pyridine (150ml). Levulinic acid anhydride (6.5g, 30mmol) and N,N-dimethylaminopyridine (0.1g, catalytic amount) were added and the solution was allowed to stir at room temperature for 5 hrs. When no starting material, as evaluated by tlc, remained in the solution, the solution was concentrated down to a viscous mass, then taken up in ethyl acetate. The ethyl acetate solution was washed with a sodium bicarbonate solution followed by a sodium chloride solution. The ethyl acetate layer was dried over sodium sulfate, then concentrated to foam to give 2'-deoxy-2'-O-methyl-3'-O-levulynoyl-5'-O-dimethoxytrityl-uridine was characterized by proton NMR. This material was used in the next step without purification.

2'-Deoxy-2'-O-methyl-3'-O-levulynoyl-5'-O-dimethoxytrityl-uridine (14.0g) was then treated with 80% (vol./vol.) acetic acid solution in water (500 ml) for 1.5 hrs at room temperature. Pyridine (400 mL) was added to this solution, and the mixture was concentrated to viscous liquid under reduced pressure. The viscous mass was triturated with ether to provide a gummy solid, which was purified by silica gel column chromatography using ethyl acetate with increasing percentage of methanol. The desired fractions were pooled and concentrated to a solid. 2'-Deoxy-2'-O-methyl-3'-O-levulynoyluridine crystallized from a methylene chloride and ether mixture. The product was characterized by proton NMR.

### B. Synthesis of 2'-deoxy-2'-O-methyl-5'-O-DMT-uridine-3'-O-P-(OCH₂CH₂CN)-5'-O-uridine-2'-deoxy-2'-O-methyl-3'-O-levulinate (coupling reaction):

2'-Deoxy-2'-O-methyl-3'-O-levuiynoyl-uridine (5.0g, 14.0mmol) synthesized in step A and tetrazole were dissolved in dry acetonitrile and co-evaporated under reduced pressure. The dried material was pumped down with a high vacuum pump for 1 hr then dissolved in acetonitrile (70ml). 2'-Deoxy-2'-O-methyl-5'-O-DMT-uridine-3'-phosphoramidite (9.5g, 12.5mmol) was added to the solution and the reaction mixture was allowed to stir for 16 hr at room temperature. The organic solvent was removed under reduced pressure, and residue was taken up in ethyl acetate which has been saturated with argon. The solution was washed with a solution of sodium bicarbonate followed by a sodium chloride solution. The organic layer was dried over anhydrous sodium sulfate, then concentrated after under reduced pressure. The crude product was purified by silica gel chromatography using ethyl acetate and increasing percentage of methanol. The desired pooled fractions were combined and concentrated to yield 9.5 g of the fully protected dimer. Finally the product was characterized by proton and phosphorus NMR.

### C. Synthesis of 2'-deoxy-2'-O-methyl-5'-O-DMT-uridine-3'-O-P-(OCH₂CH₂CN)-5'-O-uridine-2'-deoxy-2'-O-methyl (removal of 3'-O-levulynoyl group):

The fully protected di-nucleoside phosphite triester (8.0g, 8.75 mmol) synthesized in step B was treated with a solution of hydrazine hydrate (0.5 M, 50 ml) in pyridine: acetic acid (4:1) for 30 min at room temperature. Pyridine (50ml) was added to the solution, then the mixture was concentrated to a viscous liquid. This material was then taken up in chloroform, then washed with a sodium bicarbonate solution and water. The chloroform extract was dried over sodium sulfate, then evaporated to dryness. The crude material thus obtained was purified by silica gel column chromatography to give the 3'-deprotected dimer (6.5g, 8.0mmol). This was characterized by proton and phosphorus NMR.

### D. Synthesis of 2'-deoxy-2'-O-methyl-5'-O-DMT-uridine-3'-O-P-(OCH₂CH₂CN)-5'-O-uridine-2'-deoxy-2'-O-methyl-3'-O-β-cyanoethyl-(N,N-diisopropylamino)-phosphoramidite (Dimer nucleoside phosphoramidite synthon):

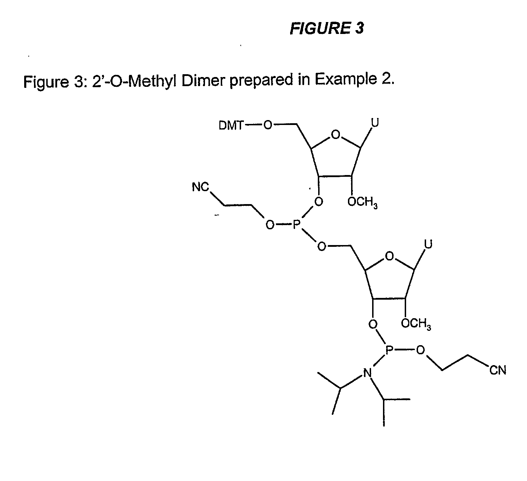

The 3'-deprotected dinucleosides phosphite triester (6.1g, 7.46mmol) synthesized in step C was dried by co-evaporating with dry acetonitrile under reduced pressure. The dried material was dissolved in freshly distilled and dry methylene chloride (50ml). Phosphitylating reagent bis-(N,N-diisopropylamino)-(3-cyanoethoxylphosphine (3.4ml, 11.2mmol) was added to the solution, followed by S-ethylthiotetrazole (0.75g, 6.0mmol), and the reaction mixture was stirred for 16 hr at room temperature. n-octanol (0.13ml) was added to the reaction mixture to react with excess phosphitylating reagent, and the reaction was stirred for another 2 hrs. Finally, the reaction mixture was diluted with 5% diisopropylethyl amine in chloroform (vol./vol.) (350ml) and washed immediately with a sodium bicarbonate solution (2 x 150 ml) and a sodium chloride solution (150 ml). The chloroform layer was dried over sodium sulfate, then concentrated to a solid. The dimer phosphoramidite product (see Figure 3) was purified by silica gel chromatography using 0.1% (vol./vol.) diisopropylethyl amine in ethyl acetate. 6.5 g of the desired pooled fractions was obtained in 98% purity by P-NMR.

### EXAMPLE 3: Synthesis of trimer phosphoramidites:

A trimer phosphoramidite can be prepared by the reacting a phosphoramidite dimer nucleoside (e.g., the compound formed in Example 1D or 2D) with a 5'-hydroxy-2'-deoxy-3'-O-levulynoyl-nucleoside or a 5'-hydroxy-2'-O-protected-3'-O-levulynoylnucleoside in presence of 4.0 eq. of tetrazole in acetonitrile using reaction conditions similar to those given in Example 1B or 2B. Levulynoyl from 3'-end of the trimer-nucleoside can be removed by using hydrazine solution in pyridine and acetic acid using reaction conditions similar to those given in Example 1C or 2C. After purification, the 3'-deprotected trimer nucleosides with two phosphite triester linkages can be phosphitylated with bis-(N,N-diisopropylamino)-β-cyanoethoxy-phosphine and S-ethyl thio-tetrazole using reaction conditions similar to those given in Example 1D or 2D to give the phosphoramidite of trimer-nucleoside with two phosphite triester linkages.

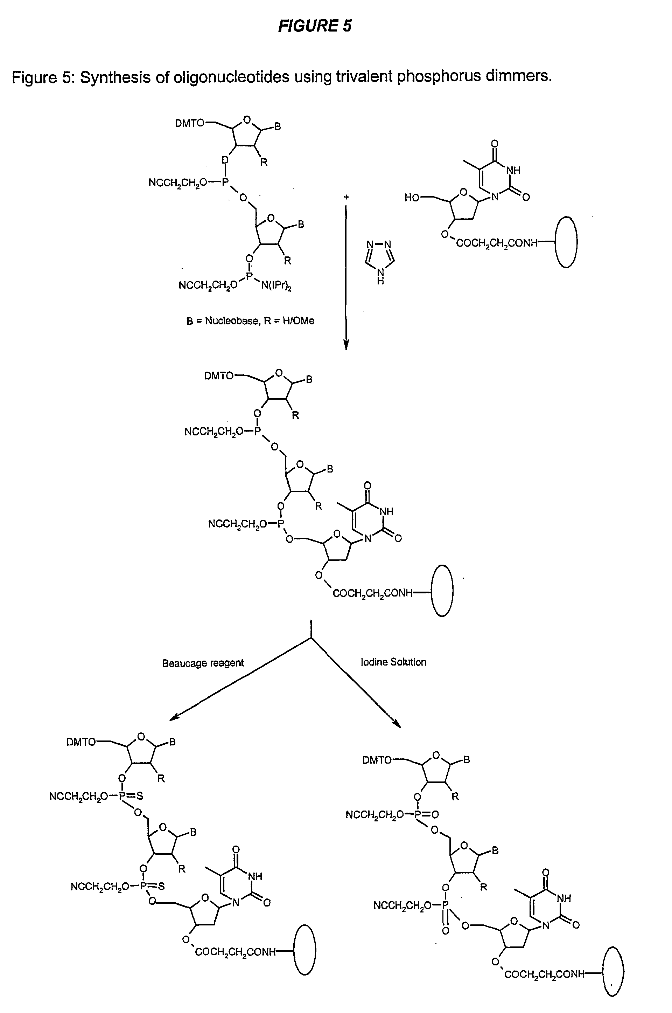

### EXAMPLE 4: Synthesis of an oligonucleotide having both phosphate diester and phosphorothioate diester linkages (see Figure 5):

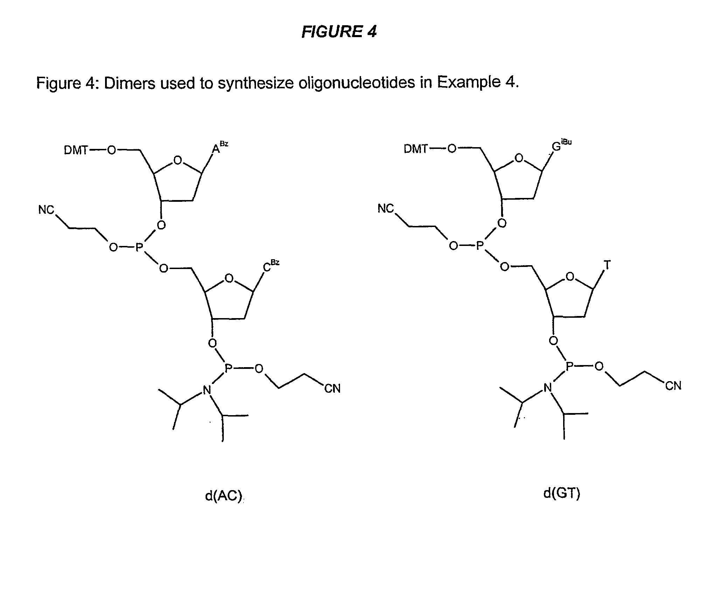

Synthesis of the oligonucleotide was carried out on a DNA synthesizer 8909 Expedite (Applied Biosystems). The standard phosphoramidite chemistry protocol was followed for the synthesis with slight modifications. The concentration of phosphoramidite dimer nucleosides having phosphite triester linkages was 0.1M in acetonitrile. The dimers used for the synthesis are shown in Figure 4. The coupling time used for chain elongation using dimer phosphoramidites were 50% longer compared to monomer phosphoramidites. After coupling phosphite triester linkages were converted either to stable phosphate triester with iodine solution or to stable phosphorothioate with Beaucage reagent. Ten reaction cycles were performed in each synthesis to form an oligonucleotide with 21 nucleotide bases. A phosphate 21mer having the sequence ACACACACACACACACACACT (SEQ ID No. 1) and two phosphorothioate 21mers having the sequences ACACACACACACACACACACT (SEQ ID No. 1) and GTGTGTGTGTGTGTGTGTGTT (SEQ ID No. 2) were prepared. At the end of the synthesis, solid supports linked with fully protected oligonucleotide chain was treated with concentrated ammonium hydroxide for 16 hr at 50°C in order to release the chain and to remove the β-cyanoethyl protecting groups and the nucleoside base protecting groups. The crude oligonucleotides were analyzed by ion exchange HPLC. The main peak in the chromatogram was the desired product. In the case of oligonucleotide with phosphorothioated diester linkage, the crude product was also analyzed by phosphorus NMR. The spectra gave the characteristic chemical shift for phosphorothioate diester and no chemical shift for phosphate diester linkage could be seen.

## Claims

1. A phosphoramidite compound comprising two or more nucleoside moieties linked by one or more internucleoside phosphorus atoms, wherein the internucleoside phosphorus atoms are phosphorus (III) atoms comprising a phosphite triester group and the phosphoramidite moiety is a group of formula -X¹-PR³NR⁴R⁵ wherein X¹ represents O; R³ represents a beta-cyanoethyloxy, beta-cyanoethylthio, 4-cyanobut-2-enylthio, 4-cyanobut-2-enyloxy, allylthio, allyloxy, crotylthio, or crotyloxy group; and R⁴ and R⁵ are each, independently, a substituted or unsubstituted aliphatic group or together with the nitrogen to which they are bound form a heterocycloalkyl group.

2. A phosphoramidite compound according to claim 1 which comprises a phosphoramidite moiety bonded to the 3'-position of the nucleoside moiety carrying the phosphoramidite moiety.

3. A phosphoramidite compound according to either of claims 1 and 2, wherein R³ represents a beta-cyanoethyloxy group.

4. A phosphoramidite compound according to claim 3, wherein R⁴ and R⁵ represent isopropyl groups.

5. A phosphoramidite compound according to claim 1, wherein the phosphite triester group links the 5'-position of a nucleoside moiety carrying the phosphoramidite moiety with the 3'-position of a second nucleoside moiety.

6. A compound according to claim 1 having the following structural formula, or a stereoisomer thereof: 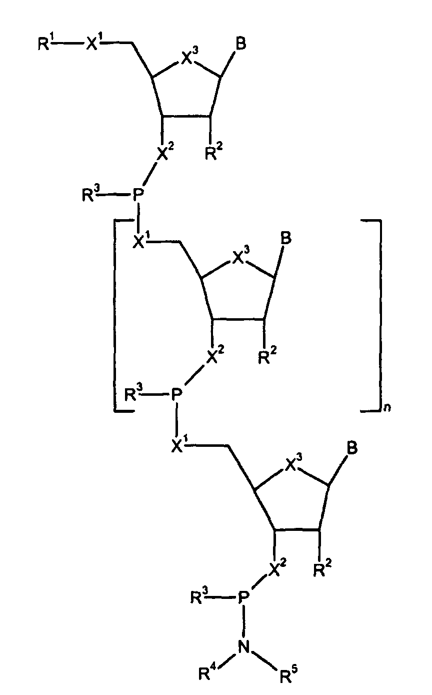 wherein:
each X¹ is -O-;
each X² is -O-;
each X³ is, independently, -O-, -S-, -CH₂-, or -(CH₂)₂-;
R¹ is a protecting group;
each R² is, independently, -H, -F, -NHR⁶, -CH₂R⁸ or -OR⁶;
each R³ is, independently, a beta-cyanoethyloxy, beta-cyanoethylthio, 4-cyanobut-2-enylthio, 4-cyanobut-2-enyloxy, allylthio, allyloxy, crotylthio, or crotyloxy group;
R⁴ and R⁵ are each, independently, a substituted or unsubstituted aliphatic group or, together with the nitrogen to which they are bound form a heterocycloalkyl group;
R⁶ is -H, a substituted or unsubstituted aliphatic group, a substituted or unsubstituted aromatic group, a substituted or unsubstituted aralkyl, or a protecting group;
each B is, independently, H or a protected or an unprotected nucleoside base;
and
n is 0 or a positive integer.

7. The compound of Claim 6, wherein each X³ is -O-.

8. The compound of Claims 6 or 7, wherein R³ is -OCH₂CH₂CN or -SCH₂CH₂CN.

9. The compound of Claims 6, 7 or 8, wherein R¹ is an acid labile protecting group.

10. The compound of Claims 6, 7, 8 or 9, wherein R² is -H.

11. The compound of Claims 6, 7, 8 or 9, wherein R² is -OR⁶ and R⁶ is Me, -CH₂CH₂OMe or a hydroxy protecting group.

12. A compound according to claim 6, having the following structural formula, or a stereoisomer thereof: 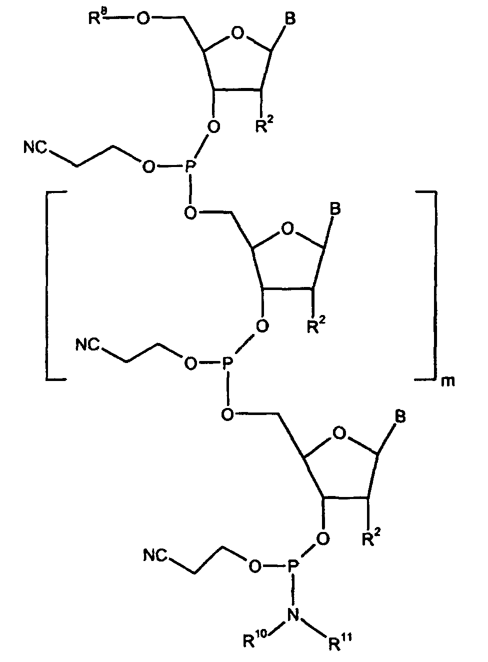 wherein:
R² is -H or -OR⁶;
R⁶ is -H, a substituted or unsubstituted aliphatic group, a substituted or unsubstituted aromatic group, a substituted or unsubstituted aralkyl, or an alcohol protecting group;
R⁸ is a substituted or unsubstituted trityl;
R¹⁰ and R¹¹ are each, independently a substituted or unsubstituted aliphatic group;
each B is, independently, H or a protected or an unprotected nucleoside base;
and
m is 0 or 1.

13. The compound of Claim 12, wherein:
R⁸ is 4,4'-dimethoxytrityl;
R² is -H; and
R¹⁰ and R¹¹ are isopropyl.

14. The compound of Claim 12, wherein:
R⁸ is 4,4'-dimethoxytrityl;
R² is -OR⁶;
R¹⁰ and R¹¹ are isopropyl; and
R⁶ is Me, -CH₂CH₂OMe, *t*-butyldimethylsilyl, tetrahydropyranyl, 4-methoxytetrahydropyranyl or Fpmp.

15. A method of preparing an oligonucleotide represented by the following structural formula, or a stereoisomer thereof: 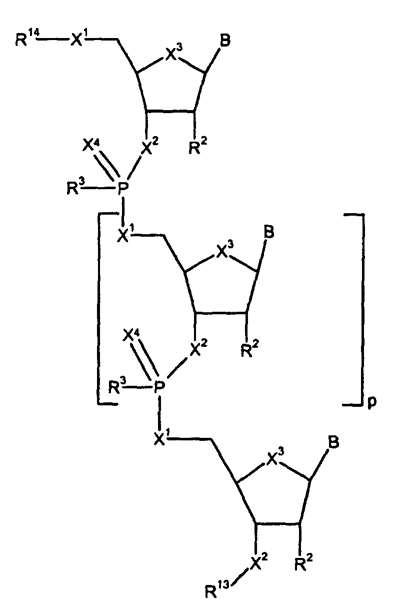 wherein:
each X¹ is -O-;
each X² is -O-;
each X³ is, independently, -O-, -S-, -CH₂-, or -(CH₂)₂-;
each X⁴ is, independently, =O or =S;
each R² is, independently, -H, -F, -NHR⁶, -CH₂R⁶ or -OR⁶;
R⁶ is -H, a substituted or unsubstituted aliphatic group, a substituted or unsubstituted aromatic group, a substituted or unsubstituted aralkyl, or a protecting group;
each R³ is, independently, a beta-cyanoethyloxy, beta-cyanoethylthio, 4-cyanobut-2-enylthio, 4-cyanobut-2-enyloxy, allylthio, allyloxy, crotylthio, or crotyloxy group;
R¹³ is an alcohol protecting group, a group of the formula -Y²-L-Y¹, a group of the formula -Y²-L-Y²-R¹⁵ or a solid support;
R¹⁴ is -H or a protecting group;
R¹⁵ is a solid support;
each B is, independently, H or a protected or an unprotected nucleoside base;
p is a positive integer;
Y¹ is an ester or a carboxylic acid group;
Y² is a single bond, -C(O)-, -C(O)NR¹⁷-, -C(O)O-, -NR¹⁷- or -O-;
L is a substituted or unsubstituted aliphatic group or a substituted or unsubstituted aromatic group; and
R¹⁷ is -H, a substituted or unsubstituted aliphatic group or a substituted or unsubstituted aromatic group, comprising the steps of:
a) coupling a trivalent phosphorus multimer represented by the following structural formula, or a stereoisomer thereof: 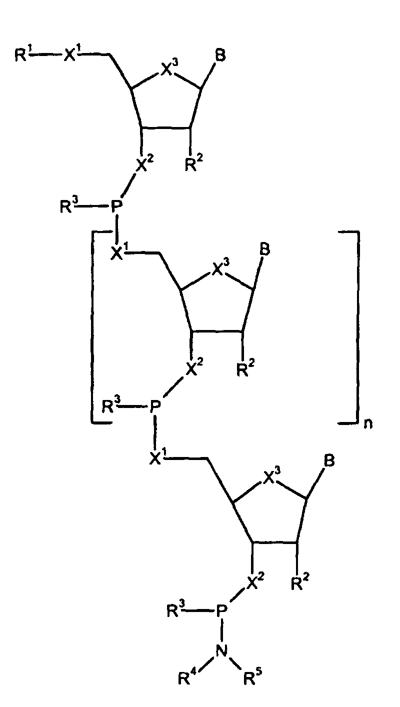 wherein:
R¹ is a protecting group;
R⁴ and R⁵ are each, independently, a substituted or unsubstituted aliphatic group, or R⁴ and R⁵ taken together with the nitrogen to which they are bound form a heterocycloalkyl group; and
n is 0 or a positive integer,
with a 5'-deprotected nucleoside or oligonucleotide represented by the following structural formula, or a stereoisomer thereof: 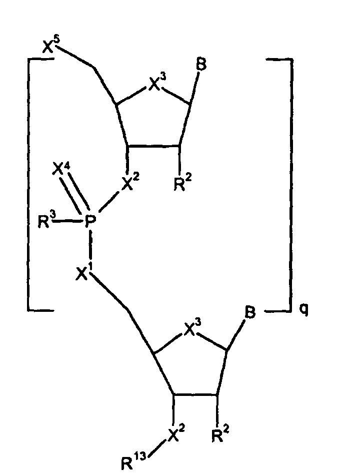 wherein:
X⁵ is -OH; and
q is 0 or a positive integer, to produce a first intermediate represented by the following structural formula, or a stereoisomer thereof: 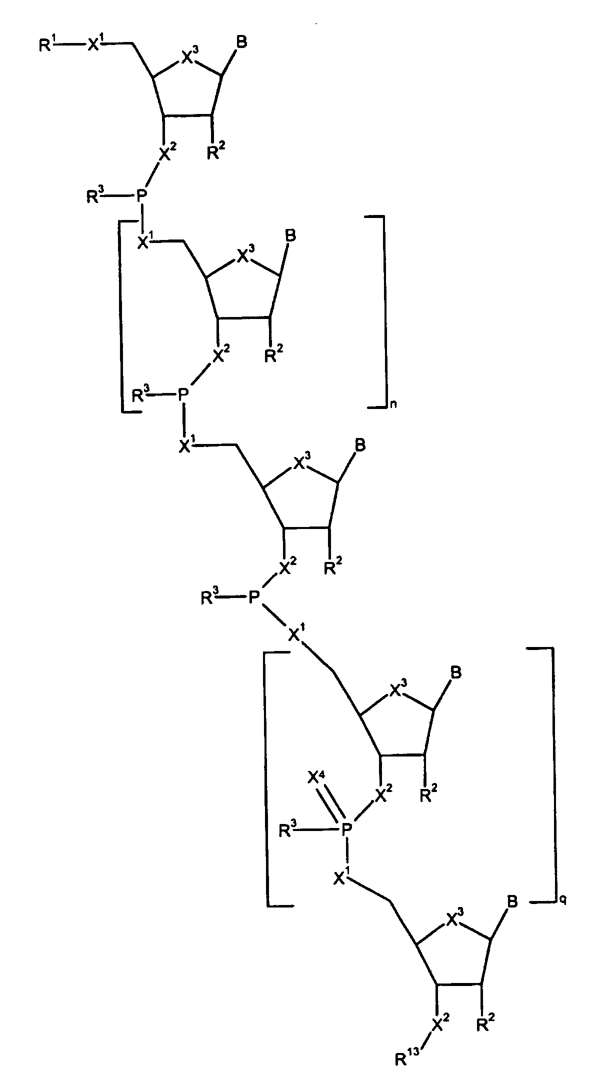
b) oxidizing or sulfurizing the trivalent phosphorus groups in the first intermediate to form a second intermediate represented by the following structural formula, or a stereoisomer thereof: 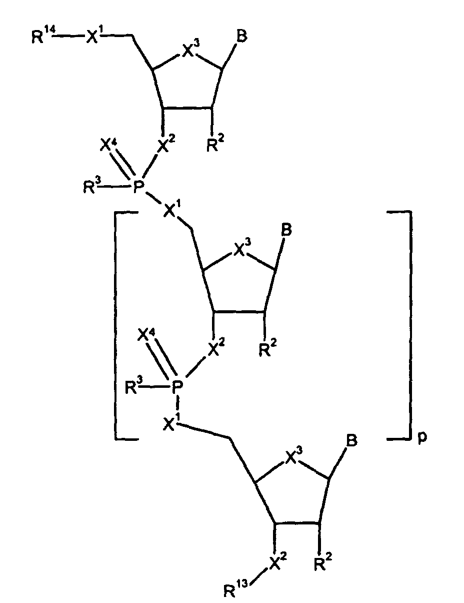
c) optionally capping X⁵ groups which did not react with the trivalent phosphorus multimer in step a);
d) treating the second intermediate to remove R¹ to form a 5'- deprotected oligonucleotide; and
e) optionally repeating steps a)-d) one or more times, wherein the final step is step c) or step d), thereby preparing an oligonucleotide.

16. The method of Claim 15, wherein R¹³ is a solid support.

17. The method of Claim 16, further comprising the step of cleaving the oligonucleotide product from the solid support.

18. The method of Claims 15, 16 or 17, further comprising deprotecting the nucleotide bases of the oligonucleotide.

19. The method of Claim 15, wherein R¹³ is an alcohol protecting group.

20. The method of Claim 19, further comprising the step of removing the R¹³ protecting group from the oligonucleotide product.

21. The method of Claims 15, 16, 17, 18, 19 or 20, wherein R¹ is an unsubstituted trityl, a monoalkoxytrityl, a dialkoxytrityl, a trialkoxytrityl, tetrahydropyranyl or a pixyl group.

22. The method of Claim 21, wherein R¹ is removed with an acid selected from a solution of dichloroacetic acid in dichloromethane and a solution of trichloroacetic acid in dichloromethane.

23. The method of any one of Claims 15 to 21, wherein each R³ is -OCH₂CH₂CN or -SCH₂CH₂CN.

24. The method of Claim 23, further comprising removing -CH₂CH₂CN from -OCH₂CH₂CN or -SCH₂CH₂CN by treating the oligonucleotide with a base.

25. The method of Claim 24, wherein the nucleotide bases are deprotected during the treatment of the oligonucleotide with a base.

26. The method of any one of Claims 15 to 25, wherein the trivalent phosphorus groups are oxidized by treating first intermediate with a solution containing I₂ and water.

27. The method of any one of Claims 15 to 25, wherein the trivalent phosphorus groups are sulfurized by treating the first intermediate with 3-amino-[1,2,4]-dithiazole-5-thione or 3*H*-benzodithiol-3-one 1,1-dioxide.

28. The method of any one of Claims 15 to 25, wherein more than one cycle of steps a), b), c) and d) is performed, and the oligonucleotide produced is a chimeric oligonucleotide.

29. The method of any one of Claims 15 to 26, wherein the oligonucleotide produced is a phosphate.

30. The method of any one of Claims 15 to 27, wherein the oligonucleotide produced is a phosphorothioate.

31. The method of any one of Claims 15 to 30, wherein the oligonucleotide prepared has up to 50 nucleotide bases.

32. A method of preparing a trivalent phosphorus multimer represented by the following structural formula, or a stereoisomer thereof: 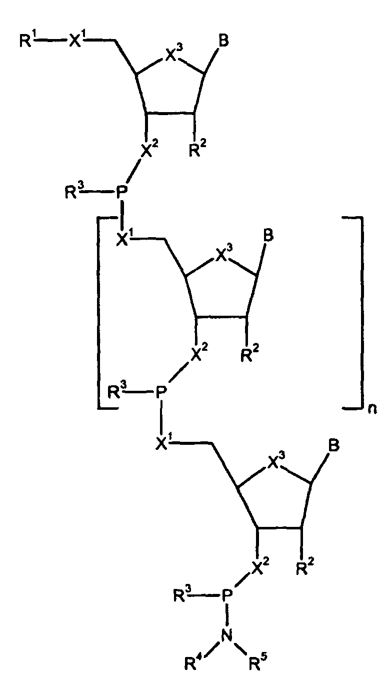 wherein:
each X¹ is -O-;
each X² is, -O-;
each X³ is, independently, -O-, -S-, -CH₂-, or -(CH₂)₂-;
R¹ is a protecting group;
each R² is, independently, -H, -F, -NHR⁶, -CH₂R⁶ or -OR⁶;
each R³ is, independently, a beta-cyanoethyloxy, beta-cyanoethylthio, 4-cyanobut-2-enylthio, 4-cyanobut-2-enyloxy, allylthio, allyloxy, crotylthio, or crotyloxy group;
R⁴ and R⁵ are each, independently, a substituted or unsubstituted aliphatic group or R⁴ and R⁵ taken together with the nitrogen to which they are bound form a heterocycloalkyl group;
R⁶ is -H, a substituted or unsubstituted aliphatic group, a substituted or unsubstituted aromatic group, a substituted or unsubstituted aralkyl, or a protecting group;
each B is, independently, H or a protected or an unprotected nucleoside base; and
n is 0 or a positive integer, comprising the steps of:
a) protecting the 3'-substituent of a nucleoside represented by the following structural formula, or a stereoisomer thereof: 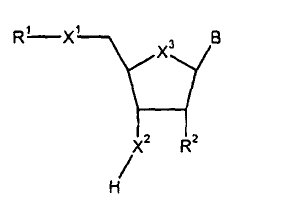 thereby forming a first intermediate represented by the following structural formula, or a stereoisomer thereof: 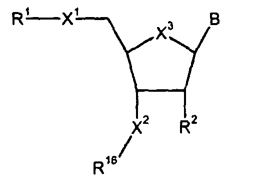 wherein R¹⁶ is a protecting group which is orthogonal to R¹;
b) treating the first intermediate to remove R¹, thereby forming a 5'-deprotected nucleoside;
c) reacting the 5'-deprotected nucleoside in the presence of a coupling catalyst with a compound represented by the following structural formula, or a stereoisomer thereof: 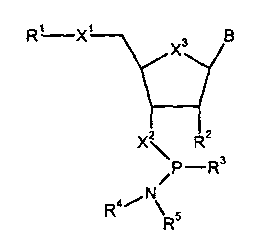 thereby forming a 3',5'-protected multimer represented by the following structural formula, or a stereoisomer thereof: 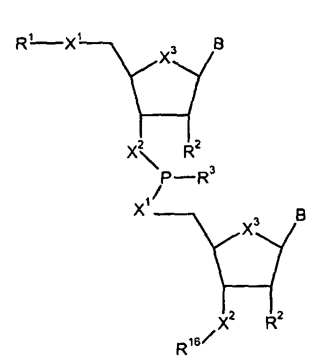
d) optionally treating the 3',5'-protected multimer to remove R¹⁶, thereby forming a 3'-deprotected multimer; then
e) reacting in the presence of a coupling catalyst the 3'-deprotected multimer with a compound represented by the following structural formula, or a stereoisomer thereof: 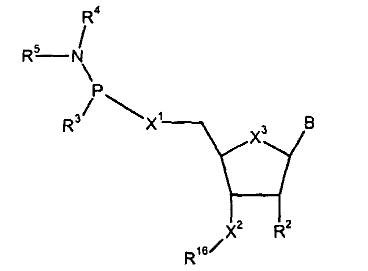 thereby forming a 3',5'-protected compound represented by the following structural formula, or a stereoisomer thereof: 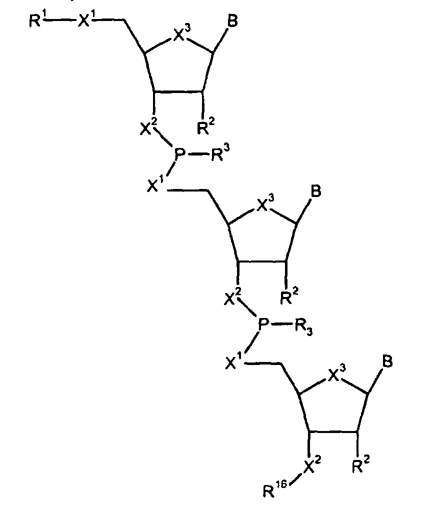
f) optionally repeating steps d) and e) one or more times, thereby forming an extended 3',5'-protected multimer;
g) treating the 3',5'-protected multimer to remove R¹⁶, thereby forming a 3'-deprotected multimer;
h) reacting the 3'-deprotected multimer with a trivalent phosphorus compound represented by one of the following structural formulas: 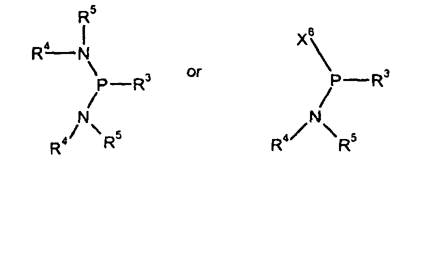
wherein X⁶ is a halogen, thereby forming the trivalent phosphorus multimer.

33. The method of Claim 32, wherein the coupling catalyst is tetrazole or S-ethylthiotetrazole.

34. The method of Claims 32 or 33, wherein R¹⁶ is levulynoyl and is removed by treating the 3',5'-protected multimer with hydrazine hydrate in a pyridine/acetic acid.

35. A method of preparing a trivalent phosphorus multimer represented by the following structural formula, or a stereoisomer thereof: 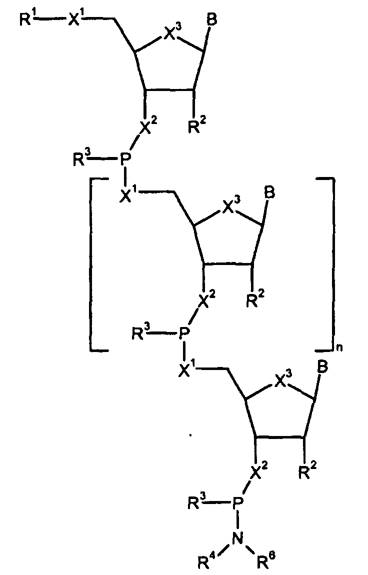 wherein:
each X¹ is, independently, -O-;
each X² is, independently, -O-;
each X³ is, independently, -O-, -S-, -CH₂-, or -(CH₂)₂-;
R¹ is a protecting group;
each R² is, independently, -H, -F, -NHR⁶, -CH₂R⁶ or -OR⁶;
each R³ is, independently, a beta-cyanoethyloxy, beta-cyanoethylthio, 4-cyanobut-2-enylthio, 4-cyanobut-2-enyloxy, allylthio, allyloxy, crotylthio, or crotyloxy group;
R⁴ and R⁵ are each, independently, a substituted or unsubstituted aliphatic group, or R⁴ and R⁵ taken together with the nitrogen to which they are bound form a heterocycloalkyl group;
R⁶ is -H, a substituted or unsubstituted aliphatic group, a substituted or unsubstituted aromatic group, a substituted or unsubstituted aralkyl, or a protecting group;
each B is, independently, H or a protected or an unprotected nucleoside base; and
n is 0 or a positive integer, comprising the steps of:
a) protecting the 3'-substituent of a nucleoside represented by the following structural formula, or a stereoisomer thereof: 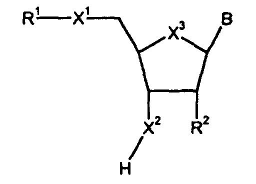 thereby forming a first intermediate represented by the following structural formula, or a stereoisomer thereof: 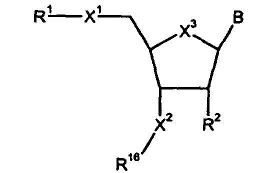 wherein R¹⁶ is a protecting group which is orthogonal to R¹;
b) treating the first intermediate to remove R¹, thereby forming a 5'-deprotected nucleoside;
c) reacting the 5'-deprotected nucleoside with a trivalent phosphorus compound represented by one of the following structural formulas: 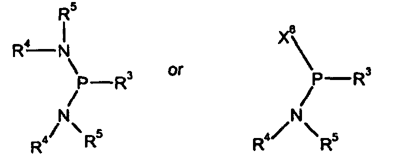 wherein X⁶ is a halogen, thereby forming a 5'-phosphoramidite;
d) reacting the 5'-phosphoramidite in the presence of a coupling catalyst with a compound represented by the following structural formula, or a stereoisomer thereof: 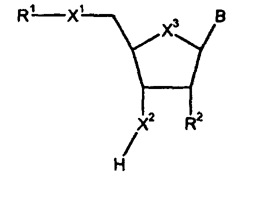 thereby forming a 3',5'-protected multimer represented by the following structural formula, or a stereoisomer thereof: 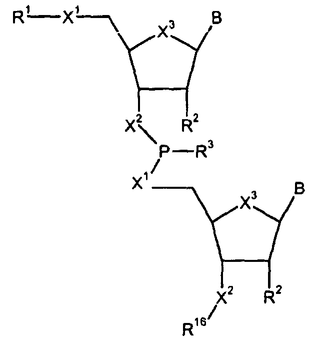
e) optionally, treating the 3',5'-protected multimer to remove R¹⁶, thereby forming a 3'-deprotected multimer; then
f) reacting in the presence of a coupling catalyst the 3'-deprotected multimer with a compound represented by the following structural formula, or a stereoisomer thereof: 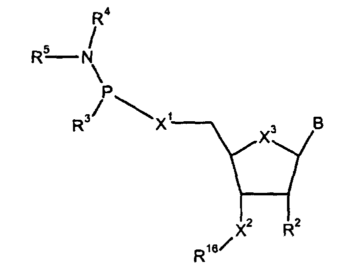 thereby forming a 3',5'-protected compound represented by the following structural formula, or a stereoisomer thereof: 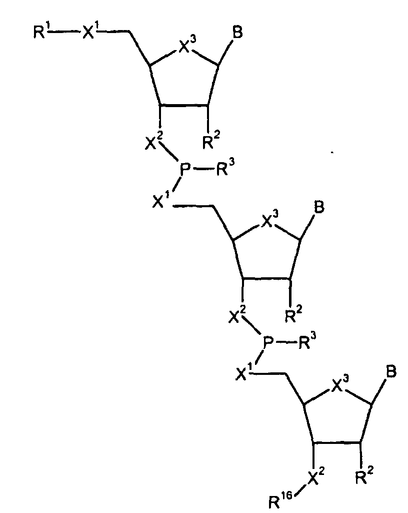
g) optionally repeating steps e) and f) one or more times, thereby forming an extended 3',5'-protected multimer;
h) treating the 3',5'-protected multimer to remove R¹⁶, thereby forming a 3'-deprotected multimer;
i) reacting the 3'-deprotected multimer with a trivalent phosphorus compound represented by one of the following structural formulas: 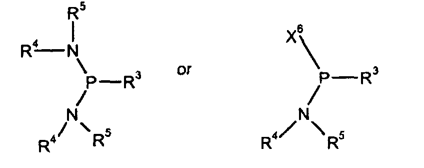
wherein X⁶ is a halogen, thereby forming the trivalent phosphorus multimer.

36. The method of Claim 35, wherein the coupling catalyst is tetrazole or S-ethylthiotetrazole.

37. The method of Claims 30 or 36, wherein R¹⁶ is levulynoyl and is removed by treating the 3',5'-protected multimer with hydrazine hydrate in a pyridine/acetic acid.

38. A trivalent phosphorus multimer derivatized solid support represented by the following structural formula, or a stereoisomer thereof: 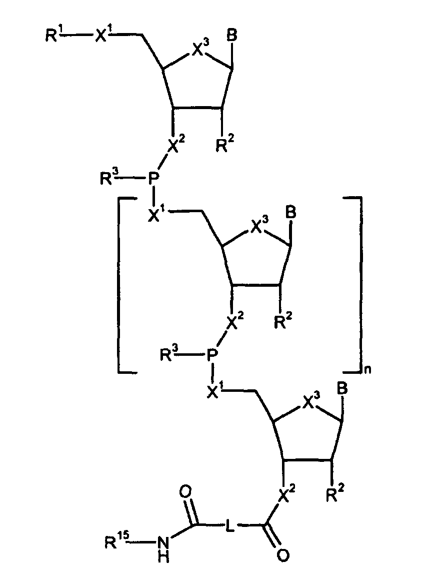 wherein:
each X¹ is, independently, -O-;
each X² is, independently, -O-;
each X³ is, independently, -O-, -S-, -CH₂-, or -(CH₂)₂-;
R¹ is a protecting group;
each R² is, independently, -H, -F, -NHR⁶, -CH₂R⁶ or -OR⁶;
each R³ is, independently, a beta-cyanoethyloxy, beta-cyanoethylthio, 4-cyanobut-2-enylthio, 4-cyanobut-2-enyloxy, allylthio, allyloxy, crotylthio, or crotyloxy group;
R⁶ is -H, a substituted or unsubstituted aliphatic group, a substituted or unsubstituted aromatic group, a substituted or unsubstituted aralkyl, or a protecting group;
each B is, independently, H or a protected or an unprotected nucleoside base;
L is a substituted or unsubstituted aliphatic group or a substituted or unsubstituted aromatic group;
n is a positive integer; and
R¹⁵ is a solid support.

39. The solid support of Claim 38, wherein each X³ is -O-.

40. The solid support of Claims 38 or 39, wherein R³ is -OCH₂CH₂CN.

41. The solid support of Claims 38. 39 or 40, wherein R¹ is an acid labile protecting group.

42. The solid support of Claim 41, wherein R¹ is 4,4'-dimethoxytrityl.

43. The solid support of Claims 38, 39, 40, 41 or 42, wherein R² is -H.

44. The solid support of Claims 38, 39, 40, 41 or 42, wherein R₂ is -OR⁶ and R⁶ is a hydroxy protecting group.

45. The solid support of any one of Claims 38 to 44, wherein L is -CH₂CH₂-.

46. The solid support of any one of Claims 38 to 45, wherein R¹⁵ comprises controlled-pore glass, polystyrene or microporous polyamide.

47. A method of preparing a multimer derivatized solid support represented by the following structural formula, or a stereoisomer thereof: 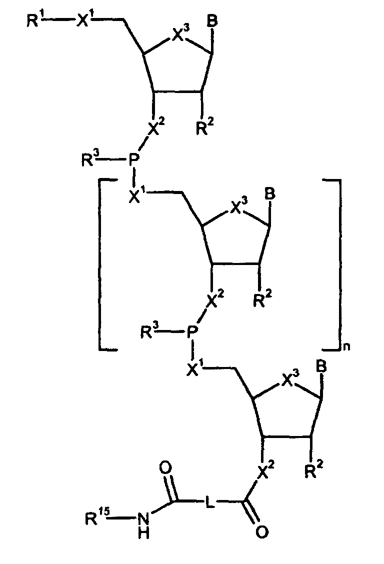 wherein:
each X¹ is -O-;
each X² is -O-;
each X³ is, independently, -O-, -S-, -CH₂-, or -(CH₂)₂-;
R¹ is a protecting group;
each R² is, independently, -H, -F, -NHR⁶, -CH₂R⁶ or -OR⁶;
each R³ is, independently, a beta-cyanoethyloxy, beta-cyanoethylthio, 4-cyanobut-2-enylthio, 4-cyanobut-2-enyloxy, allylthio, allyloxy, crotylthio, or crotyloxy group;
R⁶ is -H, a substituted or unsubstituted aliphatic group, a substituted or unsubstituted aromatic group, a substituted or unsubstituted aralkyl, or a protecting group;
each B is, independently, H or a protected or an unprotected nucleoside base;
L is a substituted or unsubstituted aliphatic group or a substituted or unsubstituted aromatic group;
n is 0 or a positive integer; and
R¹⁵ is a solid support, comprising the steps of:
a) protecting a 3'-substituent of a nucleoside represented by the following structural formula, or a stereoisomer thereof: 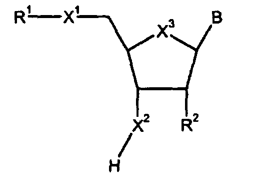 thereby forming a first intermediate represented by the following structural formula, or a stereoisomer thereof: 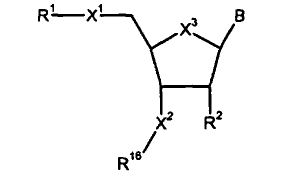 wherein R¹⁶ is a protecting group which is orthogonal to R¹;
b) treating the first intermediate to remove R¹, thereby forming a 5'-deprotected nucleoside;
c) reacting the 5'-deprotected nucleoside in the presence of a coupling catalyst with a compound represented by the following structural formula, or a stereoisomer thereof: 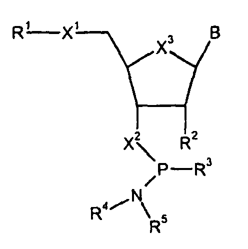 wherein:
R⁴ and R⁵ are each, independently, a substituted or unsubstituted aliphatic group, or R⁴ and R⁵ taken together with the nitrogen to which they are bound form a heterocycloalkyl group; thereby forming a 3',5'-protected multimer represented by the following structural formula, or a stereoisomer thereof: 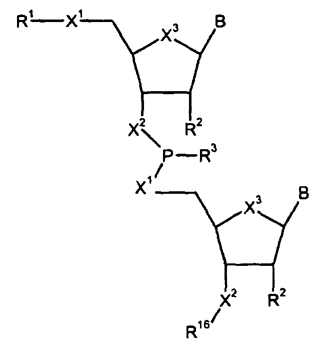
d) optionally treating the 3',5'-protected multimer to remove R¹⁶, thereby forming a 3'-deprotected multimer; then
e) reacting in the presence of a coupling catalyst the 3'-deprotected multimer with a compound represented by the following structural formula, or a stereoisomer thereof: 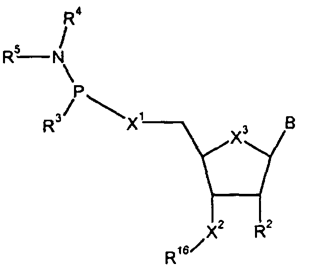 thereby forming a 3',5'-protected compound represented by the following structural formula, or a stereoisomer thereof: 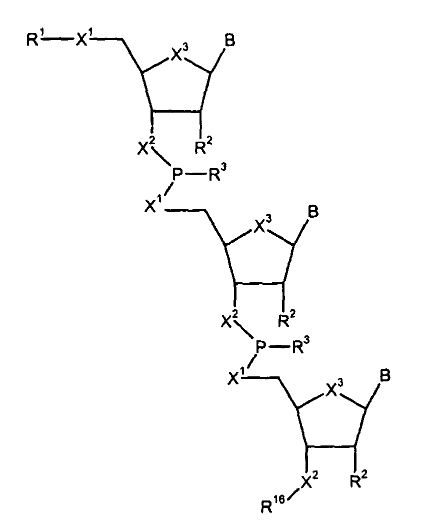
f) optionally repeating steps d) and e) one or more times, thereby forming an extended 3',5'-protected multimer;
g) treating the 3',5'-protected multimer to remove R¹⁶, thereby forming a 3'-deprotected multimer;
h) reacting the 3'-deprotected multimer in the presence of a base with a compound selected from the group consisting of: 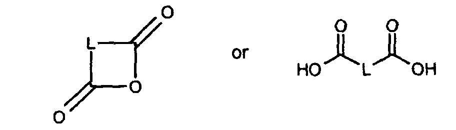 thereby forming a solid support loading reagent represented by the following structural formula, or a stereoisomer thereof: 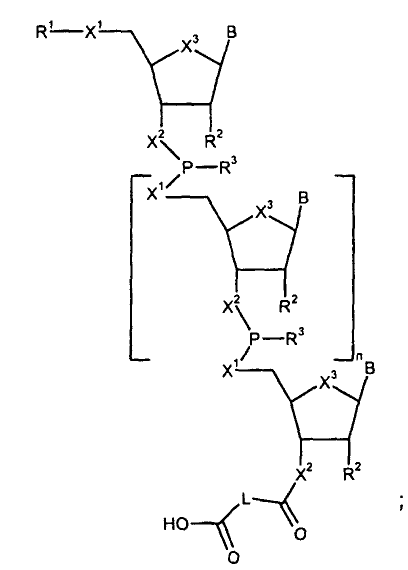 and
i) reacting the solid support loading reagent with a solid support functionalized with primary or secondary amine groups in the presence of a base and a substituted or unsubstituted dialiphatic carbodiimide, thereby preparing said multimer derivatized solid support.

48. The method of Claim 47, further comprising the step of reacting the solid support loading reagent formed in step h) with p-nitrophenol in the presence of a base and a substituted or unsubstituted dialiphatic carbodiimide, thereby forming an activated solid support loading reagent.

49. The method of Claim 48, wherein the substituted or unsubstituted dialiphatic carbodiimide is dicyclohexyl carbodiimide or diisopropyl carbodiimide.

50. A method of preparing a multimer derivatized solid support represented by the following structural formula, or a stereoisomer thereof: 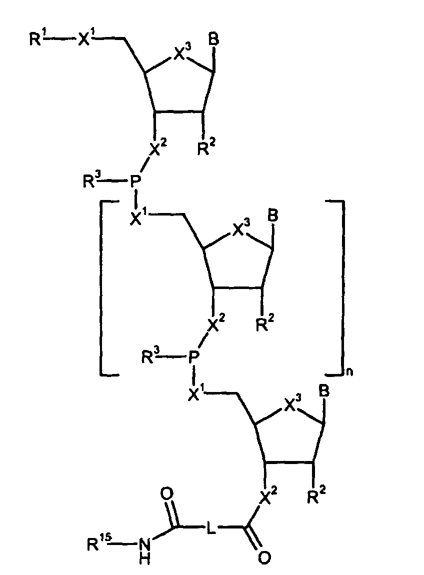 wherein:
each X¹ is, independently, -O-;
each X² is, independently, -O-;
each X³ is, independently, -O-, -S-, -CH₂-, or -(CH₂)₂-;
R¹ is a protecting group;
each R² is, independently, -H, -F, -NHR⁶, -CH₂R⁶ or -OR⁶;
each R³ is, independently, a beta-cyanoethyloxy, beta-cyanoethylthio, 4-cyanobut-2-enylthio, 4-cyanobut-2-enyloxy, allylthio, allyloxy, crotylthio, or crotyloxy group;
R⁶ is -H, a substituted or unsubstituted aliphatic group, a substituted or unsubstituted aromatic group, a substituted or unsubstituted aralkyl, or a protecting group;
each B is, independently, H or a protected or an unprotected nucleoside base;
L is a substituted or unsubstituted aliphatic group or a substituted or unsubstituted aromatic group;
n is 0 or a positive integer; and
R¹⁵ is a solid support, comprising the steps of:
a) protecting the 3'-substituent of a nucleoside represented by the following structural formula, or a stereoisomer thereof: 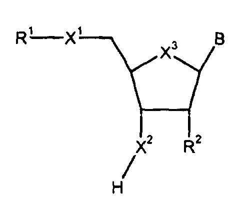 thereby forming a first intermediate represented by the following structural formula, or a stereoisomer thereof: 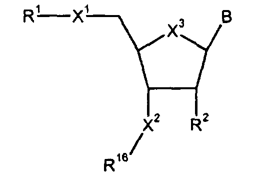 wherein R¹⁶ is a protecting group which is orthogonal to R¹;
b) treating first intermediate with an acid to remove R¹, thereby forming a 5'-deprotected nucleoside;
c) reacting the 5'-deprotected nucleoside with a trivalent phosphorus compound represented by one of the following structural formulas: 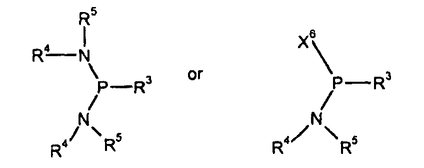 wherein:
X⁶ is a halogen, thereby forming a 5'-phosphoramidite, thereby forming a 5'-phosphoramidite; and
R⁴ and R⁵ are each, independently, a substituted or unsubstituted aliphatic group, or R⁴ and R⁵ taken together with the nitrogen to which they are bound form a heterocycloalkyl group;
d) reacting the 5'-phosphoramidite in the presence or a coupling catalyst with a compound represented by the following structural formula, or a stereoisomer thereof: 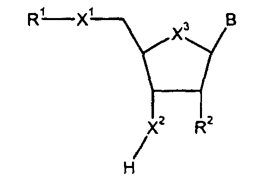 thereby forming a 3',5'-protected multimer represented by the following structural formula, or a stereoisomer thereof: 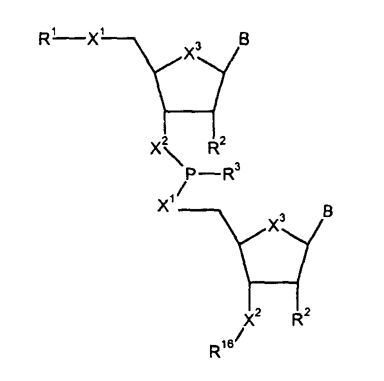
e) optionally treating the 3',5'-protected multimer to remove R¹⁶, thereby forming a 3'-deprotected multimer; then
f) reacting in the presence of a coupling catalyst the 3'-deprotected multimer with a compound represented by the following structural formula, or a stereoisomer thereof: 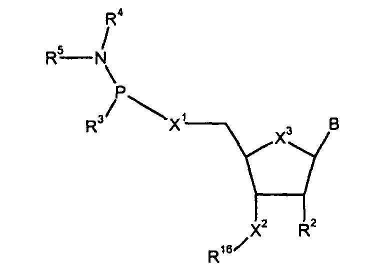 thereby forming a 3',5'-protected compound represented by the following structural formula, or a stereoisomer thereof: 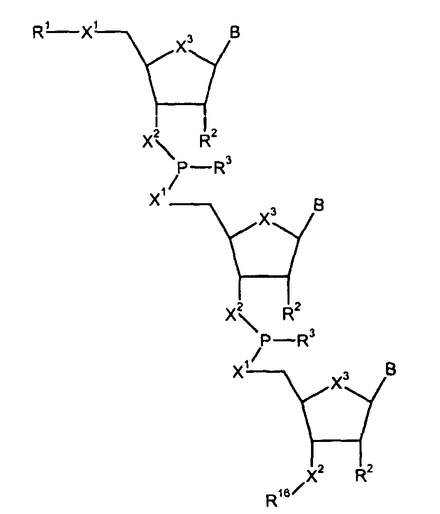
g) optionally repeating steps e) and f) one or more times, thereby forming an extended 3',5'-protected multimer;
h) treating the 3',5'-protected multimer to remove R¹⁶, thereby forming a 3'-deprotected multimer;
i) reacting the 3'-deprotected multimer in the presence of a base with a compound selected from the group consisting of: 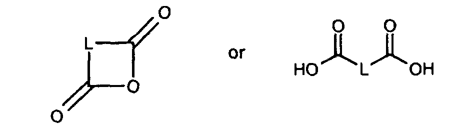 thereby forming a solid support loading reagent represented by the following structural formula, or a stereoisomer thereof: 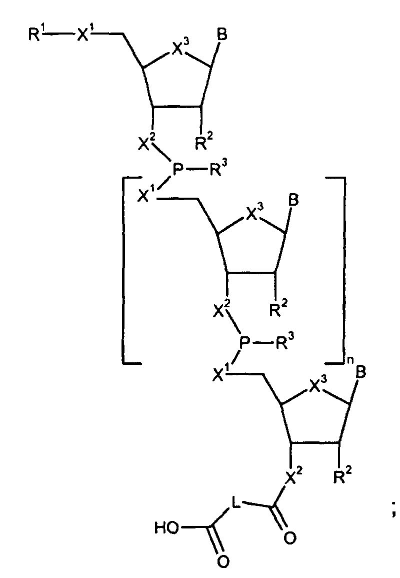 and
j) reacting the solid support loading reagent with a solid support functionalized with primary or secondary amine functional in the presence of a base and a substituted or unsubstituted dialiphatic carbodiimide, thereby preparing said multimer derivatized solid support.

51. The method of Claim 50, further comprising the step of reacting the solid support loading reagent formed in step i) with p-nitrophenol in the presence of a base and a substituted or unsubstituted dialiphatic carbodiimide, thereby forming an activated solid support loading reagent.

52. The method of Claim 51, wherein the substituted or unsubstituted dialiphatic carbodiimide is dicyclohexyl carbodiimide or diisopropyl carbodiimide.

53. Use of a phosphoramidite compound according to any one of claims 1 to 14 for the synthesis of oligonucleotides.

54. Use of a trivalent phosphorus multimer derivatized solid support according to any one of claims 38 to 46 for the synthesis of oligonucleotides.

## Patentansprüche

1. Phosphoramiditverbindung, umfassend zwei oder mehr mit einem oder mehr Internukleosid-Phosphoratomen verknüpfte Nukleosidreste, wobei die Internukleosid-Phosphoratome Phosphor(III)atome sind, welche eine Phosphit-Triestergruppe umfassen, und der Phosphoramiditrest eine Gruppe der Formel -X¹-PR³NR⁴R⁵ ist, wobei X¹ O darstellt; R³ eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe darstellt; und R⁴ und R⁵ jeweils, unabhängig, eine substituierte oder unsubstituierte aliphatische Gruppe sind, oder zusammen mit dem Stickstoff, an den sie gebunden sind, eine Heterocycloalkylgruppe bilden.

2. Phosphoramiditverbindung nach Anspruch 1, die einen Phosphoramiditrest umfasst, der an die 3'-Position des Nukleosidrests gebunden ist, welcher den Phosphoramiditrest trägt.

3. Phosphoramiditverbindung nach einem der beiden Ansprüche 1 und 2, wobei R³ eine beta-Cyanoethyloxygruppe darstellt.

4. Phosphoramiditverbindung nach Anspruch 3, wobei R⁴ und R⁵ Isopropylgruppen darstellen.

5. Phosphoramiditverbindung nach Anspruch 1, wobei die Phosphit-Triestergruppe die 5'-Position eines Nukleosidrests, welcher den Phosphoramiditrest trägt, mit der 3'-Position eines zweiten Nukleosidrests verknüpft.

6. Verbindung nach Anspruch 1, welche die folgende Strukturformel, oder ein Stereoisomer davon, aufweist: 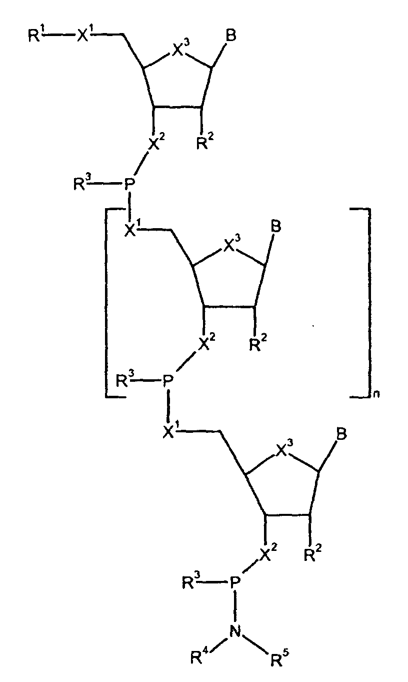 wobei:
jedes X¹ -O- ist;
jedes X² -O- ist;
jedes X³, unabhängig, -O-, -S-, -CH₂-, oder -(CH₂)₂- ist;
R¹ eine Schutzgruppe ist;
jedes R², unabhängig, -H, -F, -NHR⁶, -CH₂R⁶ oder -OR⁶ ist;
jedes R³, unabhängig, eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe ist;
R⁴ und R⁵ jeweils, unabhängig, eine substituierte oder unsubstituierte aliphatische Gruppe sind, oder zusammen mit dem Stickstoff, an den sie gebunden sind, eine Heterocycloalkylgruppe bilden;
R⁶ -H, eine substituierte oder unsubstituierte aliphatische Gruppe, eine substituierte oder unsubstituierte aromatische Gruppe, ein substituiertes oder unsubstituiertes Aralkyl, oder eine Schutzgruppe ist;
jedes B, unabhängig, H oder eine geschützte oder ungeschützte Nukleosidbase ist;
und
n 0 oder eine positive ganze Zahl ist.

7. Verbindung nach Anspruch 6, wobei jedes X³ -O- ist.

8. Verbindung nach Ansprüchen 6 oder 7, wobei R³ -OCH₂CH₂CN oder -SCH₂CH₂CN ist.

9. Verbindung nach Ansprüchen 6, 7 oder 8, wobei R¹ eine Säure-labile Schutzgruppe ist.

10. Verbindung nach Ansprüchen 6, 7, 8 oder 9, wobei R² -H ist.

11. Verbindung nach Ansprüchen 6, 7, 8 oder 9, wobei R² -OR⁶ ist und R⁶ Me, -CH₂CH₂OMe oder eine HydroxySchutzgruppe ist.

12. Verbindung nach Anspruch 6, welche die folgende Strukturformel oder ein Stereoisomer davon aufweist: 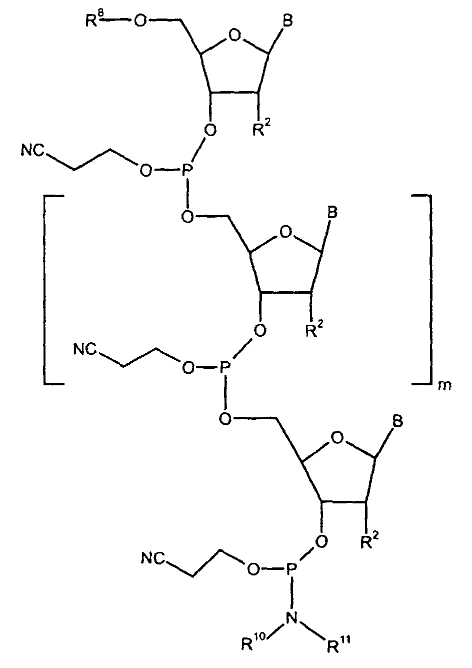 wobei:
R² -H oder -OR⁶ ist;
R⁶ -H, eine substituierte oder unsubstituierte aliphatische Gruppe, eine substituierte oder unsubstituierte aromatische Gruppe, ein substituiertes oder unsubstituiertes Aralkyl, oder eine Alkohol-Schutzgruppe ist;
R⁸ ein substituiertes oder unsubstituiertes Trityl ist;
R¹⁰ und R¹¹ jeweils, unabhängig, eine substituierte oder unsubstituierte aliphatische Gruppe sind;
jedes B, unabhängig, H oder eine geschützte oder ungeschützte Nukleosidbase ist;
und
m 0 oder 1 ist.

13. Verbindung nach Anspruch 12, wobei:
R⁸ 4,4'-Dimethoxytrityl ist;
R² -H ist; und
R¹⁰ und R¹¹ Isopropyl sind.

14. Verbindung nach Anspruch 12, wobei:
R⁸ 4,4'-Dimethoxytrityl ist;
R² -OR⁶ ist;
R¹⁰ und R¹¹ Isopropyl sind; und
R⁶ Me, -CH₂CH₂OMe, t-Butyldimethylsilyl, Tetrahydropyranyl, 4-Methoxy-tetrahydropyranyl oder Fpmp ist.

15. Verfahren zur Herstellung eines Oligonukleotids, dargestellt durch die folgende Strukturformel oder einem Stereoisomer davon: 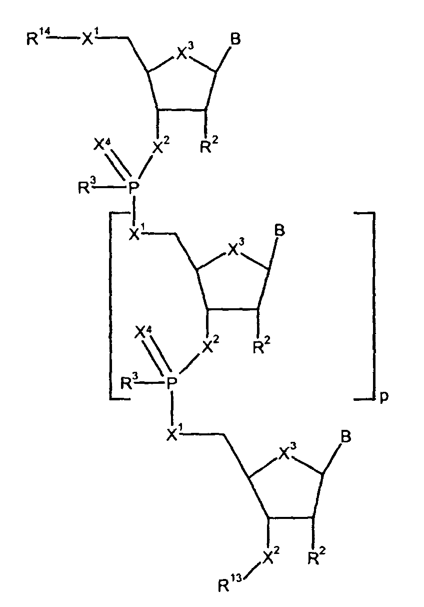 wobei:
jedes X¹ -O- ist;
jedes X² -O- ist;
jedes X³, unabhängig, -O-, -S-, -CH₂-, oder -(CH₂)₂- ist;
jedes X⁴, unabhängig, =O oder =S ist;
jedes R², unabhängig, -H, -F, -NHR⁶, -CH₂R⁶ oder -OR⁶ ist;
R⁶ -H, eine substituierte oder unsubstituierte aliphatische Gruppe, eine substituierte oder unsubstituierte aromatische Gruppe, ein substituiertes oder unsubstituiertes Aralkyl, oder eine Schutzgruppe ist;
jedes R³, unabhängig, eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe ist;
R¹³ eine Alkoholschutzgruppe, eine Gruppe der Formel -Y²-L-Y¹, eine Gruppe der Formel -Y²-L-Y²-R¹⁵ oder ein fester Träger ist;
R¹⁴ -H oder eine Schutzgruppe ist;
R¹⁵ ein fester Träger ist;
jedes B, unabhängig, H oder eine geschützte oder ungeschützte Nukleosidbase ist;
p eine positive ganze Zahl ist;
Y¹ ein Ester oder eine Carbonsäuregruppe ist;
Y² eine Einzelbindung, -C(O)-, -C(O)NR¹⁷-, -C(O)O-, -NR¹⁷- oder -O- ist;
L eine substituierte oder unsubstituierte aliphatische Gruppe oder eine substituierte oder unsubstituierte aromatische Gruppe ist; und
R¹⁷ -H, eine substituierte oder unsubstituierte aliphatische Gruppe oder eine substituierte oder unsubstituierte aromatische Gruppe ist,
umfassend die Schritte:
a) Koppeln eines trivalenten Phosphormultimers, dargestellt durch die folgende Strukturformel oder einem Stereoisomer davon: 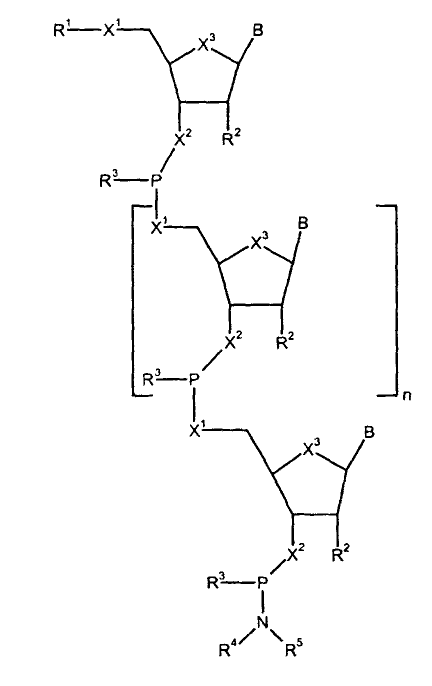 wobei:
R¹ eine Schutzgruppe ist;
R⁴ und R⁵ jeweils, unabhängig, eine substituierte oder unsubstituierte aliphatische Gruppe sind, oder R⁴ und R⁵ zusammen genommen mit dem Stickstoff, an den sie gebunden sind, eine Heterocycloalkylgruppe bilden; und
n 0 oder eine positive ganze Zahl ist,
mit einem 5'-entschützten Nukleosid oder Oligonukleotid, dargestellt durch die folgende Strukturformel oder einem Stereoisomer davon: 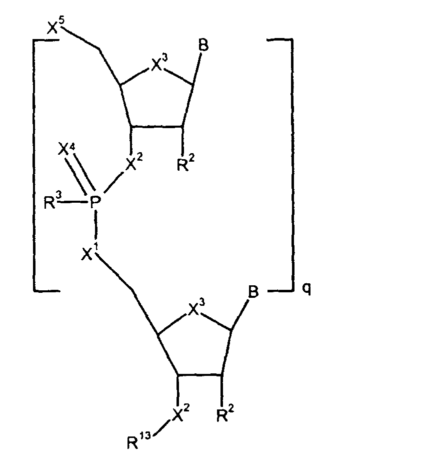
wobei:
X⁵-OH ist; und
q 0 oder eine positive ganze Zahl ist, um ein erstes Zwischenprodukt herzustellen, dargestellt durch die folgende Strukturformel oder einem Stereoisomer davon: 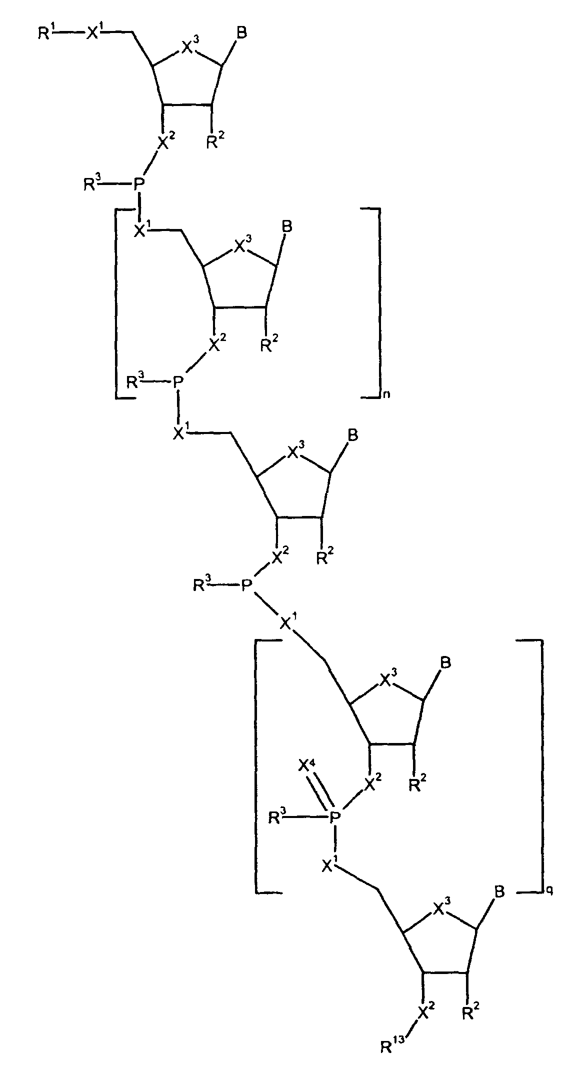
b) Oxidieren oder Sulfurisieren der trivalenten Phosphorgruppen im ersten Zwischenprodukt, um ein zweites Zwischenprodukt, dargestellt durch die folgende Strukturformel oder einem Stereoisomer davon, zu bilden: 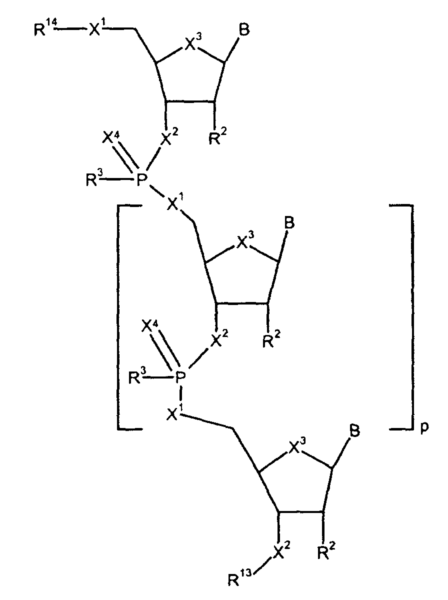
c) wahlweise das Bedecken von X⁵ Gruppen, die nicht mit dem trivalenten Phosphormultimer in Schritt a) reagiert haben;
d) Behandeln des zweiten Zwischenprodukts, um R¹ zu entfernen, um ein 5'-entschütztes Oligonukleotid zu bilden; und
e) wahlweise das Wiederholen der Schritte a)- d) ein oder mehrere Male, wobei der letzte Schritt, Schritt c) oder Schritt d) ist, dabei Herstellen eines Oligonukleotids.

16. Verfahren nach Anspruch 15, wobei R¹³ ein fester Träger ist.

17. Verfahren nach Anspruch 16, weiter umfassend den Schritt des Spaltens des Oligonukleotidprodukts von dem festen Träger.

18. Verfahren nach Ansprüchen 15, 16 oder 17, weiter umfassend das Entschützen der Nukleotidbasen des Oligonukleotids.

19. Verfahren nach Anspruch 15, wobei R¹³ eine Alkoholschutzgruppe ist.

20. Verfahren nach Anspruch 19, weiter umfassend den Schritt des Entfernens der R¹³ Schutzgruppe von dem Oligonukleotidprodukt.

21. Verfahren nach Ansprüchen 15, 16, 17, 18, 19 oder 20, wobei R¹ eine unsubstituierte Trityl-, eine Monoalkoxytrityl-, eine Dialkoxytrityl-, eine Trialkoxytrityl-, Tetrahydropyranyl- oder eine Pixylgruppe ist.

22. Verfahren nach Anspruch 21, wobei R¹ anhand einer Säure entfernt wird, welche ausgewählt ist aus einer Lösung von Dichloressigsäure in Dichlormethan und einer Lösung von Trichloressigsäure in Dichlormethan.

23. Verfahren nach einem der Ansprüche 15 bis 21, wobei jedes R³ -OCH₂CH₂CN oder -SCH₂CH₂CN ist.

24. Verfahren nach Anspruch 23, weiter umfassend das Entfernen von -CH₂CH₂CN von -OCH₂CH₂CN oder -SCH₂CH₂CN, durch Behandeln des Oligonukleotids mit einer Base.

25. Verfahren nach Anspruch 24, wobei die Nukleotidbasen während der Behandlung des Oligonukleotids mit einer Base entschützt werden.

26. Verfahren nach einem der Ansprüche 15 bis 25, wobei die trivalenten Phosphorgruppen durch Behandeln des ersten Zwischenprodukts mit einer Lösung, die I₂ und Wasser enthält, oxidiert werden.

27. Verfahren nach einem der Ansprüche 15 bis 25, wobei die trivalenten Phosphorgruppen durch Behandeln des ersten Zwischenprodukts mit 3-Amino-[1,2,4]-dithiazol-5-thion oder 3*H*-benzodithiol-3-on-1,1-dioxid sulfurisiert werden.

28. Verfahren nach einem der Ansprüche 15 bis 25, wobei mehr als ein Durchlauf der Schritte a), b), c) und d) durchgeführt wird, und das hergestellte Oligonukleotid ein chimäres Oligonukleotid ist.

29. Verfahren nach einem der Ansprüche 15 bis 26, wobei das hergestellte Oligonukleotid ein Phosphat ist.

30. Verfahren nach einem der Ansprüche 15 bis 27, wobei das hergestellte Oligonukleotid ein Phosphorthioat ist.

31. Verfahren nach einem der Ansprüche 15 bis 30, wobei das hergestellte Oligonukleotid bis zu 50 Nukleotidbasen aufweist.

32. Verfahren zur Herstellung eines trivalenten Phosphormultimers, dargestellt durch die folgende Strukturformel oder einem Stereoisomer davon: 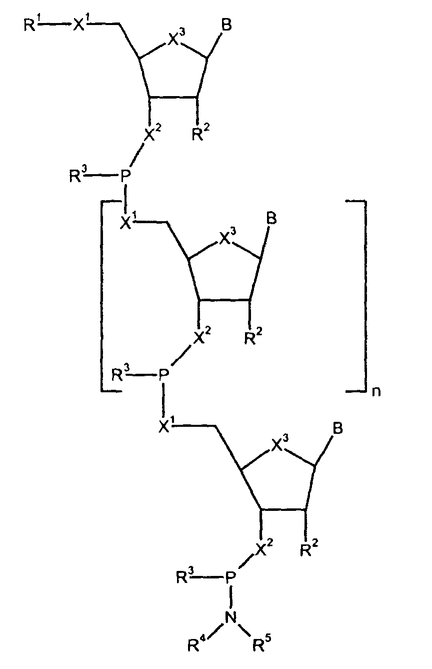 wobei:
jedes X¹ -O- ist;
jedes X² -O- ist;
jedes X³, unabhängig, -O-, -S-, -CH₂-, oder -(CH₂)₂- ist;
R¹ eine Schutzgruppe ist;
jedes R², unabhängig, -H, -F, -NHR⁶, -CH₂R⁶ oder -OR⁶ ist;
jedes R³, unabhängig, eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe ist;
R⁴ und R⁵ jeweils, unabhängig, eine substituierte oder unsubstituierte aliphatische Gruppe sind, oder zusammen mit dem Stickstoff, an den sie gebunden sind, eine Heterocycloalkylgruppe bilden;
R⁶ -H, eine substituierte oder unsubstituierte aliphatische Gruppe, eine substituierte oder unsubstituierte aromatische Gruppe, ein substituiertes oder unsubstituiertes Aralkyl oder eine Schutzgruppe ist;
jedes B, unabhängig, H oder eine geschützte oder ungeschützte Nukleosidbase ist;
und
n 0 oder eine positive ganze Zahl ist;
umfassend die Schritte:
a) Schützen des 3'-Substituenten eines Nukleosids, dargestellt durch die folgende Strukturformel oder einem Stereoisomer davon: 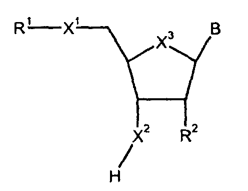 dadurch Bilden eines ersten Zwischenprodukts, dargestellt durch die folgende Strukturformel oder einem Stereoisomer davon: 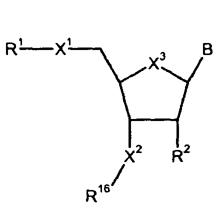 wobei R¹⁶ eine Schutzgruppe ist, welche orthogonal zu R¹ liegt.
b) Behandeln des ersten Zwischenprodukts, um R¹ zu entfernen, dadurch Bilden eines 5'entschützten Nukleosids;
c) Umsetzen des 5'-entschützten Nukleosids mit einer Verbindung, dargestellt durch die folgende Strukturformel oder einem Stereoisomer davon: 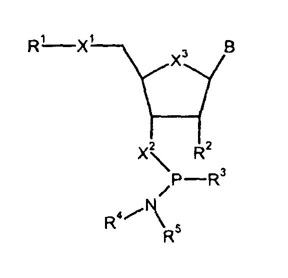 in der Gegenwart eines Kopplungskatalysators, dadurch Bilden eines 3',5'-geschützten Multimers, dargestellt durch die folgende Strukturformel oder einem Stereoisomer davon: 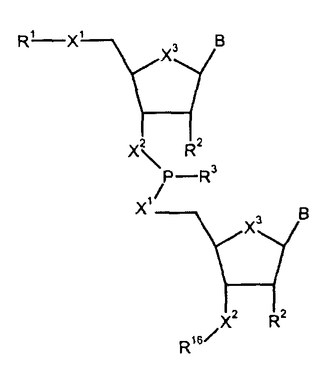
d) wahlweises Behandeln des 3',5'-geschützten Multimers, um R¹⁶ zu entfernen, dadurch Bilden eines 3'-entschützten Multimers;
e) Umsetzen des 3'-entschützten Multimers mit einer Verbindung, dargestellt durch die folgende Strukturformel oder einem Stereoisomer davon: 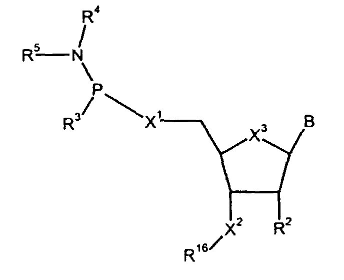 in der Gegenwart eines Kopplungskatalysators, dadurch Bilden einer 3',5'-geschützten Verbindung, dargestellt durch die folgende Strukturformel oder einem Stereoisomer davon: 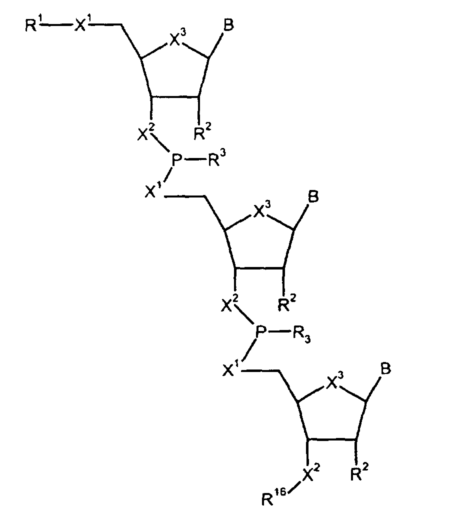
f) wahlweises Wiederholen der Schritte d) und e) ein oder mehrere Male, dadurch Bilden eines 3',5'-geschützten Multimers;
g) Behandeln des 3',5'-geschützten Multimers, um R¹⁶ zu entfernen, dadurch Bilden eines 3'entschützten Multimers;
h) Umsetzen des 3'-entschützten Multimers mit einer trivalenten Phosphorverbindung, dargestellt durch eine der folgenden Strukturformeln: 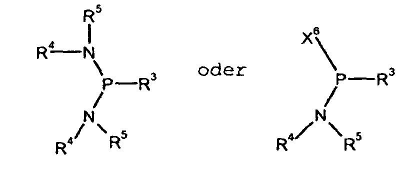
wobei X⁶ ein Halogen ist, dadurch Bilden des trivalenten Phosphormultimers.

33. Verfahren nach Anspruch 32, wobei der Kopplungskatalysator Tetrazol oder S-Ethylthiotetrazol ist.

34. Verfahren nach Ansprüchen 32 oder 33, wobei R¹⁶ Levulynoyl ist und durch Behandlung des 3',5'-geschützten Multimers mit Hydrazinhydrat in einer Pyridin/Essigsäure entfernt wird.

35. Verfahren zur Herstellung eines trivalenten Phosphormultimers, dargestellt durch die folgende Strukturformel oder einem Stereoisomer davon: 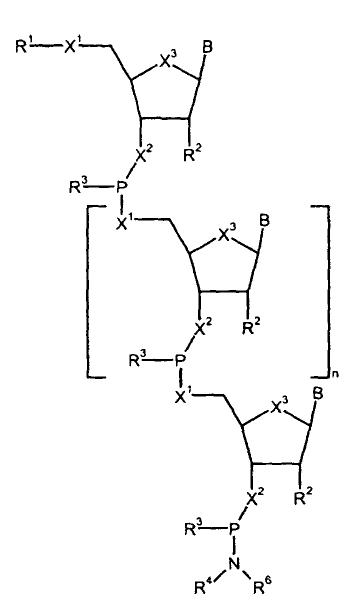 wobei:
jedes X¹, unabhängig, -O- ist;
jedes X², unabhängig, -O- ist;
jedes X³, unabhängig, -O-, -S-, -CH₂-, oder -(CH₂)₂- ist;
R¹ eine Schutzgruppe ist;
jedes R², unabhängig, -H, -F, -NHR⁶, -CH₂R⁶ oder -OR⁶ ist;
jedes R³, unabhängig, eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe ist;
R⁴ und R⁵, jeweils unabhängig, eine substituierte oder unsubstituierte aliphatische Gruppe sind, oder zusammen mit dem Stickstoff, an den sie gebunden sind, eine Heterocycloalkylgruppe bilden;
R⁶ -H, eine substituierte oder unsubstituierte aliphatische Gruppe, eine substituierte oder unsubstituierte aromatische Gruppe, ein substituiertes oder unsubstituiertes Aralkyl oder eine Schutzgruppe ist;
jedes B, unabhängig, -H oder eine geschützte oder ungeschützte Nukleosidbase ist; und
n 0 oder eine positive ganze Zahl ist;
umfassend die Schritte:
a) Schützen des 3'-Substituenten eines Nukleosids, dargestellt durch die folgende Strukturformel oder einem Stereoisomer davon: 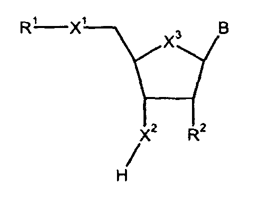 dadurch Bilden eines ersten Zwischenprodukts, dargestellt durch die folgende Strukturformel oder einem Stereoisomer davon: 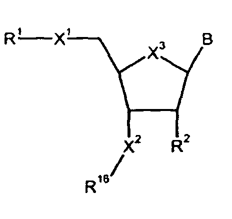 wobei R¹⁶ eine Schutzgruppe ist, welche orthogonal zu R¹ liegt.
b) Behandeln des ersten Zwischenprodukts, um R¹ zu entfernen, dadurch Bilden eines 5'-entschützten Nukleosids;
c) Umsetzen des 5'-entschützten Nukleosids mit einer trivalenten Phosphorverbindung, dargestellt durch eine der folgenden Strukturformeln: 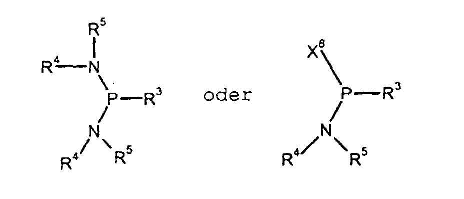 wobei X⁶ ein Halogen ist, dadurch Bilden eines 5'-Phosphoramidits;
d) Umsetzen des 5'-Phosphoramidits mit einer Verbindung, dargestellt durch die folgende Strukturformel oder einem Stereoisomer davon: 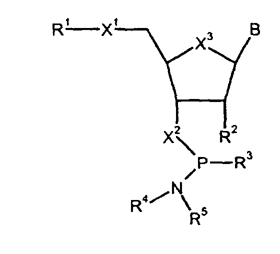 in der Gegenwart eines Kopplungskatalysators, dadurch Bilden eines 3',5'-geschützten Multimers, dargestellt durch die folgende Strukturformel oder einem Stereoisomer davon: 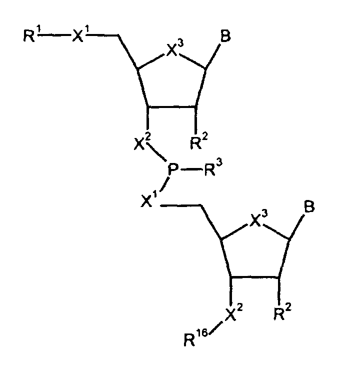
e) wahlweises Behandeln des 3',5'-geschützten Multimers, um R¹⁶ zu entfernen, dadurch Bilden eines 3'-entschützten Multimers;
f) Umsetzen des 3'-entschützten Multimers mit einer Verbindung, dargestellt durch die folgende Strukturformel oder einem Stereoisomer davon: 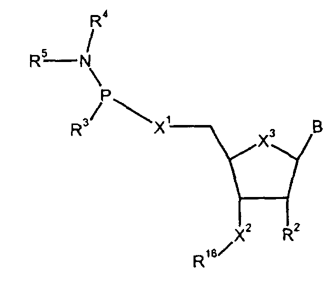 in der Gegenwart eines Kopplungskatalysators, dadurch Bilden einer 3',5'-geschützten Verbindung, dargestellt durch die folgende Strukturformel oder einem Stereoisomer davon: 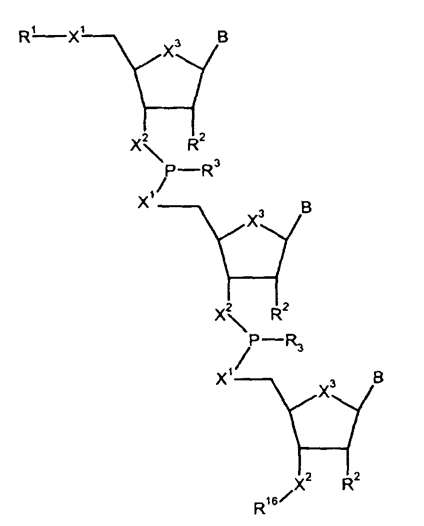
g) wahlweises Wiederholen der Schritte e) und f) ein oder mehrere Male, dadurch Bilden eines verlängerten 3',5'-geschützten Multimers;
h) Behandeln des 3',5'-geschützten Multimers, um R¹⁶ zu entfernen, dadurch Bilden eines 3'-entschützten Multimers;
i) Umsetzen des 3'-entschützten Multimers mit einer trivalenten Phosphorverbindung, dargestellt durch eine der folgenden Strukturformeln 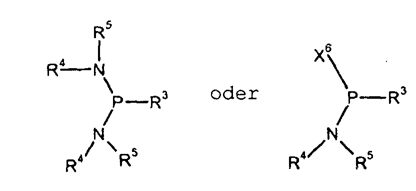
wobei X⁶ ein Halogen ist, dadurch Bilden des trivalenten Phosphormultimers.

36. Verfahren nach Anspruch 35, wobei der Kopplungskatalysator Tetrazol oder S-Ethylthiotetrazol ist.

37. Verfahren nach Ansprüchen 30 oder 36, wobei R¹⁶ Levulynoyl ist und durch Behandlung des 3',5'-geschützten Multimers mit Hydrazinhydrat in einer Pyridin/Essigsäure entfernt wird.

38. Mit einem trivalenten Phosphormultimer derivatisierter fester Träger, dargestellt durch die folgende Strukturformel oder einem Stereoisomer davon: 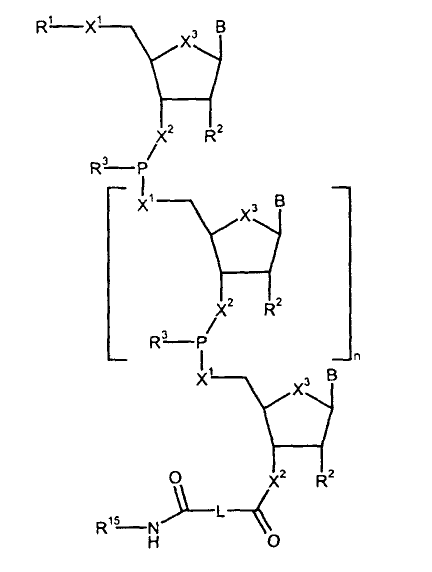 wobei:
jedes X¹, unabhängig, -O- ist;
jedes X², unabhängig, -O- ist;
jedes X³, unabhängig, -O-, -S-, -CH₂-, oder -(CH₂)₂- ist;
R¹ eine Schutzgruppe ist;
jedes R², unabhängig, -H, -F, -NHR⁶, -CH₂R⁶ oder -OR⁶ ist;
jedes R³, unabhängig, eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe ist;
R⁶ -H, eine substituierte oder unsubstituierte aliphatische Gruppe, eine substituierte oder unsubstituierte aromatische Gruppe, ein substituiertes oder unsubstituiertes Aralkyl oder eine Schutzgruppe ist,
jedes B, unabhängig, -H oder eine geschützte oder ungeschützte Nukleosidbase ist;
L eine substituierte oder unsubstituierte aliphatische Gruppe oder eine substituierte oder unsubstituierte aromatische Gruppe ist;
n eine positive ganze Zahl ist; und
R¹⁵ ein fester Träger ist.

39. Fester Träger nach Anspruch 38, wobei jedes X³ -O- ist.

40. Fester Träger nach Ansprüchen 38 oder 39, wobei R³ -OCH₂CH₂CN ist.

41. Fester Träger nach Ansprüchen 38, 39 oder 40, wobei R¹ eine Säure-labile Schutzgruppe ist.

42. Fester Träger nach Anspruch 41, wobei R¹ 4,4'-Dimethoxytrityl ist.

43. Fester Träger nach Ansprüchen 38, 39, 40, 41 oder 42, wobei R² -H ist.

44. Fester Träger nach Ansprüchen 38, 39, 40, 41 oder 42, wobei R₂ -OR⁶ ist und R⁶ eine HydroxySchutzgruppe ist.

45. Fester Träger nach einem der Ansprüche 38 bis 44, wobei L -CH₂CH₂- ist.

46. Fester Träger nach einem der Ansprüche 38 bis 45, wobei R¹⁵ Glas mit kontrollierten Poren, Polystyrol oder mikroporöses Polyamid umfasst.

47. Verfahren zur Herstellung eines, mit einem Multimer derivatisierten, festen Trägers, dargestellt durch die folgende Strukturformel oder einem Stereoisomer davon: 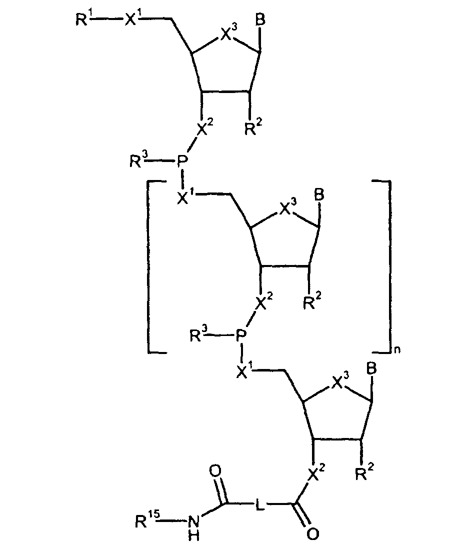 wobei:
jedes X¹ -O- ist;
jedes X² -O- ist;
jedes X³, unabhängig, -O-, -S-, -CH₂-, oder -(CH₂)₂- ist;
R¹ eine Schutzgruppe ist;
jedes R², unabhängig, -H, -F, -NHR⁶, -CH₂R⁶ oder -OR⁶ ist;
jedes R³, unabhängig, eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe ist;
R⁶ -H, eine substituierte oder unsubstituierte aliphatische Gruppe, eine substituierte oder unsubstituierte aromatische Gruppe, ein substituiertes oder unsubstituiertes Aralkyl oder eine Schutzgruppe ist;
jedes B, unabhängig, H oder eine geschützte oder ungeschützte Nukleosidbase ist;
L eine substituierte oder unsubstituierte aliphatische Gruppe oder eine substituierte oder unsubstituierte aromatische Gruppe ist;
n 0 oder eine positive ganze Zahl ist; und
R¹⁵ ein fester Träger ist;
umfassend die Schritte:
a) Schützen des 3'-Substituenten eines Nukleosids, dargestellt durch die folgende Strukturformel oder einem Stereoisomer davon: 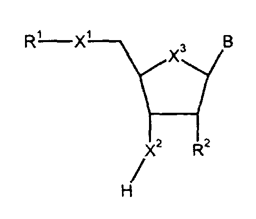 dadurch Bilden eines ersten Zwischenprodukts, dargestellt durch die folgende Strukturformel oder einem Stereoisomer davon: 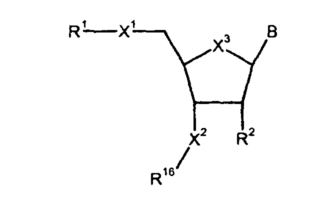 wobei R¹⁶ eine Schutzgruppe ist, welche orthogonal zu R¹ liegt.
b) Behandeln des ersten Zwischenprodukts, um R¹ zu entfernen, dadurch Bilden eines 5'-entschützten Nukleosids;
c) Umsetzen des 5'-entschützten Nukleosids mit einer Verbindung, dargestellt durch die folgende Strukturformel oder einem Stereoisomer davon: 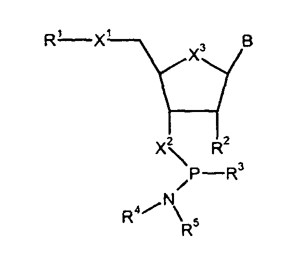 in der Gegenwart eines Kopplungskatalysators, wobei:
R⁴ und R⁵ jeweils, unabhängig, eine substituierte oder unsubstituierte aliphatische Gruppe sind, oder R⁴ und R⁵ zusammen genommen mit dem Stickstoff, an den sie gebunden sind, eine Heterocycloalkylgruppe bilden; dadurch Bilden eines 3',5'-geschützten Multimers, dargestellt durch die folgende Strukturformel oder einem Stereoisomer davon: 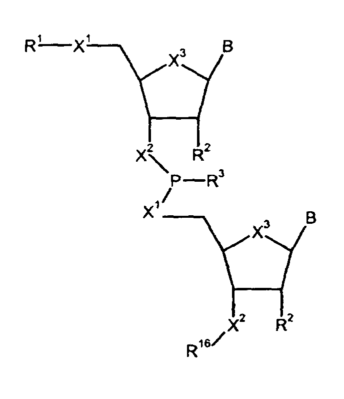
d) wahlweises Behandeln des 3',5'-geschützten Multimers, um R¹⁶ zu entfernen, dadurch Bilden eines 3'-entschützten Multimers; dann
e) Umsetzen des 3'-entschützten Multimers mit einer Verbindung, dargestellt durch die folgende Strukturformel oder einem Stereoisomer davon: 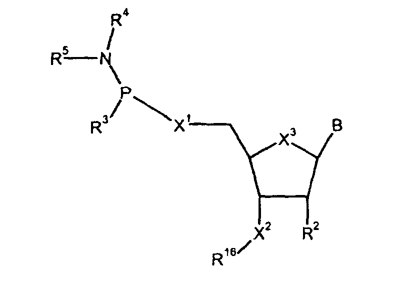 in der Gegenwart eines Kopplungskatalysators, dadurch Bilden einer 3',5'-geschützten Verbindung, dargestellt durch die folgende Strukturformel oder einem Stereoisomer davon: 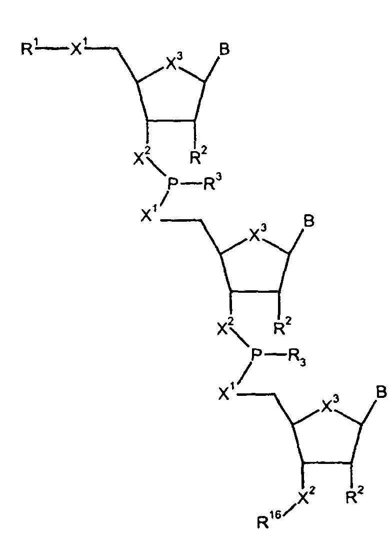
f) wahlweises Wiederholen der Schritte d) und e) ein oder mehrere Male, dadurch Bilden eines verlängerten 3',5'-geschützten Multimers;
g) Behandeln des 3',5'-geschützten Multimers, um R¹⁶ zu entfernen, dadurch Bilden eines 3'-entschützten Multimers;
h) Umsetzen des 3'-entschützten Multimers in der Gegenwart einer Base mit einer Verbindung ausgewählt aus der Gruppe bestehend aus: 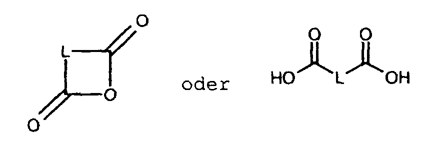 dadurch Bilden eines Beladungsreagens für einen festen Träger, dargestellt durch die folgende Strukturformel oder einem Stereoisomer davon: 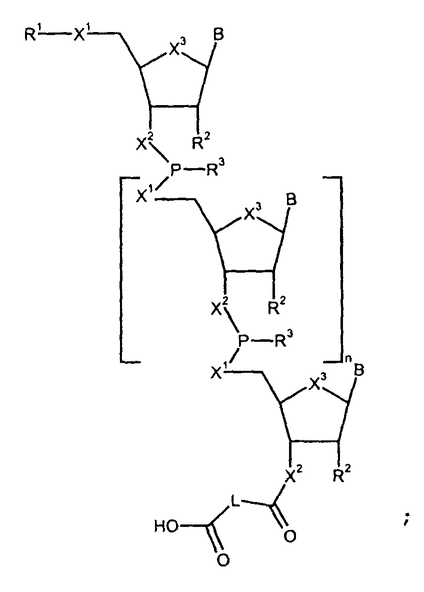 und
i) Umsetzen des Beladungsreagens für einen festen Träger mit einem festen Träger, der mit primären oder sekundären Amingruppen funktionalisiert ist, in der Gegenwart einer Base und eines substituierten oder unsubstituierten dialiphatischen Carbodiimids, dadurch Herstellen des mit einem Multimer derivatisierten, festen Trägers.

48. Verfahren nach Anspruch 47, weiter umfassend den Schritt des Umsetzens des Beladungsreagens für einen festen Träger, das in Schritt h) gebildet wurde, mit p-Nitrophenol in der Gegenwart einer Base und eines substituierten oder unsubstituierten dialiphatischen Carbodiimids, dadurch Bilden eines aktivierten Beladungsreagens für einen festen Träger.

49. Verfahren nach Anspruch 48, wobei das substituierte oder unsubstituierte dialiphatische Carbodiimid Dicyclohexylcarbodiimid oder Diisopropylcarbodiimid ist.

50. Verfahren zur Herstellung eines mit einem Multimer derivatisierten, festen Trägers, dargestellt durch die folgende Strukturformel oder einem Stereoisomer davon: 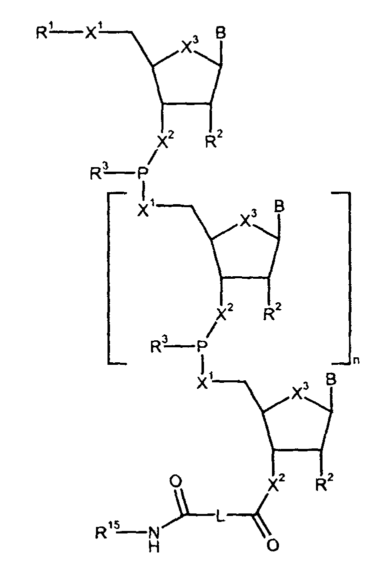 wobei:
jedes X¹, unabhängig, -O- ist;
jedes X², unabhängig, -O- ist;
jedes X³, unabhängig, -O-, -S-, -CH₂-, oder -(CH₂)₂- ist;
R¹ eine Schutzgruppe ist;
jeder R², unabhängig, -H, -F, -NHR⁶, -CH₂R⁶ oder -OR⁶ ist;
jeder R³, unabhängig, eine beta-Cyanoethyloxy-, beta-Cyanoethylthio-, 4-Cyanobut-2-enylthio-, 4-Cyanobut-2-enyloxy-, Allylthio-, Allyloxy-, Crotylthio-, oder Crotyloxygruppe ist;
R⁶ -H, eine substituierte oder unsubstituierte aliphatische Gruppe, eine substituierte oder unsubstituierte aromatische Gruppe, ein substituiertes oder unsubstituiertes Aralkyl oder eine Schutzgruppe ist;
jedes B, unabhängig, H oder eine geschützte oder ungeschützte Nukleosidbase ist;
L eine substituierte oder unsubstituierte aliphatische Gruppe oder eine substituierte oder unsubstituierte aromatische Gruppe ist;
n 0 oder eine positive ganze Zahl ist; und
R¹⁵ ein fester Träger ist;
umfassend die Schritte:
a) Schützen des 3'-Substituenten eines Nukleosids, dargestellt durch die folgende Strukturformel oder einem Stereoisomer davon: 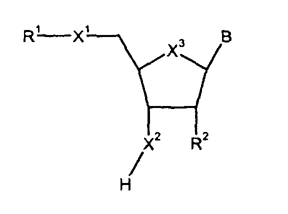 dadurch Bilden eines ersten Zwischenprodukts, dargestellt durch die folgende Strukturformel oder einem Stereoisomer davon: 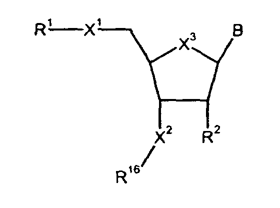 wobei R¹⁶ eine Schutzgruppe ist, welche orthogonal zu R¹ liegt.
b) Behandeln des ersten Zwischenprodukts mit einer Säure, um R¹ zu entfernen, dadurch Bilden eines 5'-entschützten Nukleosids;
c) Umsetzen des 5'-entschützten Nukleosids mit einer trivalenten Phosphorverbindung, dargestellt durch eine der folgenden Strukturformeln: 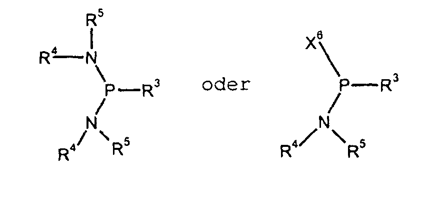 wobei:
X⁶ ein Halogen ist, dadurch Bilden eines 5'-Phosphoramidits; und
R⁴ und R⁵ jeweils, unabhängig, eine substituierte oder unsubstituierte aliphatische Gruppe sind, oder R⁴ und R⁵ zusammen genommen mit dem Stickstoff, an den sie gebunden sind, eine Heterocycloalkylgruppe bilden;
d) Umsetzen des 5'-Phosphoramidits mit einer Verbindung, dargestellt durch die folgende Strukturformel oder einem Stereoisomer davon: 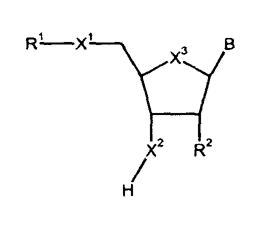 in der Gegenwart eines Kopplungskatalysators, dadurch Bilden eines 3',5'-geschützten Multimers, dargestellt durch die folgende Strukturformel oder einem Stereoisomer davon: 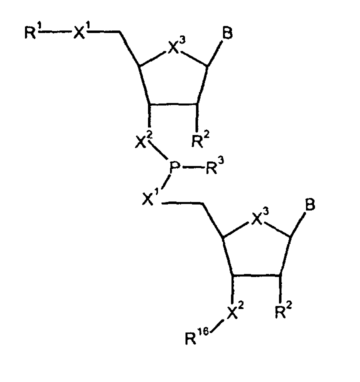
e) wahlweises Behandeln des 3',5'-geschützten Multimers, um R¹⁶ zu entfernen, dadurch Bilden eines 3'-entschützten Multimers; dann
f) Umsetzen des 3'-entschützten Multimers mit einer Verbindung, dargestellt durch die folgende Strukturformel oder einem Stereoisomer davon: 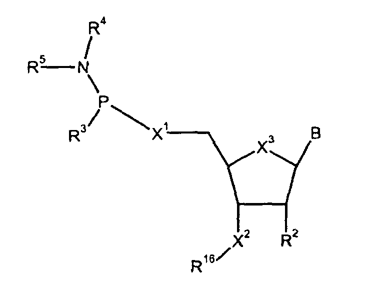 in der Gegenwart eines Kopplungskatalysators, dadurch Bilden einer 3',5'-geschützten Verbindung, dargestellt durch die folgende Strukturformel oder einem Stereoisomer davon: 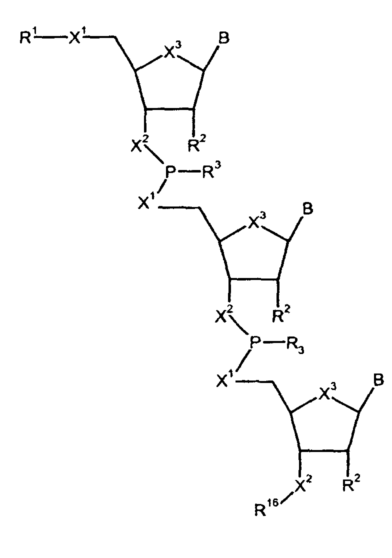
g) wahlweises Wiederholen der Schritte e) und f) ein oder mehrere Male, dadurch Bilden eines verlängerten 3',5'-geschützten Multimers;
h) Behandeln des 3',5'-geschützten Multimers, um R¹⁶ zu entfernen, dadurch Bilden eines 3'-entschützten Multimers;
i) Umsetzen des 3'-entschützten Multimers in der Gegenwart einer Base mit einer Verbindung, ausgewählt aus der Gruppe bestehend aus: 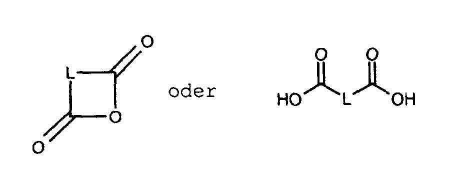 dadurch Bilden eines Beladungsreagens für einen festen Träger, dargestellt durch die folgende Strukturformel oder einem Stereoisomer davon: 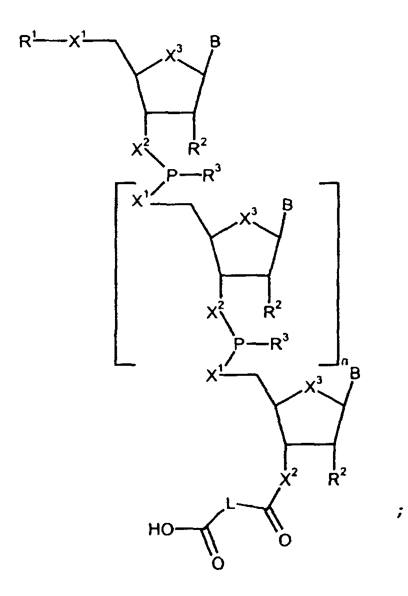 und
j) Umsetzen des Beladungsreagens für einen festen Träger mit einem festen Träger, der mit primären oder sekundären Amingruppen funktionalisiert ist, in der Gegenwart einer Base und eines substituierten oder unsubstituierten dialiphatischen Carbodiimids, dadurch Herstellen des mit einem Multimer derivatisierten, festen Trägers.

51. Verfahren nach Anspruch 50, weiter umfassend den Schritt des Umsetzens des Beladungsreagens für einen festen Träger, das in Schritt i) gebildet wurde, mit p-Nitrophenol in der Gegenwart einer Base und eines substituierten oder unsubstituierten dialiphatischen Carbodiimids, dadurch Bilden eines aktivierten Beladungsreagens für einen festen Träger.

52. Verfahren nach Anspruch 51, wobei das substituierte oder unsubstituierte dialiphatische Carbodiimid Dicyclohexylcarbodiimid oder Diisopropylcarbodiimid ist.

53. Verwendung einer Phosphoramiditverbindung nach einem der Ansprüche 1 bis 14 zur Synthese von Oligonukleotiden.

54. Verwendung eines mit einem trivalenten Phosphormultimer derivatisierten festen Trägers nach einem der Ansprüche 38 bis 46 zur Synthese von Oligonukleotiden.

## Revendications

1. Composé phosphoramidite renfermant deux fragments nucléoside ou plus liés par un ou plusieurs atomes de phosphore internucléosides, dans lequel les atomes de phosphore internucléosides sont des atomes de phosphore (III) comprenant un groupe triester phosphite et le fragment phosphoramidite est un groupe de formule -X¹-PR³NR⁴R⁵ dans laquelle X¹ représente O ; R³ un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ; et R⁴ et R⁵ sont chacun indépendamment un groupe aliphatique substitué ou non substitué ou, conjointement avec l'azote auquel ils sont liés, forment un groupe hétérocycloalkyle.

2. Composé phosphoramidite selon la revendication 1, renfermant un fragment phosphoramidite lié à la position 3' du fragment nucléoside portant le fragment phosphoramidite.

3. Composé phosphoramidite selon l'une ou l'autre des revendications 1 et 2, dans lequel R³ représente un groupe bêta-cyanoéthyloxy.

4. Composé phosphoramidite selon la revendication 3, dans lequel R⁴ et R⁵ représentent des groupes isopropyle.

5. Composé phosphoramidite selon la revendication 1, dans lequel le groupe triesterphosphite lie la position 5' d'un fragment nucléoside portant le fragment phosphoramidite à la position 3' d'un deuxième fragment nucléoside.

6. Composé selon la revendication 1 de formule structurale suivante, ou stéréoisomère de celui-ci : 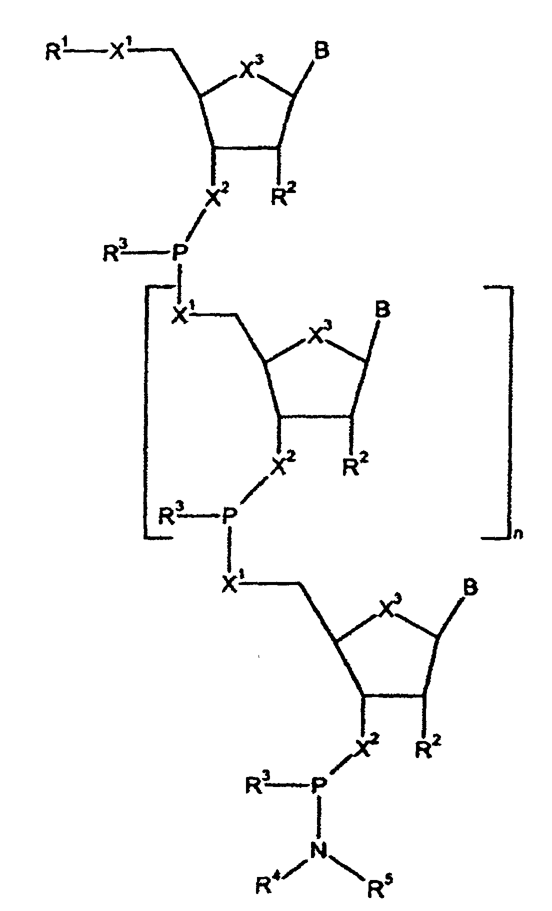 dans laquelle :
chaque X¹ est -O- ;
chaque X² est -O- ;
chaque X³ est indépendamment -O-, -S-, -CH₂-, ou -(CH₂)₂- ;
R¹ est un groupe protecteur ;
chaque R² est indépendamment -H, -F, -NHR⁶, -CH₂R⁶ ou -OR⁶ ;
chaque R³ est indépendamment un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ;
R⁴ et R⁵ sont chacun indépendamment un groupe aliphatique substitué ou non substitué ou, conjointement avec l'azote auquel ils sont liés, forment un groupe hétérocycloalkyle ;
R⁶ est -H, un groupe aliphatique substitué ou non substitué, un groupe aromatique substitué ou non substitué, un aralkyle substitué ou non substitué ou un groupe protecteur ;
chaque B est indépendamment H ou une base nucléoside protégée ou non protégée ;
et
n vaut zéro ou est un entier positif.

7. Composé selon la revendication 6, dans lequel chaque X³ est -O-.

8. Composé selon les revendications 6 ou 7, dans lequel R³ est -OCH₂CH₂CN ou -SCH₂CH₂CN.

9. Composé selon les revendications 6, 7 ou 8, dans lequel R¹ est un groupe protecteur sensible aux acides.

10. Composé selon les revendications 6, 7, 8 ou 9, dans lequel R² est -H.

11. Composé selon les revendications 6, 7, 8 ou 9, dans lequel R² est -OR⁶ et R⁶ est Me, -CH₂CH₂OMe ou un groupe protecteur d'hydroxy.

12. Composé selon la revendication 6 de formule structurale suivante, ou stéréoisomère de celui-ci : 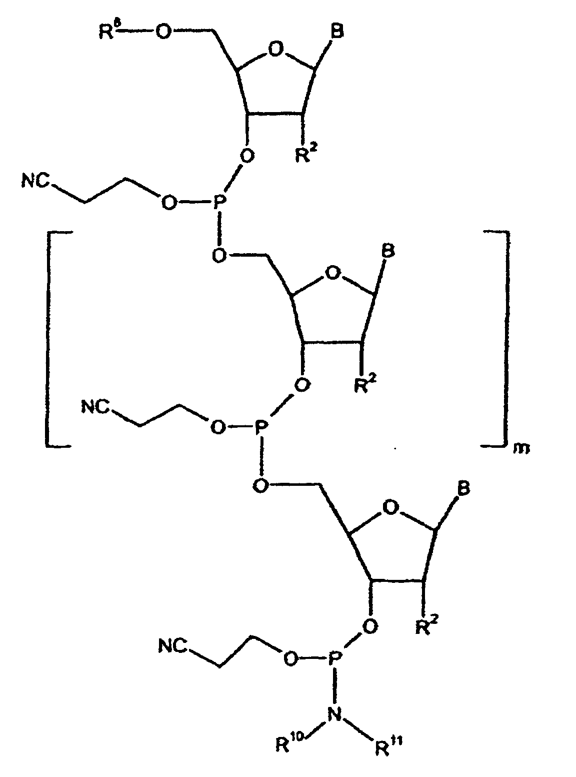 dans laquelle :
R² est -H ou -OR⁶ ;
R⁶ est -H, un groupe aliphatique substitué ou non substitué, un groupe aromatique substitué ou non substitué, un aralkyle substitué ou non substitué ou un groupe protecteur d'alcool ;
R⁸ est un trityle substitué ou non substitué ;
R¹⁰ et R¹¹ sont chacun indépendamment un groupe aliphatique substitué ou non substitué ;
chaque B est indépendamment H ou une base nucléoside protégée ou non protégée ;
et
m vaut 0 ou 1.

13. Composé selon la revendication 12, dans lequel
R⁸ est un 4,4'-diméthoxytrityle ;
R² est -H ; et
R¹⁰ et R¹¹ sont un isopropyle.

14. Composé selon la revendication 12, dans lequel
R⁸ est un 4,4'-diméthoxytrityle ;
R² est -OR⁶ ;
R¹⁰ et R¹¹ sont un isopropyle ; et
R⁶ est Me, -CH₂CH₂OMe, un t-butyldiméthylsilyle, un tétrahydropyranyle, un 4-méthoxytétrahydropyranyle ou Fpmp.

15. Procédé de préparation d'un oligonuléotide représenté par la formule structurale suivante, d'un stéréoisomère de celui-ci : 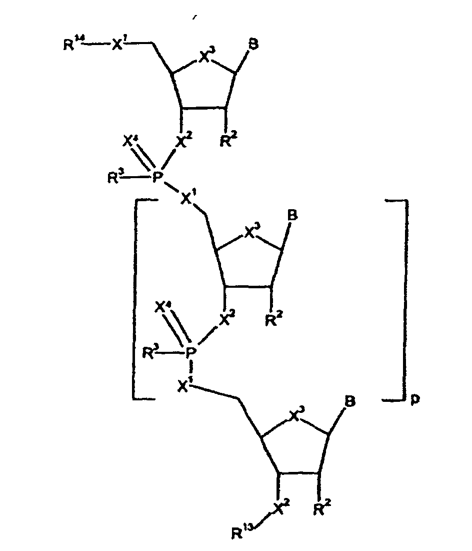 dans laquelle :
chaque X¹ est -O- ;
chaque X² est -O- ;
chaque X³ est indépendamment -O-, -S-, -CH₂-, ou -(CH₂)₂- ;
chaque X⁴ est indépendamment =O ou =S ;
chaque R² est indépendamment -H, -F, -NHR⁶, -CH₂R⁶ ou -OR⁶ ;
R⁶ est -H, un groupe aliphatique substitué ou non substitué, un groupe aromatique substitué ou non substitué, un aralkyle substitué ou non substitué, ou un groupe protecteur ;
chaque R³ est indépendamment un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ;
R¹³ est un groupe protecteur d'alcool, un groupe de formule -Y²-L-Y¹, un groupe de formule -Y²-L-Y²-R¹⁵ ou un support solide ;
R¹⁴ est -H ou un groupe protecteur ;
R¹⁵ est un support solide ;
chaque B est indépendamment H ou une base nucléoside protégée ou non protégée ;
p est un entier positif ;
Y¹ est un groupe ester ou acide carboxylique ;
Y² est une liaison simple, -C(O)- ; -C(O)NR¹⁷- ; -C(O)O-, -NR¹⁷- ou -O- ;
L est un groupe aliphatique substitué ou non substitué, ou un groupe aromatique substitué ou non substitué ; et
R¹⁷ est -H, un groupe aliphatique substitué ou non substitué, ou un groupe aromatique substitué ou non substitué, comprenant les étapes de :
a) couplage d'un multimère phosphoré trivalent représenté par la formule structurale suivante, ou d'un stéréoisomère de celui-ci : 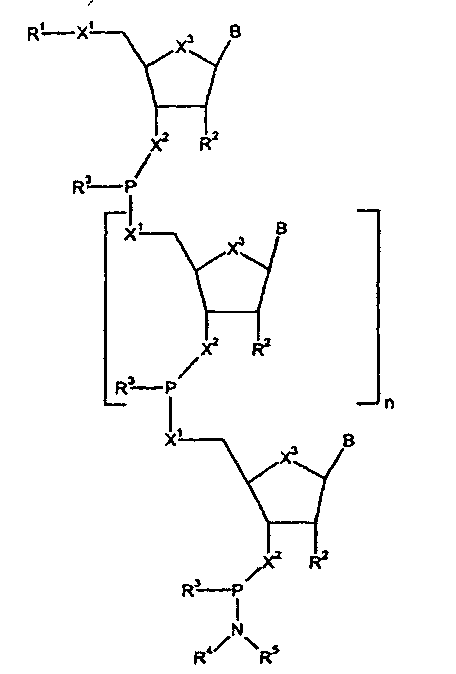 dans laquelle :
R¹ est un groupe protecteur ;
R⁴ et R⁵ sont chacun indépendamment un groupe aliphatique substitué ou non substitué, ou R⁴ et
R⁵ pris conjointement avec l'azote auquel ils sont liés, forment un groupe hétérocycloalkyle ; et
n vaut 0 ou est un entier positif,
avec un nucléoside ou oligonucléotide déprotégé en 5' représenté par la formule structurale suivante ou un stéréoisomère de celui-ci : 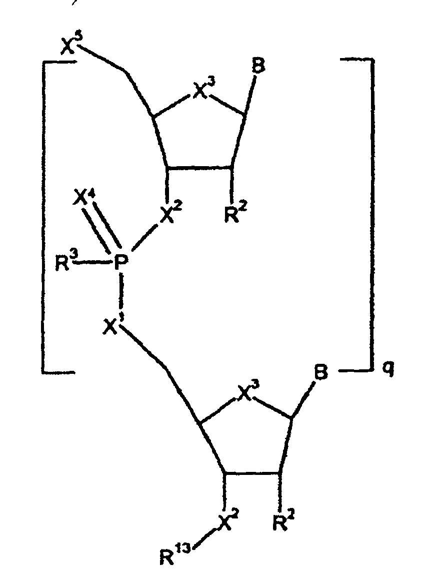
dans laquelle :
X⁵ est -OH ; et
q vaut 0 ou est un entier positif, en vue de produire un premier intermédiaire représenté par la formule structurale suivante ou un stéréoisomère de celui-ci : 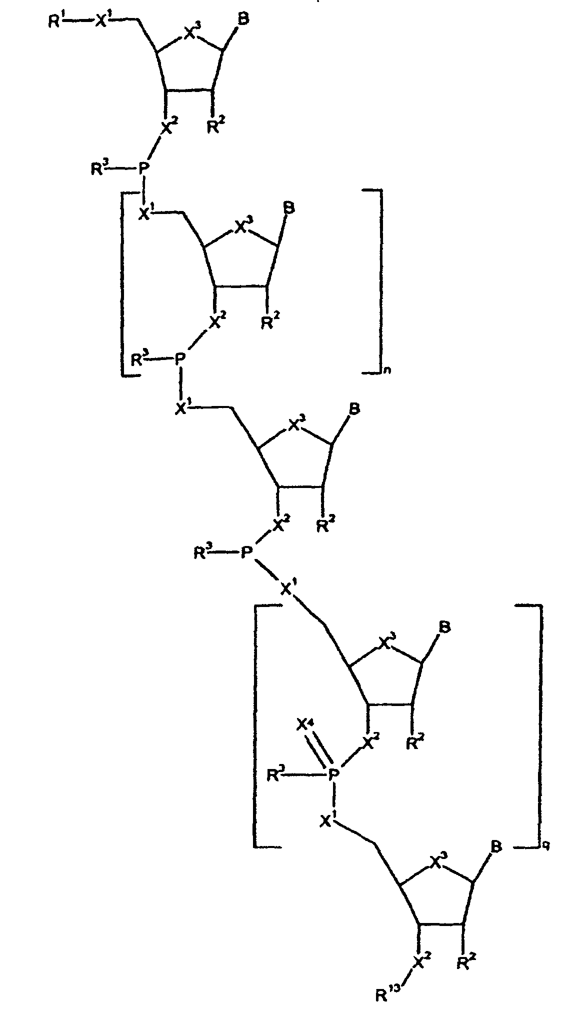
b) oxydation ou sulfuration des groupes phosphorés trivalents dans le premier intermédiaire pour former un deuxième intermédiaire représenté par la formule structurale suivante, ou un stéréoisomère de celui-ci : 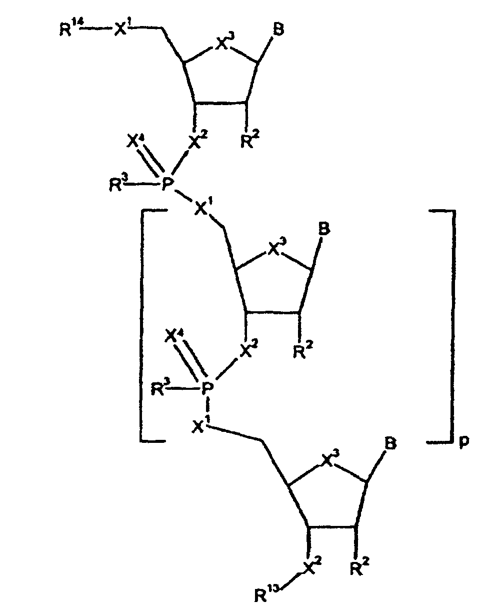
c) coiffage éventuel des groupes X⁵ qui n'ont pas réagi avec le multimère phosphoré trivalent dans l'étape a) ;
d) traitement du deuxième intermédiaire pour éliminer R¹ en vue de former un oligonucléotide déprotégé en 5' ; et
e) répétition éventuelle des étapes a)-d) une ou plusieurs fois, lorsque l'étape finale est l'étape c) ou l'étape d), en préparant ainsi un oligonucléotide.

16. Procédé selon la revendication 15, dans lequel R¹³ est un support solide.

17. Procédé selon la revendication 16, comprenant en outre l'étape de clivage du produit oligonucléotide du support solide.

18. Procédé selon les revendications 15, 16 ou 17, comprenant en outre la déprotection des bases nucléotides de l'oligonucléotide.

19. Procédé selon la revendication 15, dans lequel R¹³ est un groupe protecteur d'alcool.

20. Procédé selon la revendication 19, comprenant en outre l'étape d'élimination du groupe protecteur R¹³ du produit oligonucléotide.

21. Procédé selon les revendications 15, 16, 17, 18, 19 ou 20, dans lequel R¹ est un groupe trityle non substitué, monoalcoxytrityle, dialcoxytrityle, trialcoxytrityle, tétrahydropyranyle ou pixyle.

22. Procédé selon la revendication 21, dans lequel R¹ est éliminé avec un acide choisi parmi une solution d'acide dichloroacétique dans du dichlorométhane et une solution d'acide trichloroacétique dans du dichlorométhane.

23. Procédé selon l'une quelconque des revendications 15 à 21, dans lequel chaque R³ est un -OCH₂CH₂CN ou un -SCH₂CH₂CN.

24. Procédé selon la revendication 23, comprenant en outre l'élimination d'un -CH₂CH₂CN, d'un -OCH₂CH₂CN ou d'un -SCH₂CH₂CN par traitement de l'oligonucléotide avec une base.

25. Procédé selon la revendication 24, dans lequel les bases nucléotides sont déprotégées durant le traitement de l'oligonucléotide avec une base.

26. Procédé selon l'une quelconque des revendications 15 à 25, dans lequel les groupes phosphorés trivalents sont oxydés, par traitement du premier intermédiaire avec une solution contenant I₂ et de l'eau.

27. Procédé selon l'une quelconque des revendications 15 à 25, dans lequel les groupes phosphorés trivalents sont sulfurés par traitement du premier intermédiaire avec la 3-amino-[1,2,4]-dithiazole-5-thione ou le 3*H*-benzodithiol-3-one-1,1-dioxyde.

28. Procédé selon l'une quelconque des revendications 15 à 25, dans lequel on réalise plus d'un cycle des étapes a), b), c) et d), et l'oligonucléotide produit est un oligonucléotide chimérique.

29. Procédé selon l'une quelconque des revendications 15 à 26, dans lequel l'oligonucléotide produit est un phosphate.

30. Procédé selon l'une quelconque des revendications 15 à 27, dans lequel l'oligonucléotide produit est un phosphorothioate.

31. Procédé selon l'une quelconque des revendications 15 à 30, dans lequel l'oligonucléotide préparé renferme jusqu'à 50 bases nucléotides.

32. Procédé de préparation d'un multimère phosphoré trivalent représenté par la formule structurale suivante, ou d'un stéréoisomère de celui-ci : 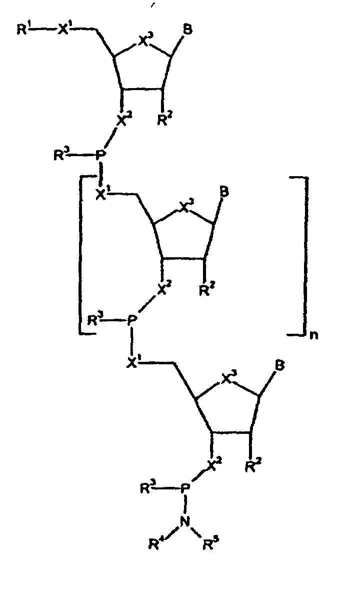 dans laquelle :
chaque X¹ est -O- ;
chaque X² est -O- ;
chaque X³ est indépendamment -O-, -S-, -CH₂-, ou -(CH₂)₂- ;
R¹ est un groupe protecteur ;
chaque R² est indépendamment -H, -F, -NHR⁶, -CH₂R⁶ ou -OR⁶ ;
chaque R³ est indépendamment un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ;
R⁴ et R⁵ sont chacun indépendamment un groupe aliphatique substitué ou non substitué ou, R⁴ et R⁵ pris conjointement avec l'azote auquel ils sont liés, forment un groupe hétérocycloalkyle ;
R⁶ est -H, un groupe aliphatique substitué ou non substitué, un groupe aromatique substitué ou non substitué, un aralkyle substitué ou non substitué ou un groupe protecteur ;
chaque B est indépendamment H ou une base nucléoside protégée ou non protégée ; et
n vaut 0 ou est un entier positif, comprenant les étapes de :
a) protection du substituant en 3' d'un nucléoside représenté par la formule structurale suivante, ou d'un stéréoisomère de celui-ci : 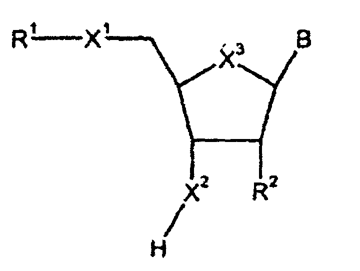 en formant ainsi un premier intermédiaire représenté par la formule structurale suivante ou un stéréoisomère de celui-ci : 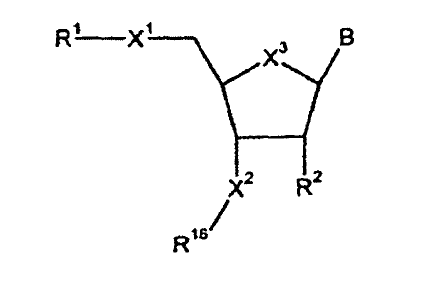 dans laquelle R¹⁶ est un groupe protecteur qui est orthogonal à R¹ ;
b) traitement du premier intermédiaire pour éliminer R¹, en formant ainsi un nucléoside déprotégé en 5' ;
c) réaction du nucléoside déprotégé en 5' en présence d'un catalyseur de couplage avec un composé représenté par la formule structurale suivante ou un stéréoisomère de celui-ci : 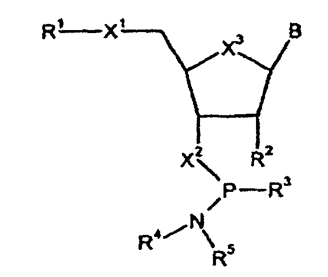 en formant ainsi un multimère protégé en 3',5' représenté par la formule structurale suivante, ou un stéréoisomère de celui-ci : 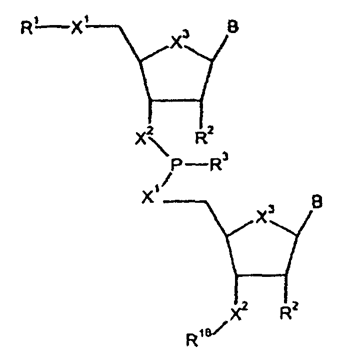
d) traitement éventuel du multimère protégé en 3',5' pour éliminer R¹⁶ en formant ainsi un multimère déprotégé en 3' ; puis
e) réaction en présence d'un catalyseur de couplage du multimère déprotégé en 3' avec un composé représenté par la formule structurale suivante, ou un stéréoisomère de celui-ci : 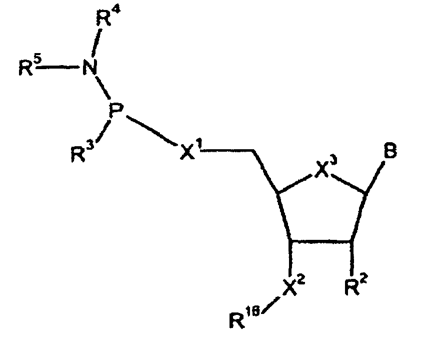 en formant ainsi un composé protégé en 3',5' représenté par la formule structurale suivante ou un stéréoisomère de celui-ci : 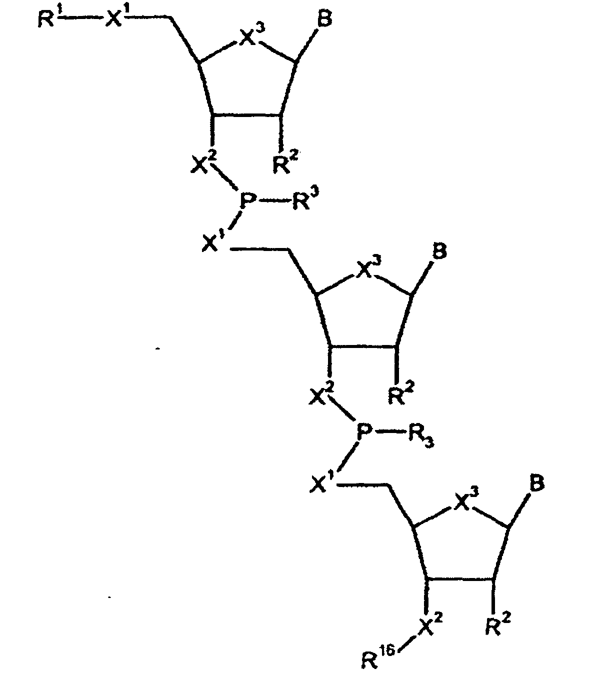
f) répétition éventuelle des étapes d) et e) une ou plusieurs fois en formant ainsi un multimère protégé en 3',5' allongé ;
g) traitement du multimère protégé en 3',5' pour éliminer R¹⁶, en formant ainsi un multimère déprotégé en 3' ;
h) réaction du multimère déprotégé en 3' avec un composé phosphoré trivalent représenté par l'une des formules structurales suivantes : 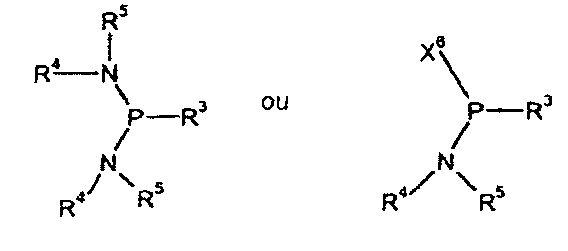 dans lesquelles X⁶ est un halogène en formant ainsi le multimère phosphoré trivalent.

33. Procédé selon la revendication 32, dans lequel le catalyseur de couplage est le tétrazole ou le S-éthylthiotétrazole.

34. Procédé selon les revendications 32 ou 33, dans lequel R¹⁶ est un lévulynoyle et est éliminé par traitement du multimère protégé en 3', 5' avec l'hydrate d'hydrazine dans de la pyridine/acide acétique.

35. Procédé de préparation d'un multimère phosphoré trivalent représenté par la formule structurale suivante, ou d'un stéréoisomère de celui-ci : 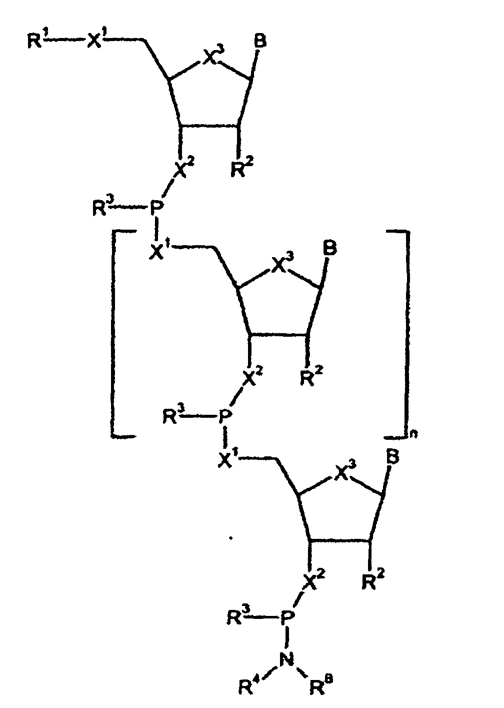 dans laquelle :
chaque X¹ est indépendamment -O- ;
chaque X² est indépendamment -O- ;
chaque X³ est indépendamment -O-, -S-, -CH₂-, ou -(CH₂)₂- ;
R¹ est un groupe protecteur ;
chaque R² est indépendamment -H, -F, -NHR⁶, -CH₂R⁶ ou -OR⁶ ;
chaque R³ est indépendamment un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ;
R⁴ et R⁵ sont chacun indépendamment un groupe aliphatique substitué ou non substitué ou, R⁴ et R⁵ pris conjointement avec l'azote auquel ils sont liés, forment un groupe hétérocycloalkyle ;
R⁶ est -H, un groupe aliphatique substitué ou non substitué, un groupe aromatique substitué ou non substitué, un aralkyle substitué ou non substitué ou un groupe protecteur ;
chaque B est indépendamment H ou une base nucléoside protégée ou non protégée ; et
n vaut 0 ou est un entier positif, comprenant les étapes de :
a) protection du substituant en 3' d'un nucléoside représenté par la formule structurale suivante, ou d'un stéréoisomère de celui-ci : 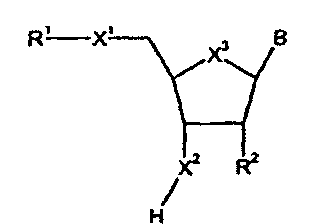 en formant ainsi un premier intermédiaire représenté par la formule structurale suivante ou un stéréoisomère de celui-ci : 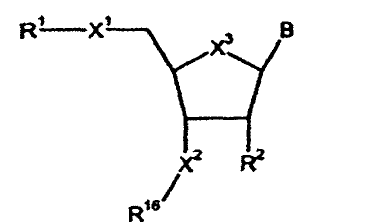 dans laquelle R¹⁶ est un groupe protecteur qui est orthogonal à R¹ ;
b) traitement du premier intermédiaire pour éliminer R¹, en formant ainsi un nucléoside déprotégé en 5' ;
c) réaction du nucléoside déprotégé en 5' avec un composé phosphoré trivalent représenté par l'une des formules structurales suivantes : 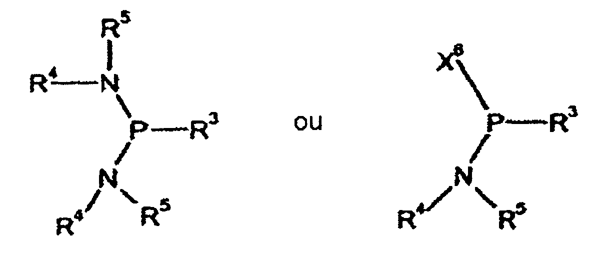 dans lesquelles X⁶ est un halogène, en formant ainsi un 5'-phosphoramidite ;
d) réaction du 5'-phosphoramidite en présence d'un catalyseur de couplage avec un composé représenté par la formule structurale suivante, ou un stéréoisomère de celui-ci : 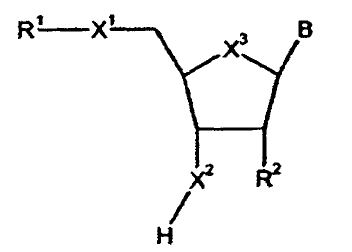 en formant ainsi un multimère protégé en 3',5' représenté par la formule structurale suivante, ou un stéréoisomère de celui-ci : 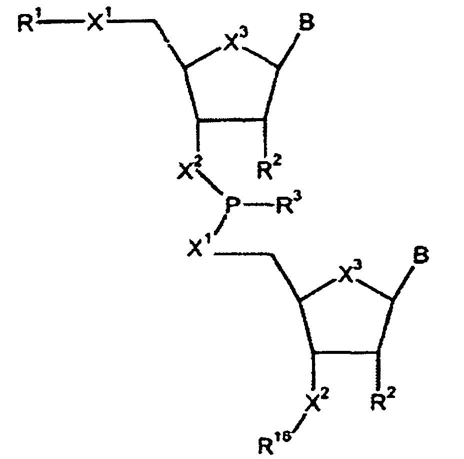
e) traitement éventuel du multimère protégé en 3',5' pour éliminer R¹⁶ en formant ainsi un multimère déprotégé en 3' ; puis
f) réaction en présence d'un catalyseur de couplage du multimère déprotégé en 3' avec un composé représenté par la formule structurale suivante, ou un stéréoisomère de celui-ci : 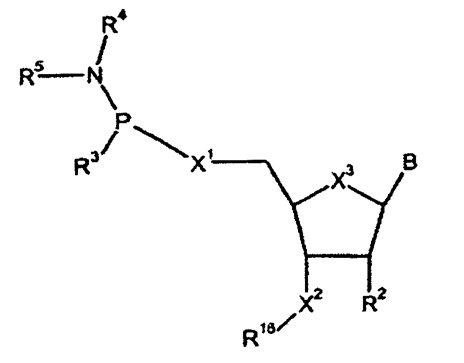 en formant ainsi un composé protégé en 3',5' représenté par la formule structurale suivante, ou un stéréoisomère de celui-ci : 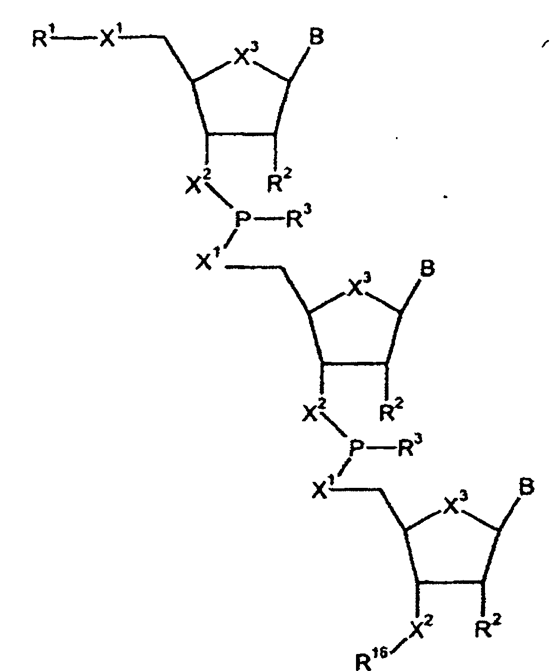
g) répétition des étapes e) et f) une ou plusieurs fois, en formant ainsi un multimère protégé en 3',5' allongé ;
h) traitement du multimère protégé en 3',5' pour éliminer R¹⁶ en formant ainsi un multimère déprotégé en 3' ;
i) réaction du multimère déprotégé en 3' avec un composé phosphoré trivalent représenté par l'une des formules structurales suivantes : 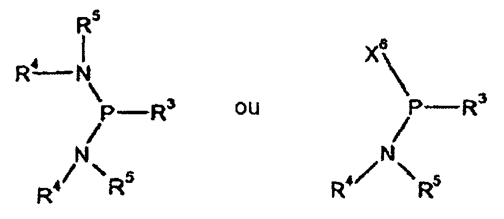
dans lesquelles X⁶ est un halogène, en formant ainsi le multimère phosphoré trivalent.

36. Procédé selon la revendication 35, dans lequel le catalyseur de couplage est le tétrazole ou le S-éthylthiotétrazole.

37. Procédé selon les revendications 30 ou 36, dans lequel R¹⁶ est un lévulynoyle et est éliminé par traitement du multimère protégé en 3',5' avec l'hydrate d'hydrazine dans de la pyridine/acide acétique.

38. Support solide dérivatisé par un multimère phosphoré trivalent représenté par la formule structurale suivante, ou stéréoisomère de celui-ci : 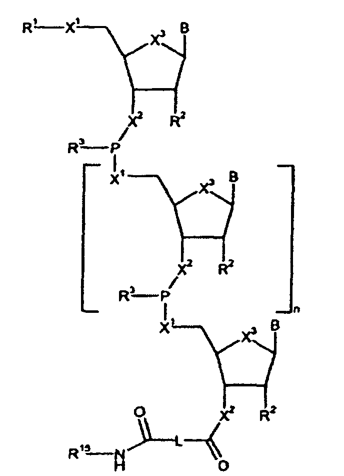 dans laquelle :
chaque X¹ est indépendamment -O- ;
chaque X² est indépendamment -O- ;
chaque X³ est indépendamment -O-, -S-, -CH₂-, ou -(CH₂)₂- ;
R¹ est un groupe protecteur ;
chaque R² est indépendamment -H, -F, -NHR⁶, -CH₂R⁶ ou -OR⁶ ;
chaque R³ est indépendamment un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ;
R⁶ est -H, un groupe aliphatique substitué ou non substitué, un groupe aromatique substitué ou non substitué, un aralkyle substitué ou non substitué ou un groupe protecteur ;
chaque B est indépendamment H ou une base nucléoside protégée ou non protégée ;
L est un groupe aliphatique substitué ou non substitué, ou un groupe aromatique substitué ou non substitué ;
n est un entier positif ; et
R¹⁵ est un support solide.

39. Support solide selon la revendication 38, dans lequel chaque X³ est -O-.

40. Support solide selon les revendications 38 ou 39, dans lequel R³ est -OCH₂CH₂CN.

41. Support solide selon les revendications 38, 39 ou 40, dans lequel R¹ est un groupe protecteur sensible aux acides.

42. Support solide selon la revendication 41, dans lequel R¹ est un 4,4'-diméthoxytrityle.

43. Support solide selon les revendications 38, 39, 40, 41 ou 42, dans lequel R² est -H.

44. Support solide selon les revendications 38, 39, 40, 41 ou 42, dans lequel R² est -OR⁶ et R⁶ est un groupe protecteur d'hydroxy.

45. Support solide selon l'une quelconque des revendications 38 à 44, dans lequel L est -CH₂CH₂-.

46. Support solide selon l'une quelconque des revendications 38 à 45, dans lequel R¹⁵ comprend du verre à pores régulés, du polystyrène ou du polyamide microporeux.

47. Procédé de préparation d'un support solide dérivatisé par un multimère représenté par la formule structurale suivante, ou d'un stéréoisomère de celui-ci : 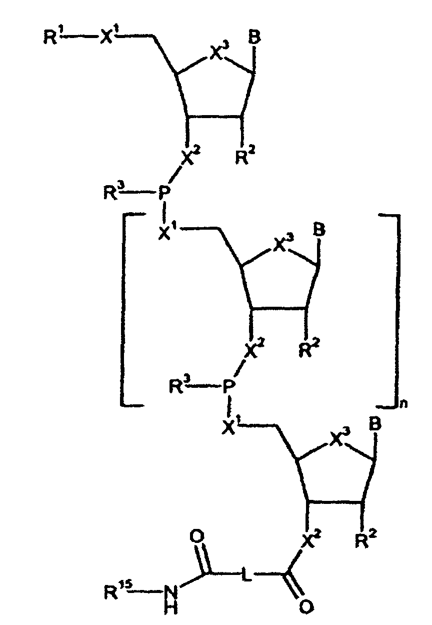 dans laquelle :
chaque X¹ est -O- ;
chaque X² est -O- ;
chaque X³ est indépendamment -O-, -S-, -CH₂-, ou -(CH₂)₂- ;
R¹ est un groupe protecteur ;
chaque R² est indépendamment -H, -F, -NHR⁶, -CH₂R⁶ ou -OR⁶ ;
chaque R³ est indépendamment un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ;
R⁶ est -H, un groupe aliphatique substitué ou non substitué, un groupe aromatique substitué ou non substitué, un aralkyle substitué ou non substitué ou un groupe protecteur ;
chaque B est indépendamment H ou une base nucléoside protégée ou non protégée ;
L est un groupe aliphatique substitué ou non substitué, ou un groupe aromatique substitué ou non substitué ;
n vaut 0 ou est un entier positif ; et
R¹⁵ est un support solide, comprenant les étapes de :
a) protection du substituant en 3' d'un nucléoside représenté par la formule structurale suivante, ou d'un stéréoisomère de celui-ci : 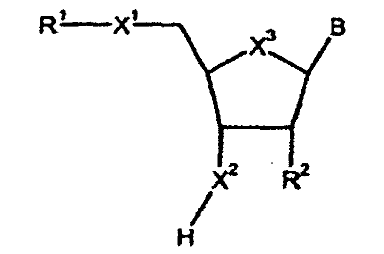 en formant ainsi un premier intermédiaire représenté par la formule structurale suivante ou un stéréoisomère de celui-ci : 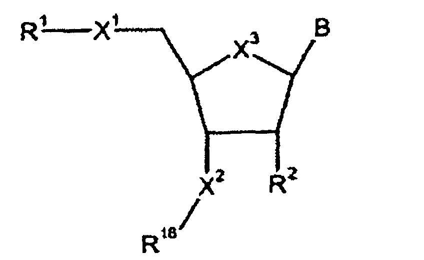 dans laquelle R¹⁶ est un groupe protecteur qui est orthogonal à R¹ ;
b) traitement du premier intermédiaire pour éliminer R¹, en formant ainsi un nucléoside déprotégé en 5' ;
c) réaction du nucléoside déprotégé en 5' en présence d'un catalyseur de couplage avec un composé représenté par la formule structurale suivante ou un stéréoisomère de celui-ci : 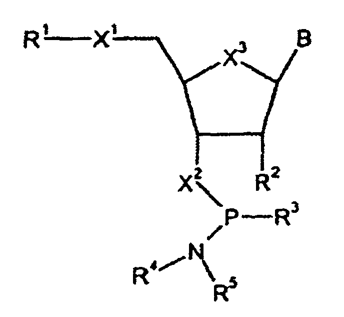 dans laquelle :
R⁴ et R⁵ sont chacun indépendamment un groupe aliphatique substitué ou non substitué, ou R⁴ et R⁵ pris conjointement avec l'azote auquel ils sont liés forment un groupe hétérocycloalkyle ; en formant ainsi un multimère protégé en 3',5' représenté par la formule structurale suivante, ou un stéréoisomère de celui-ci : 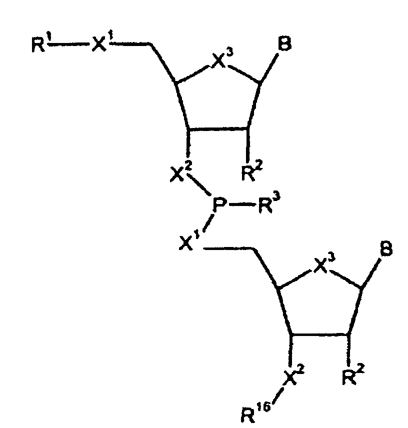
d) traitement éventuel du multimère protégé en 3',5' pour éliminer R¹⁶ en formant ainsi un multimère déprotégé en 3' ; puis
e) réaction en présence d'un catalyseur de couplage du multimère déprotégé en 3' avec un composé représenté par la formule structurale suivante, ou un stéréoisomère de celui-ci : 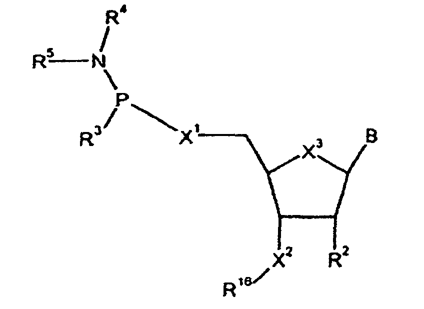 en formant ainsi un composé protégé en 3',5' représenté par la formule structurale suivante ou un stéréoisomère de celui-ci : 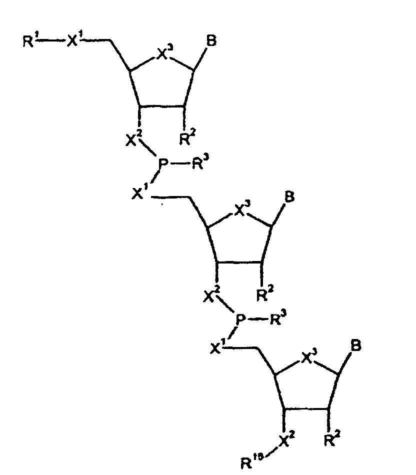
f) répétition éventuelle des étapes d) et e) une ou plusieurs fois, en formant ainsi un multimère protégé en 3',5' allongé ;
g) traitement du multimère protégé en 3',5' pour éliminer R¹⁶, en formant ainsi un multimère déprotégé en 3' ;
h) réaction du multimère déprotégé en 3' en présence d'une base avec un composé choisi parmi le groupe constitué de : 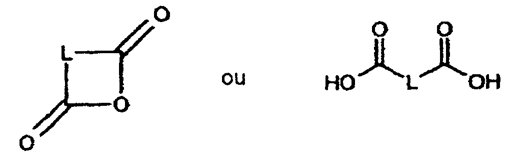 en formant ainsi un réactif de charge de support solide, représenté par la formule structurale suivante, ou un stéréoisomère de celui-ci : 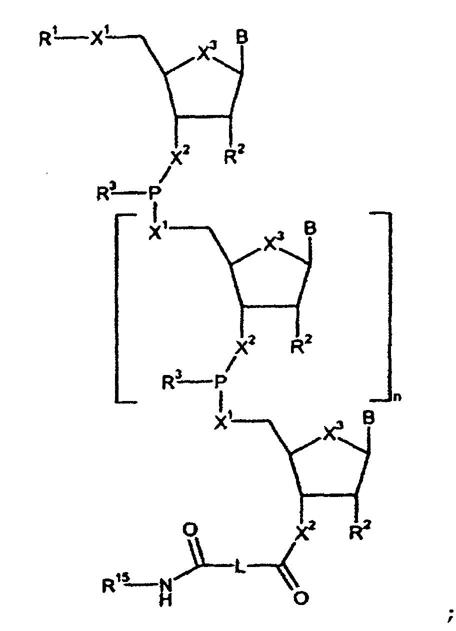 et
i) réaction du réactif de charge de support solide avec un support solide fonctionnalisé avec des groupes amine primaires ou secondaires en présence d'une base et d'un carbodiimide dialiphatique, substitué ou non substitué, en préparant ainsi ledit support solide dérivatisé par un multimère.

48. Procédé selon la revendication 47, comprenant en outre l'étape de réaction du réactif de charge de support solide formé dans l'étape h) avec le p-nitrophénol en présence d'une base et d'un carbodiimide dialiphatique substitué ou non substitué en formant ainsi un réactif de charge de support solide activé.

49. Procédé selon la revendication 48, dans lequel le carbodiimide dialiphatique substitué ou non substitué est le dicyclohexylcarbodiimide ou le diisopropyle carbodiimide.

50. Procédé de préparation d'un support solide dérivatisé par un multimère représenté par la formule structurale suivante, ou d'un stéréoisomère de celui-ci : 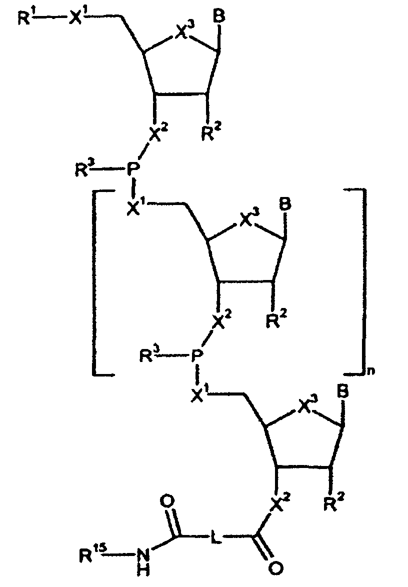 dans laquelle :
chaque X¹ est indépendamment -O- ;
chaque X² est indépendamment -O- ;
chaque X³ est indépendamment, -O-, -S-, -CH₂-, ou -(CH₂)₂- ;
R¹ est un groupe protecteur ;
chaque R² est indépendamment -H, -F, -NHR⁶, -CH₂R⁶ ou -OR⁶ ;
chaque R³ est indépendamment un groupe bêta-cyanoéthyloxy, bêta-cyanoéthylthio, 4-cyanobut-2-énylthio, 4-cyanobut-2-ényloxy, allylthio, allyloxy, crotylthio, ou crotyloxy ;
R⁶ est -H, un groupe aliphatique substitué ou non substitué, un groupe aromatique substitué ou non substitué, un aralkyle substitué ou non substitué ou un groupe protecteur ;
chaque B est indépendamment H ou une base nucléoside protégée ou non protégée ;
L est un groupe aliphatique substitué ou non substitué, ou un groupe aromatique substitué ou non substitué ;
n vaut 0 ou est un entier positif ; et
R¹⁵ est un support solide, comprenant les étapes de :
a) protection du substituant 3' d'un nucléoside représenté par la formule structurale suivante, ou d'un stéréoisomère de celui-ci : 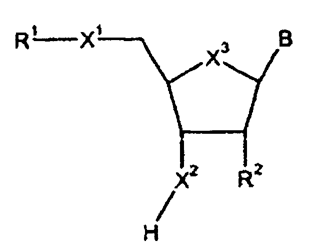 en formant ainsi un premier intermédiaire représenté par la formule structurale suivante, ou un stéréoisomère de celui-ci : 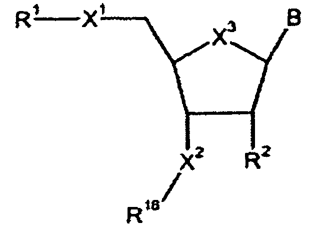 dans laquelle R¹⁶ est un groupe protecteur qui est orthogonal à R¹ ;
b) traitement du premier intermédiaire avec un acide pour éliminer R¹, en formant ainsi un nucléoside déprotégé en 5' ;
c) réaction du nucléoside déprotégé en 5' en présence d'un composé phosphoré trivalent représenté par l'une des formules structurales suivantes : 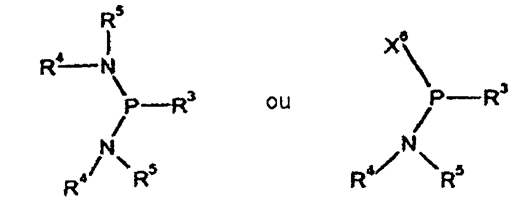 dans lesquelles X⁶ est un halogène en formant ainsi un 5'-phosphoramidite ; et
R⁴ et R⁵ sont chacun indépendamment un groupe aliphatique substitué ou non substitué, ou R⁴ et
R⁵ pris conjointement avec l'azote auquel ils sont liés, forment un groupe hétérocycloalkyle ;
d) réaction du 5'-phosphoramidite en présence d'un catalyseur de couplage avec un composé représenté par la formule structurale suivante, ou un stéréoisomère de celui-ci : 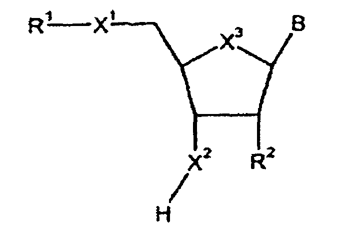 en formant ainsi un multimère protégé en 3',5' représenté par la formule structurale suivante, ou un stéréoisomère de celui-ci : 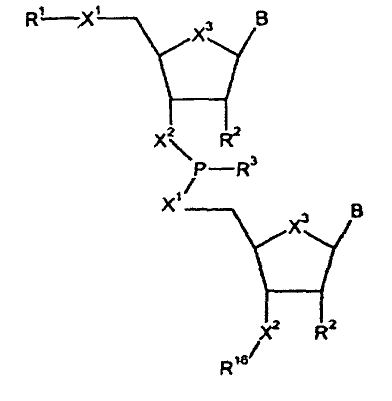
e) traitement éventuel du multimère protégé en 3',5' pour éliminer R¹⁶ en formant ainsi un multimère déprotégé en 3' ; puis
f) réaction en présence d'un catalyseur de couplage du multimère déprotégé en 3' avec un composé représenté par la formule structurale suivante, ou un stéréoisomère de celui-ci : 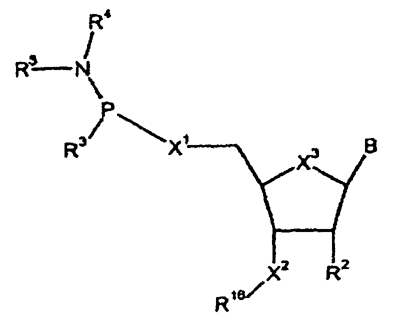 en formant ainsi un composé protégé en 3',5' représenté par la formule structurale suivante, ou un stéréoisomère de celui-ci : 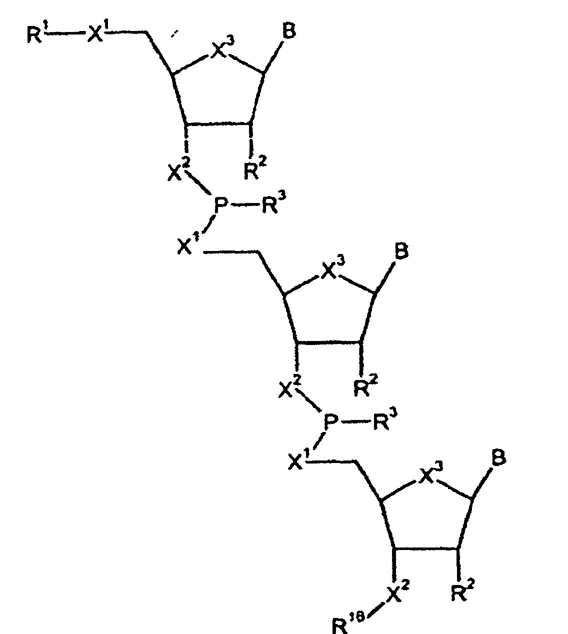
g) répétition éventuelle des étapes e) et f) une ou plusieurs fois, en formant ainsi un multimère protégé en 3',5' allongé ;
h) traitement du multimère protégé en 3',5' pour éliminer R¹⁶, en formant ainsi un multimère déprotégé en 3' ;
i) réaction du multimère déprotégé en 3' en présence d'une base avec un composé choisi parmi le groupe constitué de : 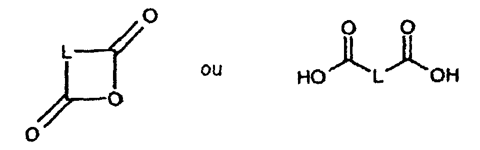 en formant ainsi un réactif de charge de support solide représenté par la formule structurale suivante ou un stéréoisomère de celui-ci : 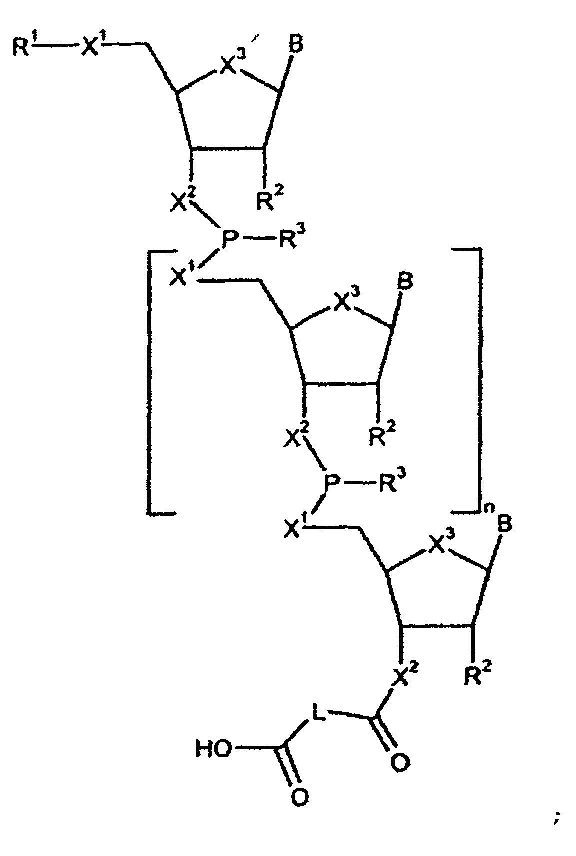 et
j) réaction du réactif de charge de support solide avec un support solide fonctionnalisé avec une amine primaire ou secondaire fonctionnelle en présence d'une base et d'un carbodiimide dialiphatique substitué ou non substitué, en préparant ainsi ledit support solide dérivatisé par un multimère.

51. Procédé selon la revendication 50, comprenant en outre l'étape de réaction du réactif de charge de support solide formé dans l'étape i) avec le p-nitrophénol en présence d'une base et d'un carbodiimide dialiphatique substitué ou non substitué en formant ainsi un réactif de charge de support solide activé.

52. Procédé selon la revendication 51, dans lequel le carbodiimide dialiphatique substitué ou non substitué est le dicyclohexylcarbodiimide ou le diisopropylcarbodiimide.

53. Utilisation d'un composé phosphoramidite selon l'une quelconque des revendications 1 à 14 pour la synthèse d'oligonucléotides.

54. Utilisation d'un support solide dérivatisé par un multimère phosphoré trivalent selon l'une quelconque des revendications 38 à 46 pour la synthèse d'oligonucléotides.
